# EUROPEAN PATENT SPECIFICATION

(11) **EP 0 492 350 B1**
(45) Date of publication and mention of the grant of the patent: **15.10.1997**
(21) Application number: 91121554.9
(22) Date of filing: 16.12.1991
(51) Int. Cl.: C07D 207/27, C07D 207/267, C07C 229/30

(54) **Process for the production of (S)-vinyl and (S) allenyl gaba**
Verfahren zur Herstellung von (S)-vinyl- und (S)-allenyl-Gaba
Procédé pour la préparation de (S)-vinyl et de (S)-allenyl-gaba

(30) Priority: 17.12.1990 US 628738
(43) Date of publication of application: 01.07.1992
(73) Proprietor: MERRELL PHARMACEUTICALS INC., Cincinnati, Ohio 45215-6300 (US)
(72) Inventor: Evans, Jonathan C., Midland, Michigan 48640 (US)
(74) Representative: VOSSIUS & PARTNER

(56) References cited:
- EP-A- 0 094 886
- EP-A- 0 116 257
- EP-A- 0 134 481
- EP-A- 0 342 613
- US-A- 3 959 356
- US-A- 3 960 927
- US-A- 4 912 232
- CHEMICAL ABSTRACTS, vol. 109, 1988, page 682, abstract no. 190111b, Columbus, Ohio, US; W. DEGER et al.: "Stereoisomeric flavor compounds. 18. Enantiodiscrimination of chiral flavor compounds by diastereomeric derivatization", & J. AGRIC. FOOD CHEM. 1988, 36(6), 1260-4

## Description

The present invention is directed to a stereospecific synthesis of (S)-vinyl-GABA, (S)-allenyl-GABA, (S)-5-allenylpyrrolidinone, and (S)-5-vinyl-pyrrolidinone. Another aspect is directed to a class of 1,5-disubstituted-pyrrolidinone intermediates as well as a new class of chiral lactic acid derivatives.

4-Amino-5-hexenoic acid is known in the art as an anti-epileptic agent and is described in United States Patent No. 3,960,927. It is also known as vinyl-GABA and is currently available from Merrell Dow Pharmaceuticals, Inc. United States Patent No. 4,621,145 describes one method for synthesizing this compound. The last step in the reaction sequence is depicted below:

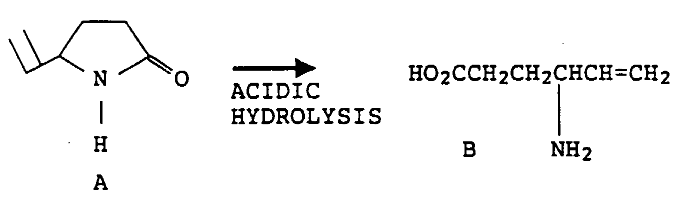

In this reaction 5-vinyl-2-pyrrolidinone (structure A) is subjected to an acidic hydrolysis thereby producing the desired compound, 4-amino-5-hexenoic acid (structure B). This acidic hydrolysis is carried out using techniques known in the art. Typically, the 5-vinyl-2-pyrrolidinone is contacted with a strong acid such as hydrochloric acid or trifluoroacteic acid at a temperature above 60°C in an aqueous solvent system.

European Patent Application 0 427 197 discloses that vinyl-GABA can be produced by subjecting 5-vinyl-2-pyrrolidinone to a basic hydrolysis as well. This hydrolysis is typically carried out by contacting the 5-vinyl-2-pyrrolidinone with a molar excess of potassium hydroxide. Typically from about 1.1 to about 1.5 equivalents are utilized. The basic hydrolysis is carried out at a temperature ranging from about 60°C to 140°C. The reaction is typically carried out for a period of time ranging from about 0.5 hours to about 24 hours.

4-Amino-hepta-5,6-dienoic acid is also known in the art as an anti-epileptic agent and is described in United States Patent No. 4,454,156. This compound is also known as allenic-GABA and is under development by Merrell Dow Pharmaceuticals, Inc. Castelhano et al. discloses that allenic-GABA (Structure E) can be produced by a hydrolysis similiar to that discussed above utilizing of 5-allenyl-2-pyrrolidinone (Structure D) as the starting material, J. Am. Chem. Soc. Vol. 106, pages 1877-1879 (1984). This reaction may be depicted as:

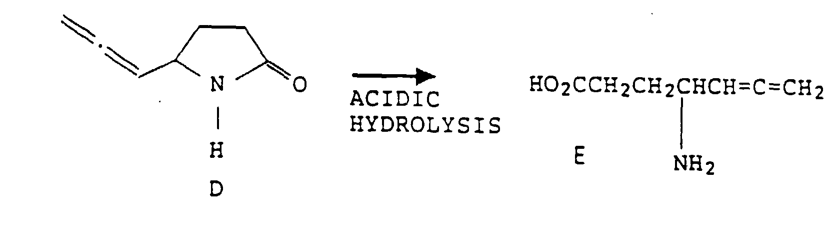

United States Patent Nos. 4,621,145 and 4,454,156 disclose that the S-enantiomersof 4-amino-5-hexenoic acid t and 4-amino-hepta-5,6-dienoic acid are the preferred isomers.

Recent efforts have focused on developing a commercially viable method for synthesizing the S-enantiomer of these compounds. The following synthetic procedure was developed as depicted by Reaction Scheme I:

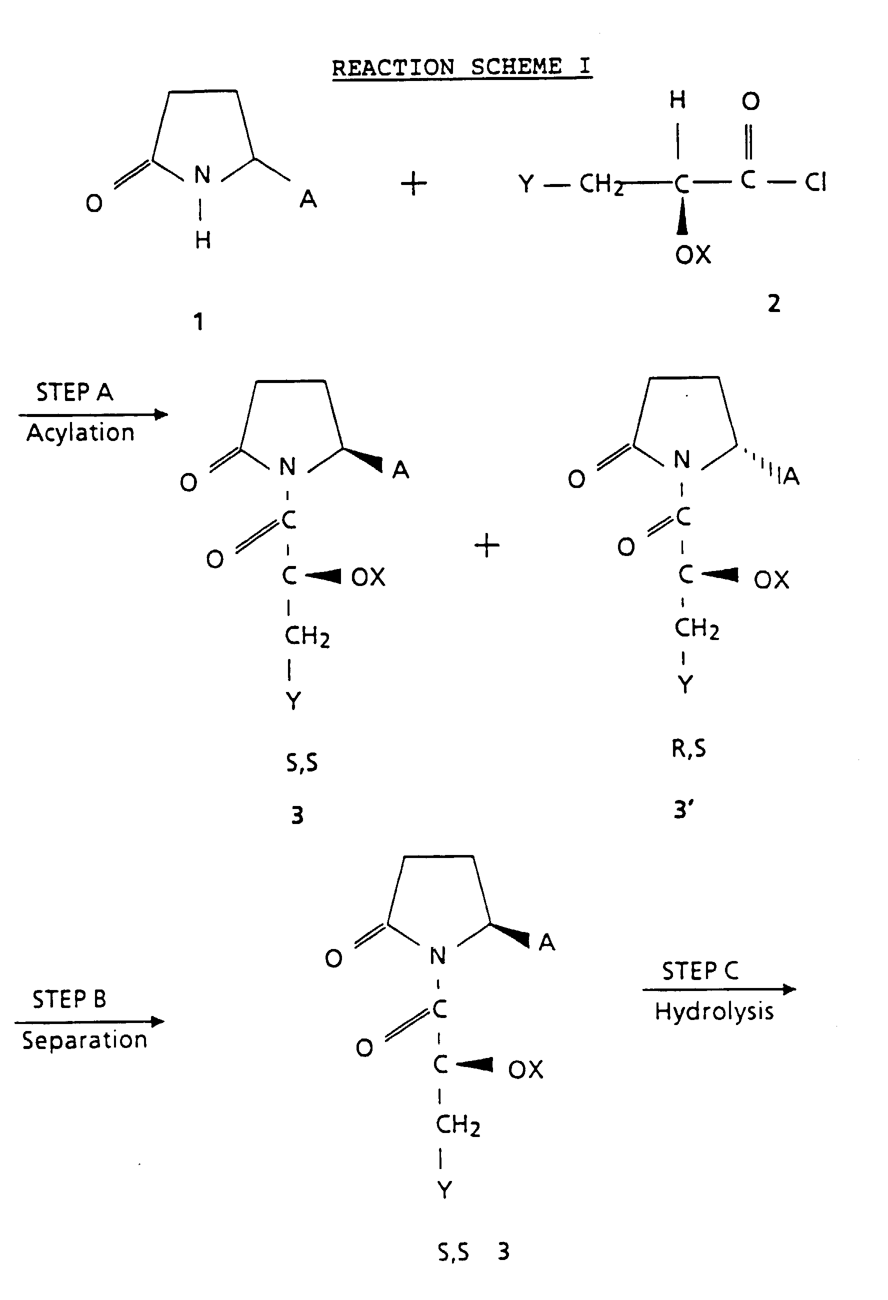

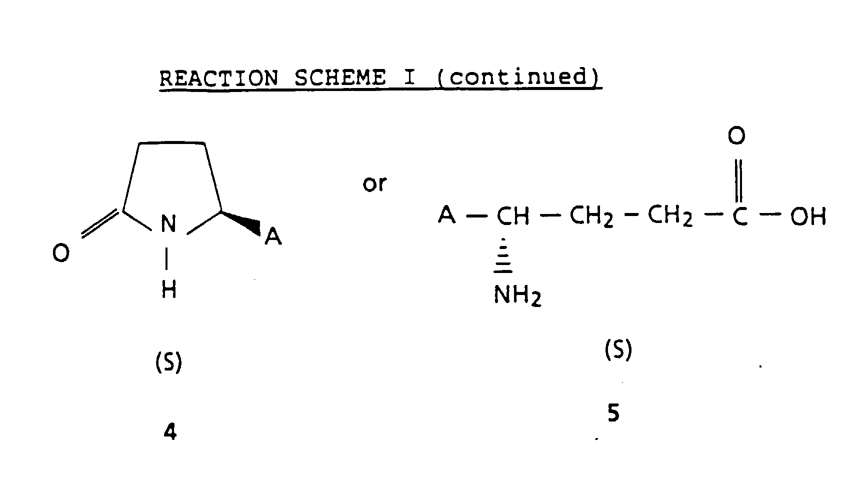

In Step A, a racemic pyrrolidinone derivative as described by structure 1 in which A is represented by -CH=C=CH₂ or -CH=CH_{2,} is acylated with a chiral auxiliary as described by structure 2 in which X and Y are simultaneously represented by the following substituents: 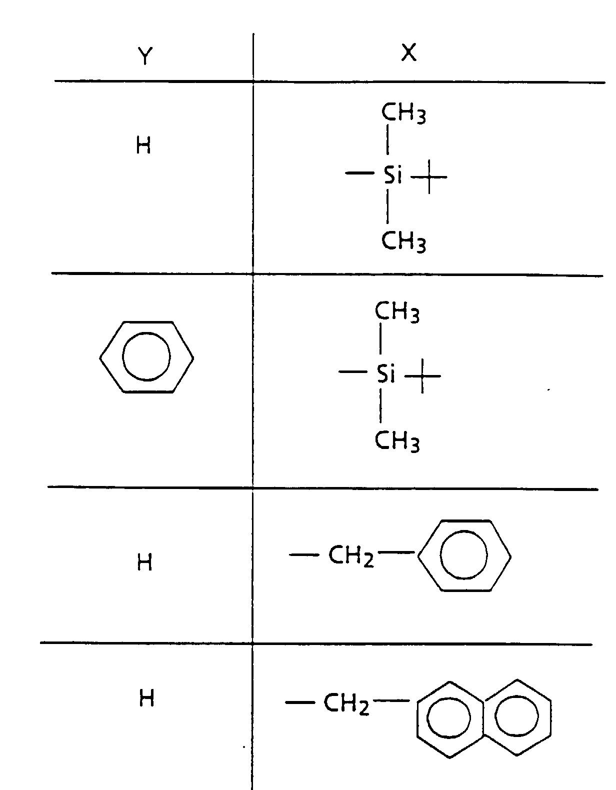 This acylation reaction produces the diastereomers described by structure 3 and 3' in which A, X, and Y are as defined above. In Step B the diastereomers are separated and the desired S,S isomer depicted by structure 3 is recovered. In Step C, this diastereomer is subjected to a hydrolysis reaction. Depending on the manner in which the hydrolysis is carried out, this reaction produces either the S-pyrroldinone derivative described by structure 4 or the (S)-amino acid derivative described by structure 5 in which A is as defined above.

The acylation reaction of Step A can be carried out using techniques known in the art. Typically the pyrrolidinone derivative of structure 1 is contacted with an approximately equivalent amount of a base such as sodium hydride, butyllithium, lithium diisopropylamine, potassium t-butoxide and potassium carbonate for a period of time ranging from 15 minutes to 3 hours. The reactants are typically contacted at a depressed temperature in the range of -40°C to 25°C in an aprotic solvent such as toluene, tetrahydrofuran, toluene/mineral oil, etc. The reaction medium is then warmed to approximately room temperature and an equivalent amount of the chiral auxiliary of structure 2 is added to the reaction. The reactants are typically stirred together for a period of time ranging from 1 minute to 3 hours. The reaction is then quenched by the addition of water and the diastereomers of structure 3 and 3' are recovered by extraction.

In Step B, the diastereomers are separated by techniques known in the art. One suitable method is flash chromatography on silica gel. Suitable eluants include hexane/ethyl acetate, t-butylmethyl ether/hexane and toluene. Another suitable separation method is recrystallization. Suitable solvent systems include polar solvents such as hexane/ethyl acetate or tetrahydrofuran.

In Step C, the (S,S)-diastereomer produced above is subjected to a hydrolysis reaction. Depending upon the manner in which the reaction is carried out, the product will either be the (S)-pyrrolidinone derivative of structure 4 or the (S)-amino acid derivative of structure 5. The pyrrolidinone derivative of Structure 4 can be produced by subjecting the diastereomer to a basic hydrolysis with a weak base such as K₂CO₃. This hydrolysis is typically carried out in methanol at a temperature range of from 25°C to 65°C for a period of time ranging from 1 to 4 hours. The amino acid derivative can be produced by subjecting the diastereomer to a hydrolysis with a stronger base such as potassium hydroxide in a solvent such as water. This hydrolysis is typically carried out at a temperature ranging from 50°C to 140°C for a period of time ranging from 1 to 24 hours. Alternatively, the amino acid derivative of structure 5 can be produced via an acidic hydrolysis in which hydrochloric acid is used.

An alternative method of producing either the (S)-pyrrolidinone of structure 4 or the (S)-amino acid of structure 5 is depicted below in Reaction Scheme II:

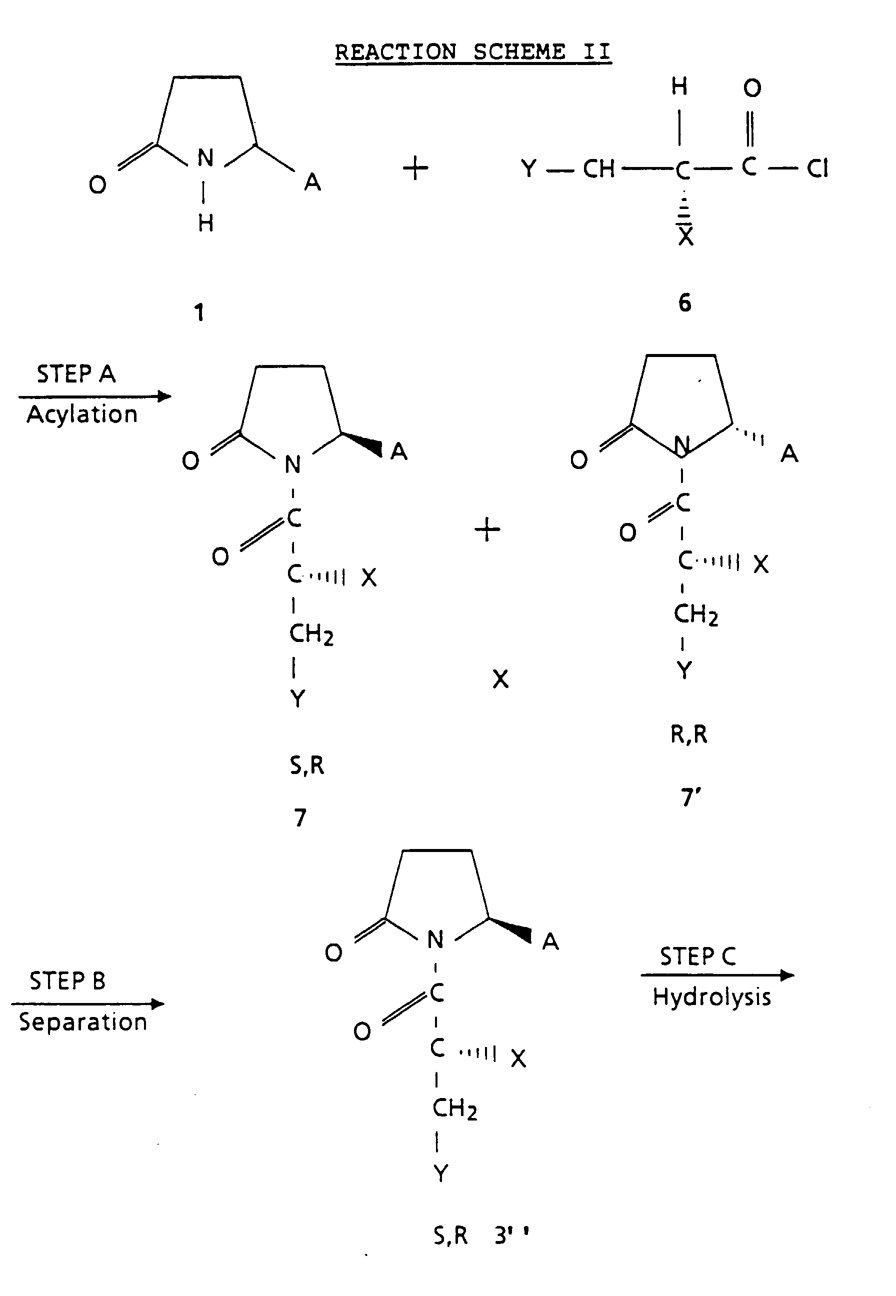

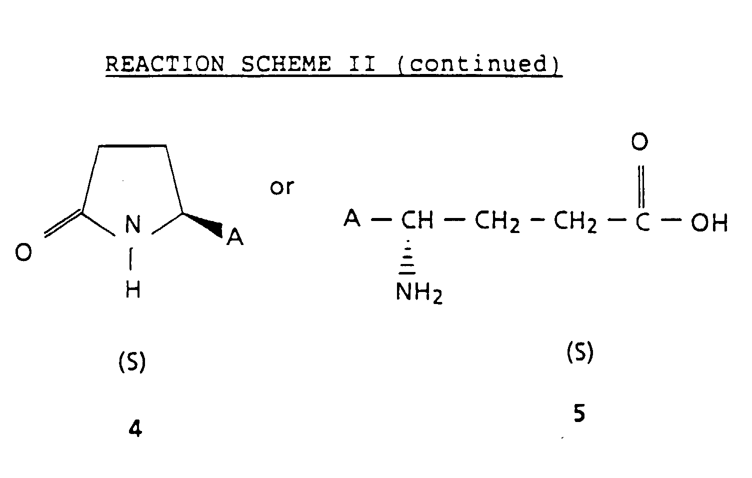

The initial step is to carry out an acylation reaction between the pyrrolidinone of structure 1 in which A is as defined above and the chiral auxiliary of structure 6 in which X and Y are simultaneously represented by

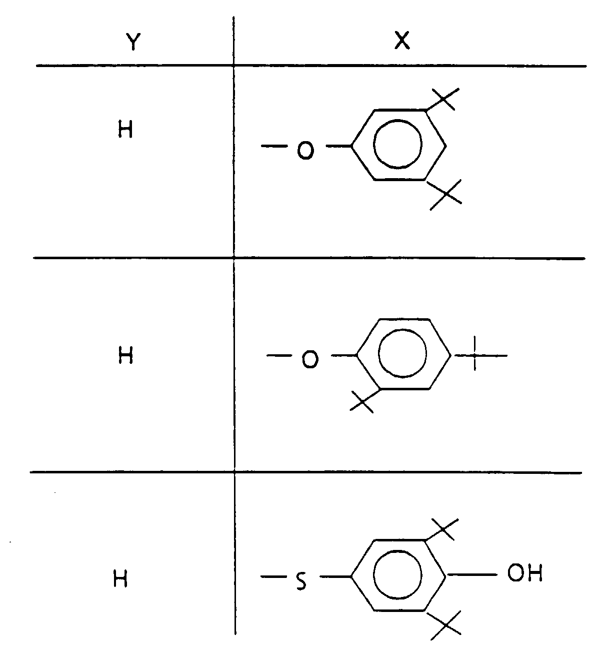

This acylation produces the diastereomers of structure 7 and 7'. This acylation can be carried out in the same manner discussed above for Step A of Reaction Scheme I. In Step B these diastereomers are separated and the (S,R)-diastereomer (7') is recovered for further processing. This separation can be carried out in the same manner as the separation of Step B in Reaction Scheme I. In Step C the diastereomer of structure 7 is subjected to a hydrolysis reaction thereby producing the (S)-pyrrolidinone derivative of structure 4 or the amino acid derivative of structure 5. This hydrolysis can be carried out in the same manner as above.

Methods for producing the chiral auxiliaries of structures 2 and 6 are known in the art. Specific methods are disclosed in Examples 1-7. Methods for producing the pyrrolidinones of structure 1 are known in the art.

The following examples are being presented in order to further illustrate the invention. They should not be construed as limiting the invention in any manner.

### EXAMPLE 1

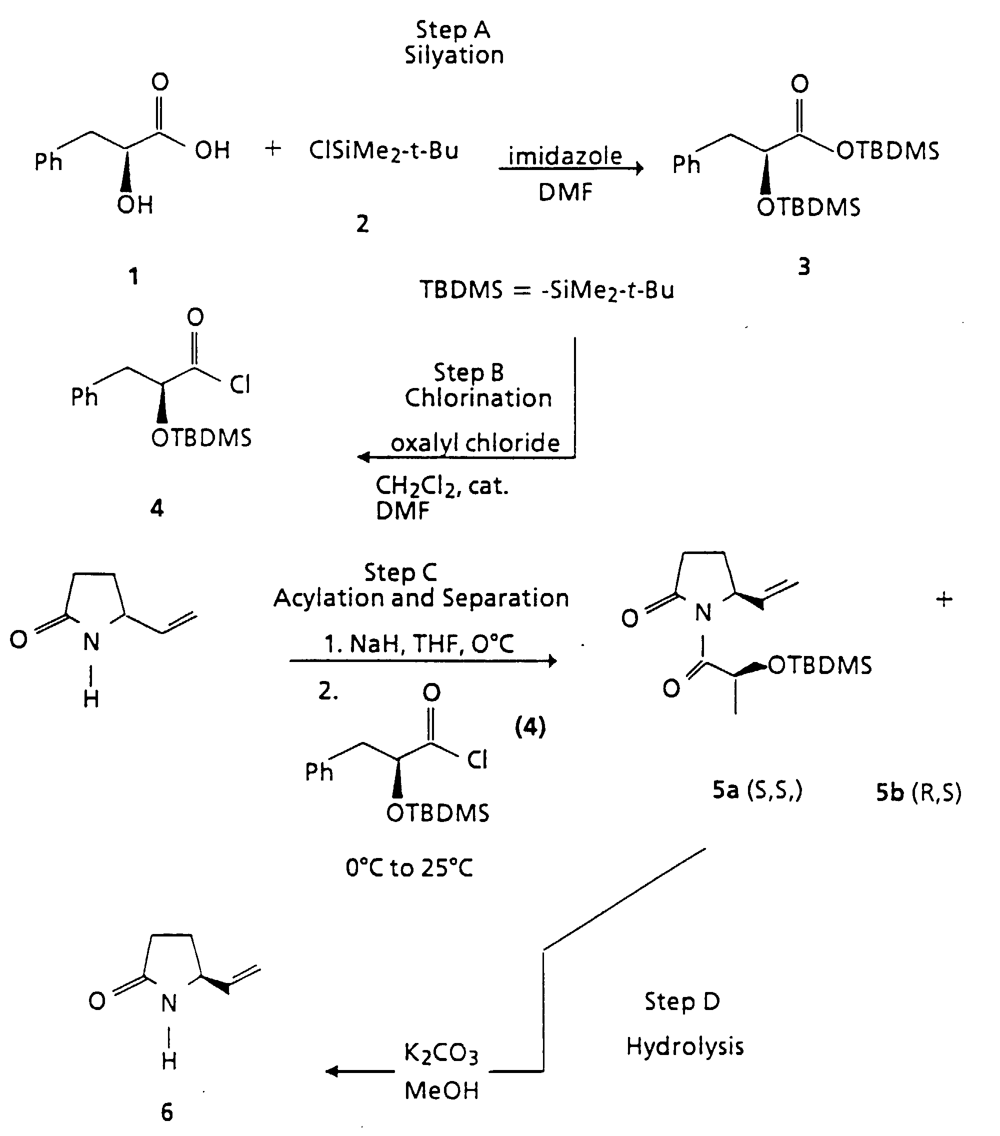

### STEP A SILYATION

### (1,1-Dimethylethyl)dimethylsilyl (2S)-1((1,1-Dimethylethyl)dimethylsilyloxy)-3-phenylpropanoate

To a solution of 10.04 g (60 mmol) of (2S)-3-phenyllacetic acid and 18.10 g (270 mmol) of imidazole in N,N-dimethylformamide(200 mL) was added 19.95 g (130 mmol) of t-butyldimethylchlorosilane at 25°C. The reaction mixture was stirred at room temperature for 18 hours under an argon atmosphere. To the reaction mixture was then added hexane (400 mL). The bi-phasic solution was stirred for 15 minutes prior to the addition of water (300 mL). The solution was stirred for 1 minute before the phases were separated. The organic phase was dried over magnesium sulfate. Concentration and vacuum distillation afforded 22.2 g (93%) of (1,1-dimethylethyl)dimethylsilyl (2S)-2-((1,1-dimethylethyl)dimethylsilyloxy)-3-phenylpropanoate (3) as a clear liquid: bp 123-125°C (0.5 mm Hg); ¹H NMR (CDCl₃) δ - 0.206 (s,3H)), -0.0741 (s,3H), 0.253 (s, 3H), 0.273 (s, 3H), 0.812 (s, 9H), 0.942 (s, 9H), 2.85-2.92 (m, 1H), 3.05-3.11 (m, 1H), 4.27-4.31 (m, 1H), 7.21-7.31 (m, 5H); ¹³C NMR (CDCl₃) δ 173.2, 137.6, 129.8, 128.3, 126.5, 74.5, 41.7, 25.7, 25.6, 18.2, 17.7, -4.89, -5.19, -5.73.

### STEP B CHLORINATION

### (2S)-2-((1,1-Dimethylethyl)dimethylsilyloxy)-3-phenylpropanoyl chloride (4)

To a solution of 21.0 g (53 mmol) of (1,1-dimethylethyl)-dimethylsilyl (2S)-2-((1,1-dimethylethyl)dimethylsilyloxy)-3-phenylpropanoate (3) and five drops of N,N-dimethylformamide in methylene chloride at 0°C was added 29.3 mL (59 mmol) of a 2.0 M solution of oxalyl chloride in methylene chloride over a 30 min period. The cooling bath was removed and the reaction mixture was allowed to stir at room temperature for 18 hours. The solvent and excess oxalyl chloride were removed by rotary evaporation resulting in a yellow liquid. Purification by vacuum distillation afforded 12.7 g (80%) of (2S)-2-((1,1-dimethylethyl)-dimethylsilyloxy)-3-phenylpropanoyl chloride (4) as a clear liquid: bp 88-90°C (0.2 mm Hg); ¹H NMR (CDCl₃) δ -0.215 (s, 3H), -0.0339 (s, 3H), 0.840 (s, 9H), 2.94-3.01 (m, 1H), 3.23-3.29 (m, 1H), 4.52-4.56 (m, 1H), 7.25-7.35 (m, 5H); ¹³C NMR (CDCl₃) δ 175.7, 135.9, 129.8, 128.4, 127.1, 81.1, 40.8, 25.5, 18.0, -5.40, -5.76.

### STEP C ACYLATION AND SEPARATION

### N-Acylation of 5-Vinylpyrrolidin-2-one with (2S)-2-((1,1-dimethylethyl)dimethylsilyloxy)-3-phenylpropanoyl chloride (4)

To 60% sodium hydride (336 mg, 10 mmol, in mineral oil) in tetrahydrofuran (20 mL) at 0°C was added dropwise a solution of 5-vinylpyrrolidin-2-one (1.0 g, 9.0 mmol) in tetrahydrofuran (10 mL) over a 5 min period. The cooling bath was removed and the solution was allowed to warm to 25°C to ensure complete anion generation. To the anion at 25°C was added dropwise a solution of (2S)-2-((1,1-dimethylethyl)dimethylsilyloxy)-3-phenylpropanoyl chloride (4, 2.98 g, 10 mmol) in tetrahydrofuran (10 mL) over a 5 min period. TLC indicated that the reaction was complete upon addition of the acid chloride (silica gel plates; hexane and ethyl acetate, 9:1). The solvent was removed by rotary evaporation affording a thick slug. The slug was dissolved in methylene chloride (75 mL) and washed with water (1x50 mL), a saturated aqueous sodium bicarbonate solution (1x50 mL) and dried (MgSO₄). Concentration and purification by flash chromatography on silica gel (EM 60, 0.040-0.063 partical size, 230-400 Mesh, column size: 4.5 cm diameter x 15 cm length) using hexane and ethyl acetate (19:1) afforded 1.54 g (46%) of (5S)-N-((2S)-2-((1,1-dimethylethyl)-dimethylsilyloxy)-3-phenylpropanoyl)-5-ethenylpyrrolidin-2-one (5a) as a white crystalline material: mp 92-93.5°C; ¹H NMR (CDCl₃) δ -0.316 (s,3H), -0.217 (s,3H), 0.703 (s, 9H), 1.94 (t, J=9.6 Hz, 1H), 2.29 (p, J=11.7 Hz, 1H), 2.50-2.75 (m, 3H), 3.11 (d, J=12.8 Hz, 1H), 4.97-5.13 (m, 1H), 5.12-5.18 (m, 2H), 5.45 (d, J=9.8 Hz, 1H), 5.75-5.86 (m, lH); ¹³C NMR (CDCl₃) δ 175.1, 174.1, 137.9, 135.4, 130.0, 128.0, 126.4, 115.8, 74.2, 58.1, 41.2, 31.7, 25.5, 24.3, 18.1, - 5.50, -5.76.

### STEP D HYDROLYSIS

### (5S)-5 -Ethenylpyrrolidin-2-one (6) From (5S)-N-((2S)-2-((1,1-Dimethylethyl)dimethylsilyloxy)-3-phenylpropanoyl)-5 -ethenylpyrrolidin-2-one (5a)

To a solution of 500 mg (1.0 mmol) of (5S)-N-((2S)-2-((1,1-dimethylethyl)dimethylsilyloxy)-3-phenylpropanoyl)-5-ethenylpyrrolidin-2-one (5a) in methanol (20 mL) and water (5 mL) was added 152 mg (1.1 mmol) of potassium carbonate. The resulting reaction mixture was stirred at 25°C for 1 hour before a sample was removed for TLC analysis. TLC was carried out on silica gel plates and eluted with hexane and ethyl acetate (2:1). An additional 20 mL of water was added and the solution was extracted with methylene chloride (2x15 mL). The aqueous layer was acidified to pH ∼4 with dilute hydrochloric acid (1.0 M) and extracted with methylene chloride (2x20 mL). The combined organic phases were dried over magnesium sulfate. Concentration and purification by flash chromatography on silica gel (EM 60, 0.040-0.063 partical size, 230-400 Mesh, column size: 3.5 cm diameter x 12 cm length) using hexane and ethyl acetate (9:1) afforded (67%) of (5S)-5-ethenyl-2-pyrrolidinone (6) as a clear thick oil: ¹H NMR (CDCl₃) δ 1.77-1.88 (m, 1H), 2.22-2.42 (m, 2H), 4.13-4.19 (m, 1H), 5.11 (d, J=10.0 Hz, 1H), 5.22 (d, J=17.0 Hz, 1H), 5.75-5.86 (m, 1H), 7.16-7.32 (b, NH, ¹H); ¹³C NMR (CDCl₃) δ 178.5, 138.6, 115.3, 56.6, 29.8, 27.8.

### EXAMPLE 2

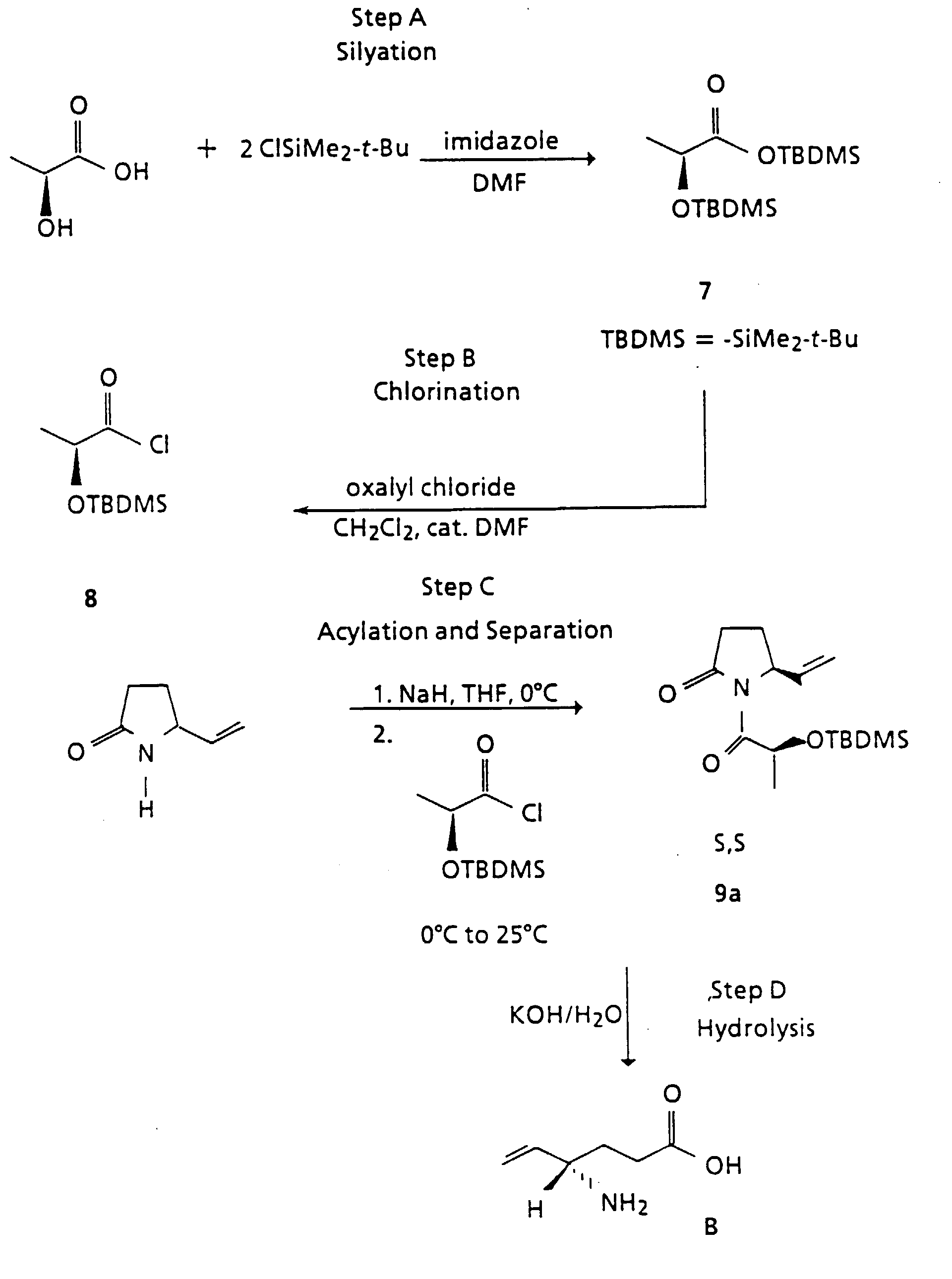

### STEP A SILYATION

### (1,1-Dimethylethyl)dimethylsilyl (2S)-2-((1,1-dimethylethyl)dimethylsilyloxy)propanoate (7)

To a solution of 25.0 g (0.28 mol) of(2S)-lactic acid and 85.8 g (1.26 mol) of imidazole in N,N-dimethylformamide (400 mL) was added 92.0 g (0.62 mol) of t-butyldimethylchlorosilane at 25°C. The reaction mixture was stirred at room temperature for 18 hours under an argon atmosphere. To the reaction mixture was then added hexane (400 mL). The bi-phasic solution was stirred for 15 minutes prior to the addition of water (400 mL). The solution was stirred for 1 minute before the phases were separated. The organic phase was dried over magnesium sulfate. Concentration and vacuum distillation afforded 82.1 g (92%) of (1,1-dimethylethyl)dimethylsilyl (2S)-2-(1,1-dimethylethyl)dimethylsilyloxy)propanoate (7) as a clear liquid: bp 83-85°C (0.02 mm Hg); ¹H NMR (CDCl₃) δ 0.00836 (s, 3H), 0.0608 (s, 3H), 0.0962 (s, 6H), 0.859 (s, 9H), 0.934 (s, 9H), 1.39 (d, J=6.7 Hz, 3H), 4.25 (q, J=6.7 Hz, 1H); ¹³C NMR (CDCl₃) δ 174.2, 69.1, 25.8, 25.6, 21.3, 18.3, 17.7, -4.83, -5.32.

### STEP B CHLORINATION

### (2S)-2-((1,1-Dimethylethyl)dimethylsilyloxy)propanoyl chloride (8)

To a solution of 41.0 g (0.13 mol) of (1,1-dimethylethyl)dimethylsilyl (2S)-2-((1,1-dimethylethyl)dimethylsilyloxy)propanoate (7) and five drops of N,N-dimethylformamide in methylene chloride at 0°C was added 70.8 mL (0.14 mol) of a 2.0 M solution of oxalyl chloride in methylene chloride over a 30 minute period. The cooling bath was removed and the reaction mixture was allowed to stir at room temperature for 18 hours. The solvent and excess oxalyl chloride were removed by rotary evaporation resulting in a yellow liquid. Purification by vacuum distillation afforded 25.6 g (88%) of (2S)-2-((1,1-dimethylethyl)dimethylsilyloxy)propanoyl chloride (8) as a clear liquid: bp 42-45°C (1.5 mm Hg); ¹H NMR (CDCl₃) δ 0.0920 (s, 3H), 0.118 (s, 3H), 0.909 (s, 9H), 1.50 (d, J=6.7 Hz, 3H), 4.49 (q, J=6.7 Hz, 1H); ¹³C NMR (CDCl₃) δ 176.6, 76.0, 25.7, 25.6, 25.3, 20.9, 18.1, -1.57, -5.04, -5.28.

### STEP C ACYLATION AND SEPARATION

### N-Acylation of 5-Vinylpyrrolidin-2-one with (2S)-2-((1,1-Dimethylethyl)dimethylsilyloxy)propanoyl chloride (8)

To 60% sodium hydride (336 mg, 10 mmol in mineral oil) in tetrahydrofuran (20 mL) at 0°C was added dropwise a solution of 5-vinylpyrrolidin-2-one (1.0 g, 9.0 mmol) in tetrahydrofuran (10 mL) over a 5 minute period. The cooling bath was removed and the solution was allowed to warm to 25°C to ensure complete anion generation. To the anion at 25°C was added dropwise a solution of (2S)-2-((1,1-dimethylethyl)dimethylsilyloxy)propanoyl chloride (2.23 g, 10 mmol) in tetrahydrofuran (10 mL) over a 5 minute period. TLC indicated that the reaction was complete upon addition of the acid chloride (silica gel plates; hexane and ethyl acetate, 9:1). The solvent was removed by rotary evaporation affording a thick slug. The slug was dissolved in methylene chloride (75 mL) and washed with water (1x50 mL) and dried (MgSO4). Concentration and purification by flash chromatography on silica gel (EM 60, 0.040-0.063 partical size, 230-400 Mesh, column size: 4.5 cm diameter x 15 cm length) using hexane and ethyl acetate (19:1) afforded 1.40 g (47%) of (5S)-N-((2S)-2-((1,1-dimethylethyl)dimethylsilyloxy)propanoyl)-5-ethenylpyrrolidin-2-one (9a) as clear liquid: ¹H NMR (CDCl₃) δ 0.0300 (s,3H), 0.0656 (s, 3H), 0.877 (s, 9H), 1.37 (d, J=6.5 Hz, 3H), 1.82-1.88 (m, 1H) 2.21 (p, J=10.7 Hz, 1H), 2.34-2.47 (m, 1H), 2.60 (p, J=9.8 Hz, 1H), 4.88 (br, 1H), 5.05-5.11 (m, 2H), 5.36-5.38 (m, 1H), 5.70-5.79 (m, 1H); ¹³C NMR (CDCl₃) δ 174.86, 174.77, 135.5, 115.4, 66.8, 57.9, 31.6, 25.5, 24.4, 21.1, 18.1, -4.87, -5.16.

### STEP D HYDROLYSIS

### (4S)-Amino-5-hexenoic Acid (B) from (5S)-N-((2S)-2-((1,1-Dimethylethyl)dimethylsilyloxy)propanoyl)-5-ethenyl pyrrolidin-2-one (9a)

To a solution of diastereomer 9a (2.0 g, 6.7 mmol) in water (2 mL) is added 87% potassium hydroxide (1.2 g, 21.6 mmol). The resulting solution is refluxed 1 hour. The solution is allowed to cool to room temperature and resulting in two phases. The top phase is bis((1,1-dimethylethyl)dimethylsilyl)oxide. The phases are separated and the aqueous phase is extracted with methylene chloride (3 x 5 mL). The aqueous phase is diluted with isopropyl alcohol (15 mL) and ethanol (15 mL) to increase the solubility of the newly formed lactic acid. The solution is acidified to pH 5.5 with concentrated hydrochloric acid and extracted with diethyl ether (3 x 25 mL). Concentration of the extracts affords (4S)-amino-5-hexenoic acid (B).

### EXAMPLE 3

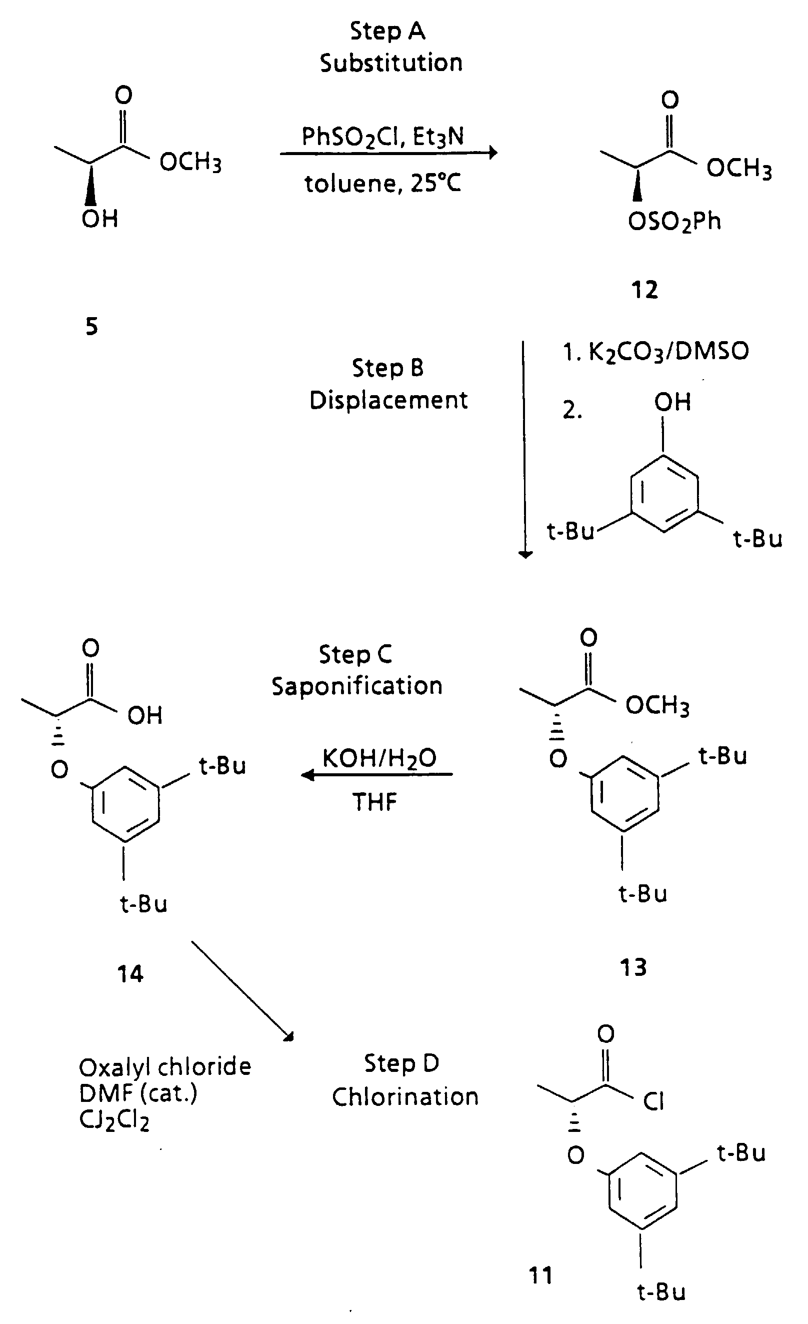

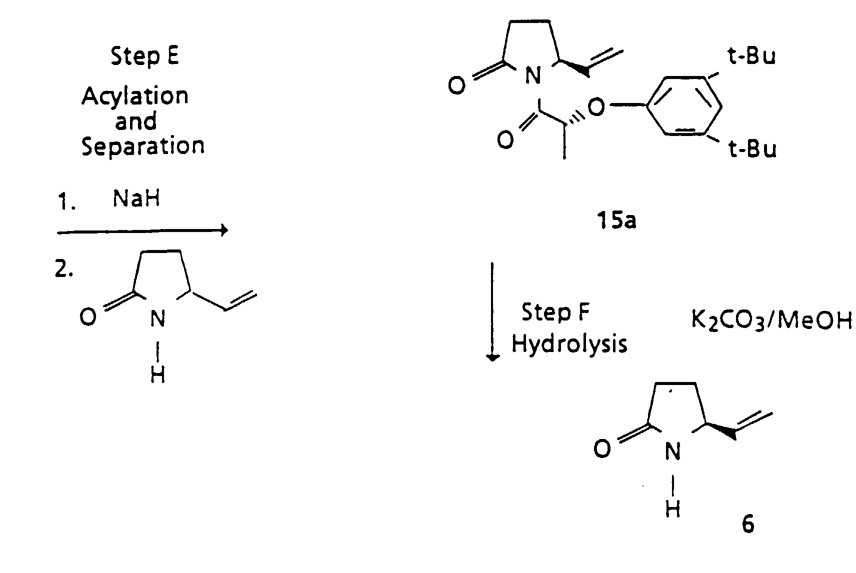

### STEP A SUBSTITUTION

### Methyl (2S)-2-(Phenylsulfonyl)oxypropanate (12)

To a solution of 200g (1.92 mol) of methyl (2S)-lactate (5) and 295 g (2.11 mol) of triethylamine in toluene (1 L) at 0°C was added 270 mL, (2.11 mol) of benzenesulfonyl chloride dropwise over a 2 hour period under a nitrogen atmosphere. The cooling bath was removed and the reaction mixture was allowed to warm to room temperature and stir there overnight. The reaction was worked-up by washing with water (3xl L) and dried (MgSO4). Filtration, concentration and purification by vacuum distillation afforded 440 g (94%) of methyl (2S)-2-(phenylsulfonyl)oxypropanate (12) as a pale yellow liquid: bp 140-142°C (0.1 mm Hg); ¹H NMR (CDCl₃) δ 1.51-1.56 (d, 3H), 3.66 (s, 3H), 4.95-5.01 (q, 1H), 7.54-7.59 (m, 2H), 7.65-7.70 (m, 1H), 7.93-7.95 (d, 2H); ¹³C NMR (CDCl₃) δ 169.3, 133.9, 129.1, 127.7, 112.3, 74.2, 52.5, 18.3; IR (neat) 2958 (w), 1762 (s), 1586 (m), 1451 (s), 1190 (vs), 1084 (s), 926 (m), 753 (m) cm⁻¹.

### STEP B DISPLACEMENT

### Methyl(2R)-2-(3,5-di-t-butylphenyloxy)propanate (13)

To a solution 8.87 g (43 mmol) of 3,5-di-t-butylphenol in dimethyl sulfoxide (150 mL) was 26.9 mL (43 mmol) of a 1.6 M *n*-butyllithium/hexane solution dropwise over a 15 min period under a nitrogen atmosphere. The solution was allowed to stir at room temperature for an additional 15 min resulting in the precipitation of the lithium salt of 3,5-di-*t*-butylphenol. A solution of 10.0 g (40 mmol) of methyl (2S)-2-(phenylsulfonyl)oxypropanate (12) in dimethyl sulfoxide (50 mL) was added dropwise to the suspension over a 10 min period. The reaction mixture was stirred overnight at room temperature. To the reaction solution was added water (300 mL) and the product was extracted with perchloroethylene (3x200 mL). The combined organic extracts were washed with water (1x200 mL), an aqueous saturated sodium chloride solution (1x200 mL) and dried (MgSO₄). Filtration, concentration and purification by flash chromatography on silica gel (EM silica gel 60, 0.040-0.063 partical size, 230-400 Mesh, column size: 7 cm diameter X 18 cm length) using hexane and ethyl acetate (19:1) afforded 11.75 g (89%) of methyl (2R)-2-(3,5-di-t-butylphenyloxy)propanate (13) as a thick oil: ¹H NMR (CDCl₃) δ 1.29 (s, 18H), 1.61-1.63 (d, 3H), 3.76 (s, 3H), 4.75-4.82 (q, 1H), 6.73 (s, 2H), 7.04 (s, 1H); ¹³C NMR (CDCl₃) δ 173.1, 157.2, 152.3, 115.8, 114.7, 109.7, 109.5, 72.7, 52.1, 34.9, 31.4, 18.6.

### STEP C DEPROTECTION

### (2R)-2-(3,5-Di-t-butylphenyloxy)propanoic Acid (14)

To a solution of 2.0 g (36 mmol) of potassium hydroxide in water (100 mL) and tetrahydrofuran (100 mL) was added 11.48 g (36 mmol) of methyl (2R)-2-(3,5-di-t-butylphenyloxy)propanate (13). The reaction mixture was allowed to stir overnight at room temperature. The reaction mixture was acidified to pH 2 with concentrated hydrochloric acid and the resulting solution was extracted with diethyl ether (3x200 mL). The combined extracts were washed with an aqueous saturated sodium chloride solution (1x300 mL) and dried (MgSO₄). Filtration and concentration resulted in a yellowish sludge. The crude product was purified by precipitation from hexane at -78°C to afford 8.2 g (75%) of (2R)-2-(3,5-di-t-butylphenyloxy)propanoic acid (14) as an off-white crystalline material: ¹H NMR (CDCl₃) δ 1.29 (s, 18H), 1.64-1.66 (d, 3H), 4.56-4.82 (q, 1H), 6.75 (s, 2H), 7.06 (s, 1H); ¹³C NMR (CDCl₃) δ 177.7, 156.8, 152.5, 116.2, 109.7, 72.3, 35.0, 31.4, 18.4.

### STEP D CHLORINATION

### (2R)-2-(3,5-Di-t-butylphenyloxy)propanoyl chloride (11)

To a mixture of 3.0 g (9.8 mmol) of (2R)-2-(3,5-di-*t*-butylphenyloxy)propanonic acid (14) and 4 drops of N,N-dimethylformamide in methylene chloride (50 mL) at 0°C under a nitrogen atmosphere was added 5.4 mL (10.8 mmol) of a 2M solution of oxalyl chloride in methylene chloride dropwise over a 20 min period. The reaction mixture was allowed to stir at room temperature overnight. The solvent and any excess oxalyl chloride was removed by rotary evaporation and dried (under vacuum) to afford 3.15 g (99%) of crude (2R)-2-(3,5-di-*t*-butylphenyloxy)propanoyl chloride (11) as yellowish liquid: ¹H NMR (CDCl₃) δ 1.30 (s, 18H), 1.73-1.76 (d, 3H), 4.92-4.98 (q, 1H), 6.72 (s, 2H), 7.10 (s, 1H); ¹³C NMR (CDCl₃) δ 175.0, 156.5, 152.7, 116.7, 109.7, 79.8, 35.0, 31.4, 18.1.

### STEP E ACYLATION AND SEPARATION

### N-Acylation of 5-Vinylpyrrolidin-2-one with (2R)-2-(3,5-Di-t-butylphenyloxy)propanoyl chloride (11)

To 60% sodium hydride (336 mg, 10 mmol) in toluene (20 mL) at 0°C was added dropwise a solution of 5-vinyl-pyrrolidin-2-one (1.0 g, 9.0 mmol) in toluene (10 mL) over a 5 min period. The cooling bath was removed and the solution was allowed to warm to 25°C to ensure complete generation of the anion. To the anion at 25°C was added dropwise a solution of (2R)-2-(3,5-di-t-butylphenyloxy)propanoyl chloride (11, 3.20 g, 9.8 mmol) in toluene (10 mL) over a 5 min period. TLC indicated that the reaction was complete upon addition of the acid chloride (silica gel plates; hexane and ethyl acetate, 2:1). Water (60 mL) was added to the reaction mixture and the phases were separated. The aqueous phase was extracted with ethylene chloride (1x40 mL). The combined organic phases were washed with a saturated aqeous sodium bicarbonate solution (lx70 mL) and dried (MgSO₄). Concentration and purification by flash chromatography on silica gel (EM silica gel 60, 0.040-0.063 partical size, 230-400 Mesh, column size: 6.5 cm diameter X 18 cm length) using hexane and ethyl acetate (19:1) afforded 1.65 g (46%) of (5S)-N-((2S)-2-(3,5-di-t-butylphenyloxy)propanoyl)-5-ethenylpyrrolidin-2-one (15a) as an oil: ¹H NMR (CDCl₃) δ 1.20 (s, 18H), 1.60-1.62 (d, 3H), 1.92-2.04 (m, 1H), 2.21-2.32 (m, 1H), 2.52-2.80 (m, 2H), 4.9-4.96 (q, 1H), 5.11-5.21 (m, 2H), 5.77-5.88 (m, 1H), 5.98-6.04 (q, 1H), 6.71 (s, 2H), 7.00 (s, 1H); ¹³C NMR (CDCl₃) δ 175.2, 172.7, 157.1, 152.1, 135.2, 115.9, 115.6, 109.6, 72.3, 58.0, 34.9, 31.6, 31.4, 24.4, 18.1; IR (neat) 2966 (vs), 1740 (vs), 1715 (vs), 1593 (s), 1380 (s), 1229 (vs), 706 (w) cm⁻¹.

### STEP F HYDROLYSIS

### (5S)-5-Ethenylpyrrolidin-2-one (6) from (5S)-N-((2S)-2-(3,5-Di-t-butylphenyloxy)propanoyl-5-ethenylpyrrolidin-2-one (15a)

A solution of diastereomer 15a (1.5 g, 3.8 mmol), potassium carbonate (233 mg, 1.7 mmol), and methanol (20 mL) is stirred at room temperature for 1.5 hours. The solvent is removed by rotary evaporation to afford a thick yellow oil. This oil is dissolved in methylene chloride (10 mL) and filtered through a silica gel plug (20 g, EM-60, 70-230 mesh) to remove any salts. The plug is rinsed with hexane/ethyl acetate (4:1), discarding the initial eluent which contains the chiral auxiliary, to give (5S)-5-ethenylpyrrolidin-2-one (6).

### EXAMPLE 4

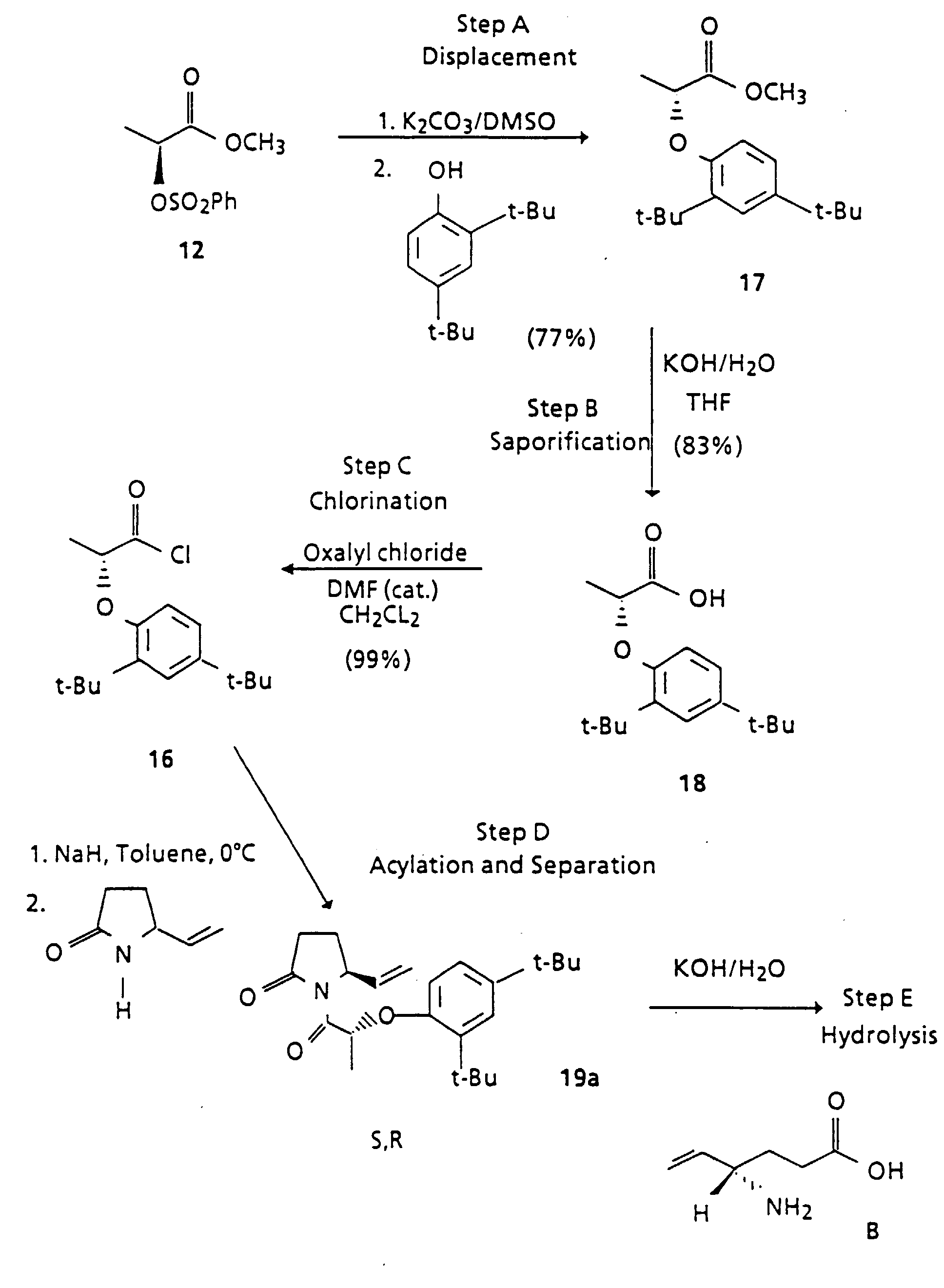

### STEP A DISPLACEMENT

### Methyl (2R)-2-(2,4-Di-t-butylphenyloxy)propanate (17)

Potassium carbonate (5.66 g, 41 mmol) and 2,4-di-*t*-butylphenol (8.87 g, 43 mmol) were dissolved in dimethyl sulfoxide (175 mL) at 25°C and allowed to stir overnight under a nitrogen atmosphere to ensure complete anion formation. A solution of 10.0 g (41 mmol) of methyl (2S)-2-(phenylsulfonyl)oxypropanate(12) in dimethyl sulfoxide (50 mL) was added to the anion at 25°C and the reaction mixture was allowed to stir overnight. The reaction was quenched with water (200 mL) and the phases were separated. The aqueous phase was extracted with perchloroethylene (3x200 mL). The combined organic extracts were washed with water (3x300 mL), a saturated aqueous sodium chloride solution (1x300 mL) and dried (MgSO₄). Concentration and purification by flash chromatography on silica gel (EM silica gel 60, 0.040-0.063 partical size, 230-400 Mesh, column size: 7 cm diameter X 18 cm length) using hexane and ethyl acetate (29:1) afforded 10.1 g (77%) of methyl (2R)-2-(2,4-di-*t*-butylphenyloxy)propanate(17) as a thick oil: ¹H NMR (CDCl₃) δ 1.29 (s, 9H), 1.43 (s, 9H), 1.60-1.65 (d, 3H), 3.73 (s, 3H), 4.75-4.82 (q, 1H), 6.54-6.57 (d, 1H), 7.08-7.12 (d, 1H), 7.33 (s, 1H); ¹³C NMR (CDCl₃) δ 172.9, 153.7, 143.0, 137.2, 124.2, 123.1, 110.5, 71.7, 52.0, 5.0, 34.2, 31.5, 29.9, 18.6; IR (neat) 2962 (vs), 1764 (s), 1740 (s), 1497 (s), 1235 (vs) cm-¹.

### STEP B DEPROTECTION

### (2R)-2-(2,4-Di-t-butylphenyloxy)propanoic Acid (18)

To a solution of 5.98 mg (11 mmol) of potassium hydroxide in water (75 mL) and tetrahydrofuran (75 mL) was added 3.34 mg (10 mmol) of methyl (2R)-2-(2,4-di-*t*-butylphenyloxy)propanate (17). The reaction mixture was allowed to stir overnight at room temperature. The reaction mixture was acidified to pH 2 with concentrated hydrochloric acid and the resulting solution was extracted with diethyl ether (3x200 mL). The combined extracts were washed with an aqueous saturated sodium chloride solution (1x300 mL) and dried (MgSO₄). Filtration and concentration afforded a thick crude oil. Purification by precipitation from hexane at -78°C afforded 2.65 g (83%) of (2R)-2-(2,4-di-t-butylphenyloxy)propanoic acid (18) as a white crystalline material: ¹H NMR (CDCl₃) δ 1.29 (s, 9H), 1.42 (s, 9H), 1.68-1.70 (d, 3H), 4.77-4.85 (q, 1H), 6.58-6.61 (d, 1H), 7.11-7.15 (d, 1H), 7.34 (s, 1H); ¹³C NMR (CDCl₃) δ 177.2, 153.5, 143.3, 137.3, 124.4, 123.3, 110.7, 71.4, 35.0, 34.3, 31.6, 30.0, 18.5.

### STEP C CHLORINATION

### (2R)-2-(2,4-Di-t-butylphenyloxy)propanoyl chloride (16)

To a mixture of 675 mg (2.2 mmol) of (2R)-2-(2,4-di-*t*-butylphenyloxy)propanoic acid (18) and 4 drops of N,N-dimethylformamide in methylene chloride (20 mL) at 0°C under a nitrogen atmophere was added 1.21 mL (2.4 mmol) of a 2M solution of oxalyl chloride in methylene chloride dropwise over a 10 min period. The reaction mixture was allowed to stir at room temperature overnight. The solvent and any excess oxalyl chloride was removed by rotary evaporation and dried (under vacuum) to afford 715 mg (99%) of (2R)-2-(2,4-di-*t*-butylphenyloxy)propanoyl chloride (16) as a crude yellow liquid: ¹H NMR (CDCl₃) δ 1.29 (s, 9H), 1.42 (s, 9H), 1.76-1.79 (d, 3H), 4.94-5.00 (q, 1H), 6.50-6.(d, 1H), 7.12-7.16 (d, 1H), 7.36 (s, 1H); ¹³C NMR (CDCl₃) δ 175.0, 152.8, 144.1, 137.4, 124.6, 123.4, 110.6, 78.6, 35.1, 34.3, 1.5, 30.0, 18.0.

### STEP D ACYLATION AND SEPARATION

### N-Acylation of 5-Vinylpyrrolidin-2-one with (2R)-2-(2,4-Di-t-butylphenyloxy)propanoyl chloride (16)

To 60% sodium hydride (74 mg, 2.2 mmol) in toluene (10 mL) at 0°C was added dropwise a solution of 5-vinylpyrrolidin-2-one (222 mg, 2.0 mmol) in toluene (5 mL) over a 5 min period. The cooling bath was removed and the solution was allowed to warm to 25°C to ensure complete generation of the anion. To the anion at 25°C was added dropwise a solution of (2R)-2-(2,4-di-*t*-butylphenyloxy)propanoyl chloride (16, 716 mg, 2.2 mmol) in toluene (5 mL) over a 5 min period. TLC indicated that the reaction was complete upon addition of the acid chloride (silica gel plates; hexane and ethyl acetate, 4:1). Water (30 mL) was added to the reaction mixture and the phases were separated. The aqueous phase was extracted with methylene chloride (1x25 mL). The combined organic phases were washed with a saturated aqeous sodium bicarbonate solution (1x50 mL) and dried (MgSO₄). Concentration and purification by flash chromatography on silica gel (EM silica gel 60, 0.040-0.063 partical size, 230-400 Mesh, column size: 4.5 cm diameter X 15 cm length) using hexane and ethyl acetate (19:1) afforded 360 mg (45%) of (5S)-N-((2R)-2-(2,4-di-t-butylphenyloxy)propanoyl)-5-ethenylpyrrolidin-2-one (19a) as a thick oil: ¹H NMR (CDCl₃) δ 1.28 (s, 9H), 1.42 (s, 9H), 1.77-1.80 (d, 3H), 1.95-2.10 (m, 1H), 2.23-2.33 (m, 1H), 2.52-2.82 (m, 2H), 4.91-4.97 (q, 1H), 5.10-5.19 (m, 2H), 5.80-5.90 (m, 1H), 5.97-6.03 (m, 1H), 6.49-6.53 (d, 1H), 7.12-7.16 (d, 1H), 7.35 (s, 1H); ¹³C NMR (CDCl₃) δ 175.4, 172.9, 157.3, 153.4, 143.1, 137.2, 135.6, 124.3, 123.2, 115.8, 71.6, 57.9, 35.2, 34.2, 31.9, 31.4, 29.9, 24.3, 18.2; IR (neat) 2965 (vs), 1741 (vs), 1715 (vs), 1590 (s), 1230 (vs), 710 (w) cm-¹.

### STEP E HYDROLYSIS

### (4S)-Amino-5-hexenoic Acid (B) from (5S)-N-((2R)-2-(2,4-Di-t-butylphenyloxy)propanoyl)-5-ethenylpyrrolidin-2-one (19a)

A solution of (5S)-N-((2R)-2-(2,4-di-t-butylphenyloxy)-propanoyl)-5-ethenylpyrrolidin-2-one (19a, 1.0 g, 2.5 mmol) and 87% potassium hydroxide (317 mg, 5.7 mmol) in water (2 mL) is refluxed for 1.5 hours. The solution is allowed to cool to room temperature. Water is added (15 mL) and the solution is acidified to pH 7 with concentrated hydrochloric acid and extracted with diethyl ether (3 x 10 mL) to remove the chiral auxiliary. The aqueous solution is then acidified to pH 5.5 with concentrated hydrochloric acid and extracted with diethyl ether (3 x 15 mL). The second series of organic extracts are combined and dried (MgSO₄). Concentration by rotary evaporation affords (4S)-4-amino-5-hexenoic acid (B).

### EXAMPLE 5

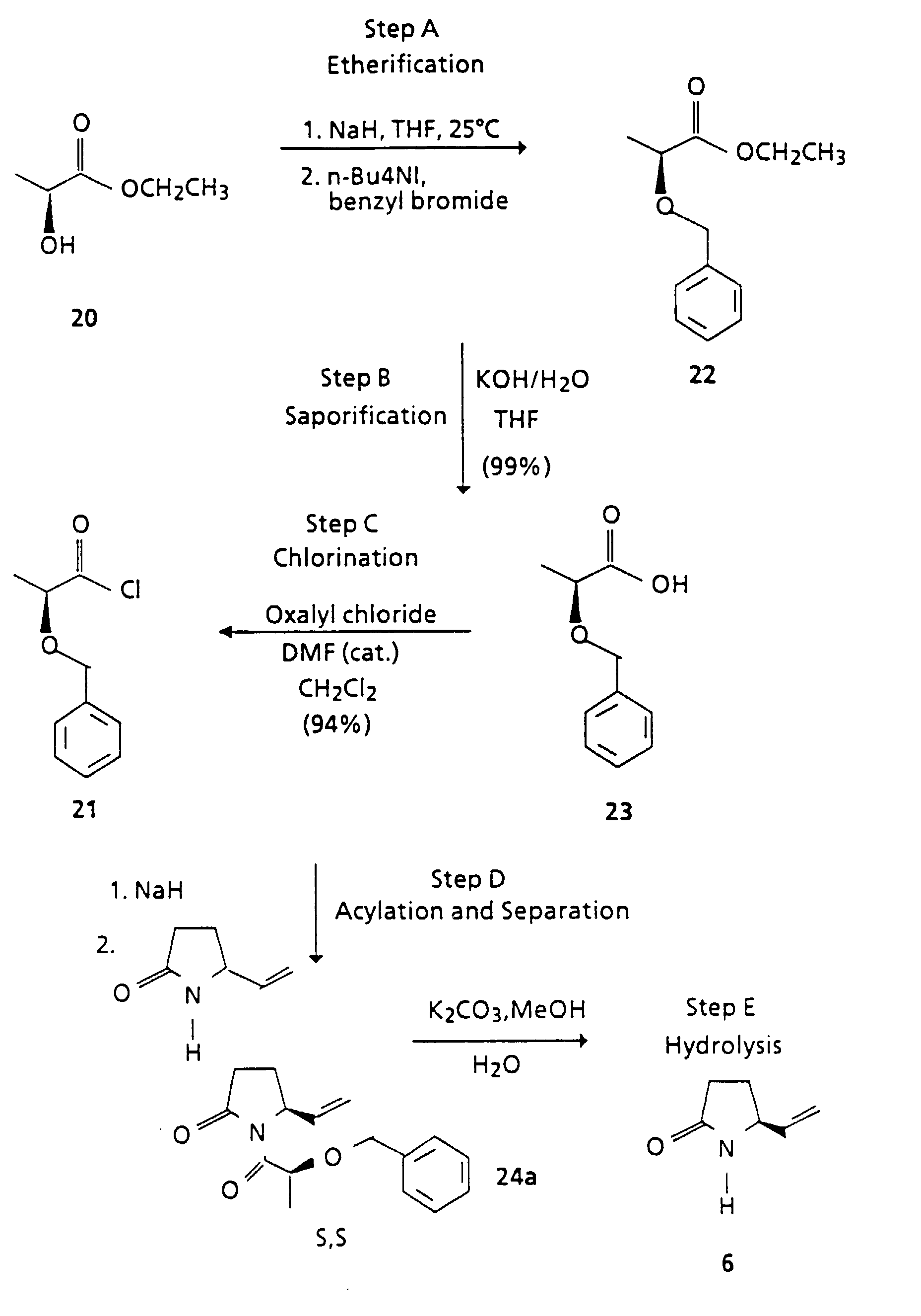

### STEP A ETHERIFICATION

### Ethyl (2S)-2(2S)-2-benzoylpropanate (22)

To a slurry of 3.13 g (93 mmol) of sodium hydride (60% in mineral oil) in tetrahydrofuran (150 mL) at 25°C was added a solution of 10.0 g (85 mmol) of (S)-ethyl lactate (20) in tetrahydrofuran (50 mL) dropwise over a 30 min period under a nitrogen atmosphere. The resulting solution was then allowed to stir at 25°C for an additional 30 min to ensure complete anion formation. To the anion was added 33.2 g (90 mmol) of tetra-n-butylammonium iodide. A solution of 10.3 mL (93 mmol) of benzyl bromide in tetrahydrofuran (50 mL) was added dropwise to the reaction mixture over a 30 min period. The reaction was complete within 2 h at 25°C, during which time a solid precipitate formed. The reaction mixture was filtered through a coarse sintered glass funnel containing Celite. The solid was washed with diethyl ether (50 mL). The filtrate was washed with a saturated aqueous sodium chloride solution (3x300 mL) and dried (MgSO₄). Concentration and purification by flash chromatography on silica gel (EM 60, 0.040-0.063 partical size, 230-400 Mesh, column size: 7 cm diameter X 20 cm length) using hexane and ethyl acetate (9:1) afforded 13.2 g (75%) of ethyl (2S)-2-benzoylpropanate (22) as a yellowish liquid: ¹H (CDCl₃) δ 1.26-1.31 (t, J=7.1 Hz, 3H), 1.42-1.44 (d, J=6.8 Hz, 2H), 4.01-4.12 (m, 1H), 4.17-4.24 (q, J=7.0 Hz, 2H), 4.42-4.46 (d, J=11.6 Hz, 1H), 4.67-4.71 (d, J=11.6 Hz, 1H), 7.28-7.38 (m, 5H); ¹³C NMR (CDCl₃) δ 173.0, 137.6, 128.3, 127.8, 127.7, 74.1, 71.9, 60.6, 18.5, 14.1; IR (neat) 2985 (s), 1746 (vs), 1455 (s), 1200 (s), 1144 (vs), 698 (vs) cm⁻¹.

### STEP B DEPROTECTION

### (2S)-2(2S)-2-Benzoylpropanonic Acid (23)

To a solution of 4.2 g (74 mmol) of potassium hydroxide in water (150 mL) and tetrahydrofuran (150 mL) was added 10.9 g (52 mmol) of ethyl (2S)-2-benzoylpropanate (22). The reaction mixture was allowed to stir overnight at room temperature. The reaction solution was acidified to pH 2 with concentrated hydrochloric acid and and extracted with diethyl ether (3x150 mL). The combined extracts were washed with an aqueous saturated sodium chloride solution (1x300 mL) and dried (MgSO₄). Filtration and concentration afforded 9.4 g (99%) of crude (2S)-2-benzoylpropanonic acid (23) as an oil: ¹H NMR (CDCl₃) δ 1.45-1.48 (d, J=6.9 Hz, 3H), 4.05-4.12 (q, J=6.9 Hz, 1H), 4.45-4.49 (d, J=11.6 Hz, 1H), 4.69-4.73 (d, J=11.6 Hz, 1H), 7.27-7.35 (m, 5H), 9.14 (br, 1H); ¹³C NMR (CDCl₃) δ 177.8, 137.1, 128.3, 127.8, 73.4 71.9, 18.2; IR (neat) 3855-2615 (br), 2989 (s), 1719 (vs), 1457 (s), 1115 (vs), 737 (w), 698 (s) cm⁻¹.

### STEP C CHLORINATION

### (2S)-2(2S)-2-Benzoylpropanoyl Chloride (21)

To a mixture of 9.0 g (50 mmol) of (2S)-2-benzoylpropanonic acid (23) and 5 drops of N,N-dimethylformamide in methylene chloride (200 mL) at 0°C under a nitrogen atmosphere was added 27.5 mL (55 mmol) of a 2M solution of oxalyl chloride in methylene chloride dropwise over a 30 min period. The reaction mixture was allowed to stir at room temperature overnight. The solvent and any excess oxalyl chloride was removed by rotary evaporation and dried (under vacuum) to afford 10.5 g (98%) of (2S)-2-benzoylpropanoyl chloride (21) as a crude yellow liquid: ¹H NMR (CDCl₃) δ 1.51-1.53 (d, J=6.8 Hz,3H), 4.22-4.28 (q, J=6.8 Hz, 1H), 4.41-4.44 (d, J=11.4 Hz, 1H), 4.70-4.73 (d, J=11.4 Hz, 1H), 7.33-7.35 (m, 5H); IR (neat) 3067 (w), 3035 (s), 2992 (w), 2875 (w), 1831 (vs), 1781 (vs), 1454 (vs), 1154 (s), 735 (s), 699 (s) cm⁻¹.

### STEP D ACYLATION AND SEPARATION

### N-Acylation of 5-Vinylpyrrolidin-2-one -2-one with (2S)-2-Benzoylpropanoyl Chloride (21)

To 60% sodium hydride (336 mg, 10.0 mmol) in toluene (20 mL) at 0°C was added dropwise a solution of 5-vinylpyrrolidin-2-one (1.0 g, 9.0 mmol) in toluene (10 mL) over a 10 min period. The cooling bath was removed and the solution was allowed to warm to 25°C to ensure complete generation of the anion. To the anion at 25°C was added dropwise a solution of (2S)-2-benzoylpropanoyl chloride (21, 2.7 g, 12.6 mmol) in toluene (10 mL) over a 10 min period. TLC indicated that the reaction was complete upon addition of the acid chloride (silica gel plates; hexane and t-butyl methyl ether, 1:1). Water (50 mL) as added to the reaction mixture and the phases were separated. The aqueous phase was extracted with diethyl ether (2x40 mL). The combined organic phases were washed with a saturated aqeous sodium bicarbonate solution (lx100 mL) and dried (MgSO₄). Concentration and purification by flash chromatography on silica gel (EM 60, 0.040-0.063 partical size, 230-400 Mesh, column size: 6.5 cm diameter X 20 cm length) using hexane and t-butyl methyl ether (10:1) afforded 1.1 g (42%) of (5S)-N-((2S)-2-benzoylpropanoyl)-5-ethenylpyrrolidin-2-one (24a) as a thick oil: ¹H NMR (CDCl₃) δ 1.44-1.46 (d, J=6.5 Hz ,3H), 1.64-1.80 (m, 1H), 2.12-2.20 (m, 1H), 2.42-2.84 (m, 2H), 4.12-4.21 (q, J=6.4 Hz, 1H), 4.38-4.47 (m, 1H), 4.58-4.67 (m, 1H), 4.89-4.91 (m, 1H), 5.06-5.19 (m, 2H), 5.77-5.85 (m, 1H), 7.28-7.40 (m, 5H); ¹³C NMR (CDCl₃) δ 174.8, 173.5, 137.7, 135.3, 128.0, 127.7, 127.4, 115.1, 74.6, 71.6, 31.3, 24.0, 18.1; IR (neat) 3066 (w), 3033 (w), 2939 (w), 1740 (vs), 1708 (vs), 1455 (s), 1370 (s), 1277 (s), 1148 (s), 1113 (s), 739 (s) cm⁻¹.

### STEP E HYDROLYSIS

### (5S)-5-Ethenylpyrrolidin-2-one (6) from (5S)-N-((2S)-2-benzoylpropanoyl)-5-ethenylpyrrolidin-2-one (24a)

To a solution of 1.0 g (3.5 mmol) of (5S)-N-((2S)-2-benzoylpropanoyl)-5-ethenylpyrrolidin-2-one (24a) in methanol (20 mL) and water (5 mL) is added 240 mg (1.7 mmol) of potassium carbonate. The resulting reaction mixture is stirred at 25°C for 1 hour. An additional 20 mL of water is added and the solution is extracted with methylene chloride (3x25 mL). The combined organic extracts are dried over magnesium sulfate. Concentration and purification by flash chromatography on silica gel using hexane and ethyl acetate (9:1) affords (5S)-5-ethenylpyrrolidin-2-one (6).

### EXAMPLE 6

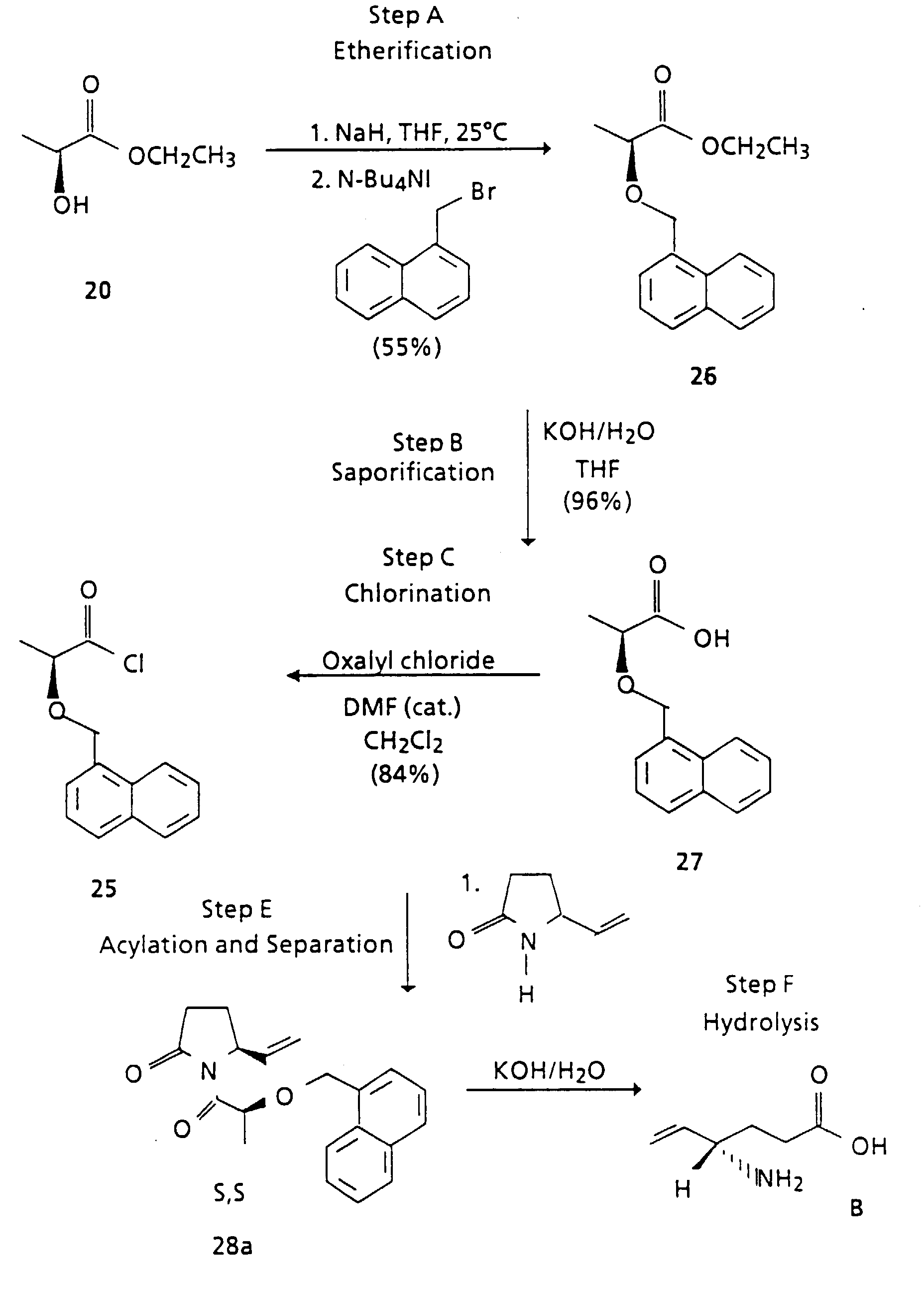

### STEP A ETHERIFICATION

### Ethyl (2S)-2-(α-(1-Methylnaphthlenyloxy)propanate (26)

To a slurry of 3.7 g (110 mmol) of sodium hydride (60% in mineral oil) in tetrahydrofuran (100 mL) at 25°C was added a solution of 12.0 g (102 mmol) of (S)-ethyl lactate (20) in tetrahydrofuran (50 mL) dropwise over a 30 min period under a nitrogen atmosphere. The resulting solution was then allowed to stir at 25°C for an additional 1 h to ensure complete anion formation. To the anion was added 40.6 g (110 mmol) of tetra-n-butylammoniun iodide. A solution of 25.0 g (113 mmol) of 1-(α-bromomethyl)naphthalene in tetrahydrofuran (100 mL) was added dropwise to the reaction mixture over a 5 min period. The reaction was complete within 72 h (over the weekend) at 25°C, during which time a solid precipitate formed. The reaction mixture was filtered through a coarse sintered glass funnel containing Celite®. The solid was washed with diethyl ether (50 mL). The filtrate was washed with a saturated aqueous sodium chloride solution (2x300 mL) and dried (MgSO₄). Concentration and purification by flash chromatography on silica gel (EM silica gel 60, 0.040-0.063 partical size, 230-400 Mesh, column size: 7 cm diameter x 20 cm length) using hexane and ethyl acetate (9:1) afforded 14.5 g (55%) of ethyl (2S)-2-(α-(1-methylnaphthlenyloxy)propanate (26) as an oil: ¹H NMR (CDCl₃) δ 1.31-1.36 (t, J=7.2 Hz, 3H), 1.48-1.51 (d, J=6.7 Hz, 3H), 4.13-4.20 (q, J=6.7 Hz, 1H), 4.24-4.31 (q, J=7.1 Hz, 2H), 4.85-4.88 (d, J=11.5 Hz, 1H), 5.25-5.29 (d, J=11.5 Hz, 1H), 7.39-7.62 (m, 4H), 7.83-7.90 (m, 2H), 8.32-8.35 (d, J=8.3 Hz, 1H); ¹³C NMR (CDCl₃) δ 173.1, 133.8, 133.1, 131.9, 128.8, 128.4, 126.7, 126.2, 125.7, 125.1, 124.3, 74.1, 70.5, 60.7, 18.7, 14.1; IR (neat) 3051 (w), 2985 (w), 2940 (w), 1744 (vs), 1200 (s), 1144 (vs), 1021 (s), 777.6 (vs) cm-¹.

### STEP B DEPROTECTION

### (2S)-2-(α-(1-Methylnapthlenyloxy)propanonic Acid (27)

To a solution of 4.2 g (74 mmol) of potassium hydroxide in water (150 mL) and tetrahydrofuran (150 mL) was added 13.5 g (52 mmol) of ethyl (2S)-2-(α-(1-methylnaphthlenyloxy)propanate (26). The reaction mixture was allowed to stir overnight at room temperature. The reaction solution was acidified to pH 2 with concentrated hydrochloric acid and and extracted with diethyl ether (3x200 mL). The combined extracts were washed with an aqueous saturated sodium chloride solution (1x300 mL) and dried (MgSO₄). Filtration and concentration afforded 11.6 g (96%) of crude (2S)-2-(α-(1-methylnapthlenyloxy)propanonic acid (27) as an orange oil: ¹H NMR (CDCl₃) δ 1.45-1.48 (d, J=6.9 Hz, 3H), 4.11-4.18 (q, J=6.9 Hz, 1H), 4.84-4.88 (d, J=11.6 Hz, 1H), 5.21-5.25 (d, J=11.6 Hz, 1H), 7.38-7.55 (m, 4H), 7.79-7.85 (m, 2H), 8.19-8.21 (d, J=8.2 Hz, 1H); ¹³C NMR (CDCl₃) δ 176.9, 133.8, 132.7, 131.8, 128.9, 128.4, 126.9, 126.3, 125.8, 125.1, 124.0, 73.6, 70.5, 67.8, 25.4, 18.4; IR (neat) 3660-2600 (br), 3051 (s), 2987 (s), 1723 (vs), 1235 (s), 1169 (s), 1115 (vs), 801 (s), 778 (s) cm-¹.

### STEP C CHLORINATION

### (2S)-2-(α-(1-Methylnaphthlenyloxy)propanoyl Chloride (25)

To a mixture of 10.0 g (43 mmol) of (2S)-2-(α-(1-methylnapthlenyloxy)propanonic acid (27) and 5 drops of N,N-dimethylformamide in methylene chloride (150 mL) at 0°C under a nitrogen atmosphere was added 32.6 mL (65 mmol) of a 2M solution of oxalyl chloride in methylene chloride dropwise over a 1 h period. The reaction mixture was allowed to stir at room temperature overnight. The solvent and any excess oxalyl chloride was removed by rotary evaporation and dried (under vacuum) to afford 9.1 g (84%) of (2S)-2-(α-(1-methylnaphthlenyloxy)propanoyl chloride (25) as a crude yellow liquid: ¹H NMR (CDCl₃) δ 1.58-1.60 (d, J=6.8 Hz, 3H), 4.38-4.44 (q, J=6.8 Hz, 1H), 4.87-4.91 (d, J=11.6 Hz, 1H), 5.31-5.35 (d, J=11.6 Hz, 1H), 7.48-7.67 (m, 4H), 7.91-7.96 (m, 2H), 8.23-8.26 (d, J=8.2 Hz, 1H); ¹³C NMR (CDCl₃) δ 175.8, 133.8, 132.0, 131.7, 129.3, 128.6, 127.2, 126.5, 126.0, 125.2, 123.9, 81.4, 70.9, 18.2; IR (neat) 3051 (w), 2941 (w), 1831 (vs), 1779 (vs), 1513 (s), 1445 (w), 1169 (s), 1109 (s), 910 (s), 893 (s), 801 (vs), 778 (vs) cm⁻¹.

### STEP D ACYLATION AND SEPARATION

### N-Acylation of 5-Vinylpyrrolidin-2-one with (2S)-2-(α-(1-Methylnaphthlenyloxy)propanoyl Chloride (25)

To a solution of 60% sodium hydide (336 mg, 10.0 mmol) and toluene (20 mL) at 0°C was added dropwise a solution of 5-vinylpyrrolidin-2-one (1.0 g, 9.0 mmol) in toluene (10 mL) over a 10 min period. The cooling bath was removed and the solution was allowed to warm to 25°C to ensure complete generation of the anion. To the anion at 25°C was added dropwise a solution of (2S)-2-(α-(1-methylnaphthlenyloxy)propanoyl chloride (25, 2.5 g, 10.0 mmol) in toluene (10 mL) over a 10 min period. TLC indicated that the reaction was complete upon addition of the acid chloride (silica gel plates; hexane and t-butyl methyl ether, 1:1). Water (50 mL) was added to the reaction mixture and the phases were separated. The aqueous phase was extracted with diethyl ether (2x40 mL). The combined organic phases were washed with a saturated aqeous sodium bicarbonate solution (1x100 mL) and dried (MgSO₄). Concentration and purification by flash chromatography on silica gel (EM silica gel 60, 0.040-0.063 partical size, 230-400 Mesh, column size: 6.5 cm diameter X 20 cm length) using hexane and t-butyl methyl ether (10:1) afforded 1.1 g (38%) of (5S)-N-((2S)-2-(α-(1-methylnaphthlenyloxy)propanoyl)-5-ethenylpyrrolidin-2-one (28a) as a thick oil: ¹H NMR (CDCl₃) δ 1.49-1.51 (d, J=6.6 Hz, 3H), 1.89-1.96 (m, 1H), 2.19-2.30 (m, 1H), 2.51-2.54 (m, 1H), 2.61-2.68 (m, 1H), 4.88-4.84 (d, J=10.3 Hz, 1H), 4.93-4.97 (t, J=6.4 Hz, 1H), 5.12-5.30 (m, 4H), 5.80-5.87 (m, 1H), 7.41-7.60 (m, 4H), 7.82-7.88 (m, 2H), 8.29-8.31 (d, J=8.3 Hz, 1H); ¹³C NMR (CDCl₃) δ 174.9, 173.6, 135.4, 133.6, 133.4, 131.8, 128.6, 128.6, 126.7, 126.1, 125.6, 125.0, 124.3, 115.3, 74.8, 70.2, 57.7, 31.5, 42.1, 18.3; IR (neat) 3051 (w), 2985 (w), 1737 (vs), 1702 (vs), 1457 (w), 1229 (s), 1108 (s), 803 (s), 780 (s) cm⁻¹.

### STEP E HYDROLYSIS

### (4S)-Amino-5-hexenoic Acid (B) from (5S)-N-((2S)-2-(α-(1-Methylnaphthlenyloxy)propanoyl)-5-ethenylpyrrolidin-2-one (28a)

A solution of diastereomer 28a (1.0 g, 3.1 mmol) and 87% potassium hydroxide (392 mg, 7.0 mmol) in water (2 mL) is refluxed for 2 hours. The solution is allowed to cool to room temperature. Additional water is added (15 mL) and the solution is acidified to pH 7 with concentrated hydrochloric acid. Extraction with diethyl ether (3 x 15 mL) removes the cleaved chiral auxiliary. The aqueous phase is acidified to pH 5.5 with concentrated hydrochloric acid and extracted with diethyl ether (3 x 15 mL). The second series of organic extracts is combined and dried (MgSO₄). Concentration by rotary evaporation affords (4S)-amino-5-hexenoic acid (B).

### EXAMPLE 7

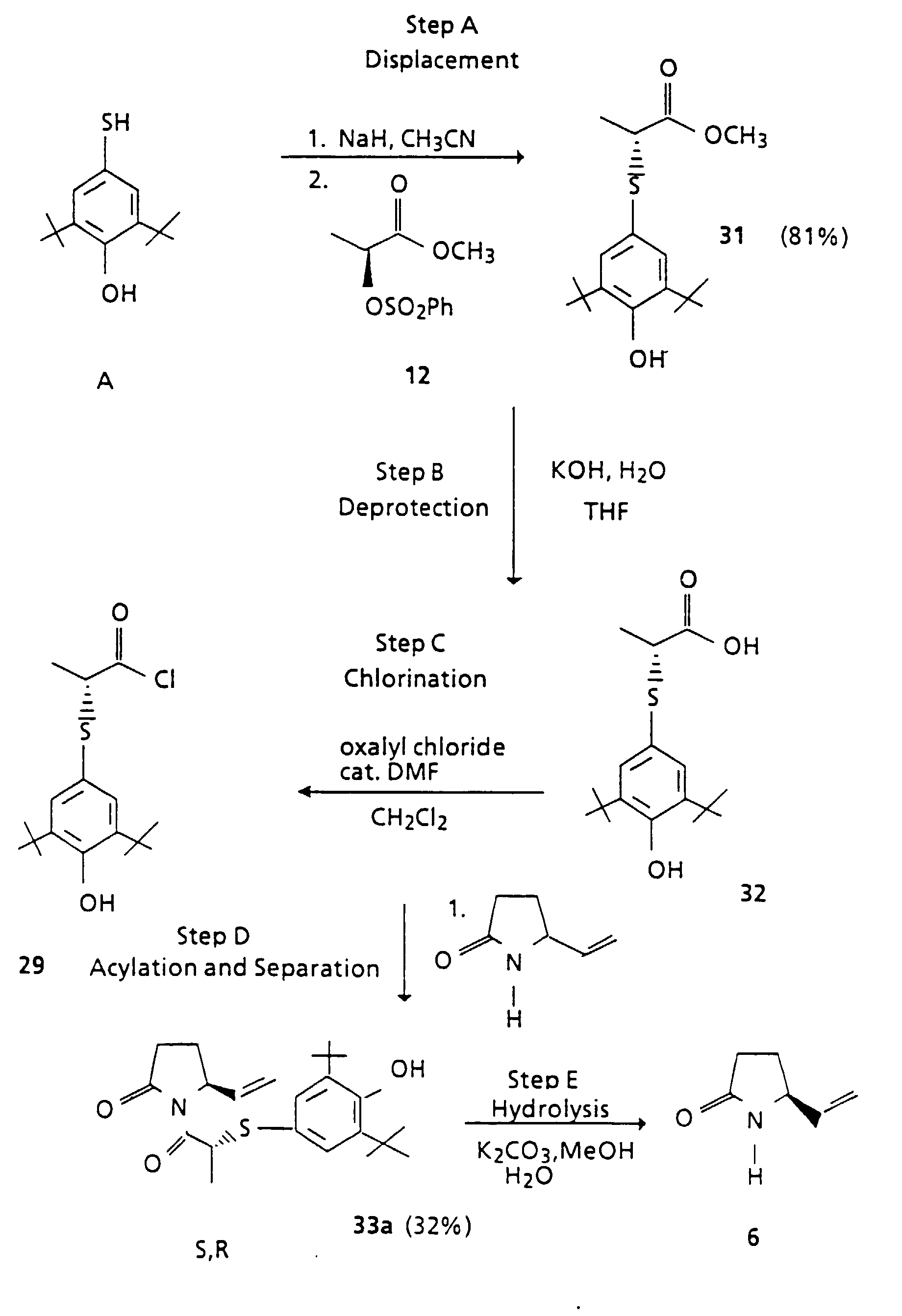

### STEP A DISPLACEMENT

### Methyl (2R)-2-(3,5-Di-t-butyl-4-hvdroxyphenyl)thiopropanoate (31)

To a solution of 60% sodium hydride in mineral oil (7.0 g, 0.20 mol) and acetonitrile (200 mL) under a nitrogen atmosphere was added dropwise a solution of 2,6-di-t-butyl-4-mercaptophenol (A) (53.7 g, 0.23 mol, 90%) in acetonitrile (100 mL) over a 1 h period. The reaction mixture was stirred at 25°C for an additional hour to ensure complete anion generation. To the mercaptophenol anion was added a solution of methyl (2S)-2-(phenylsulfonyloxy)propanate (50 g, 0.20 mol, 12) in acetonitrile (100 mL) dropwise over a 1 h period. Upon addition of methyl (2S)-2-(phenylsulfonyloxy)propanate a white precipitate was observed. The reaction mixture was allowed to stir at 25°C overnight. The mixture was worked-up by filtration through a coarse sintered glass funnel containing Celite®. The solvent was removed by rotary evaporation to afford a smelly yellow oil. Purification by flash chromatography on silica gel (EM silica gel 60, 0.040-0.063 partical size, 230-400 Mesh, column size: 10 cm diameter X 30 cm length) using hexane and ethyl acetate (9:1) afforded 51.3 g (81%) of methyl (2R)-2-(3,5-di-t-butyl-4-hydroxyphenyl)thiopropanoate (31) as a thick oil: ¹H NMR (CDCl₃) δ 1.42-1.45 (m, 21 H), 3.60-3.68 (q, J=7.2 Hz, 1H), 3.67 (s, 3H), 5.33 (s, -OH, 1H), 7.29 (s, 2H); ¹³C NMR (CDCl₃) δ 173.3, 154.6, 136.6, 131.8, 122.0, 52.0, 46.0, 34.3, 30.2, 17.4; IR (neat) 3635 (s), 2958 (vs), 2875 (s), 1737 (vs), 1426 (vs), 1237 (vs), 1160 (vs), 855 (w), 774 (w) cm⁻¹.

### STEP B DEPROTECTION

### (2R)-2-(3,5-Di-t-butyl-4-hydroxyphenyl)thiopropanoic Acid (32)

To a solution of 4.5 g (80 mmol) of potassium hydroxide in water (200 mL) and tetrahydrofuran (200 mL) was added 21.46 g (70 mmol) of methyl (2R)-2-(3,5-di-t-butyl-4-hydroxyphenyl)thiopropanoate (31). The reaction mixture was allowed to stir overnight at room temperature. The reaction solution was acidified to pH 1.2 with concentrated hydrochloric acid and and extracted with t-butyl methyl ether (3x100 mL). The combined extracts were washed with an aqueous saturated sodium chloride solution (1x300 mL) and dried (MgSO₄). Filtration, concentration and recrystallization from hexane and ethyl acetate afforded 18.35 g (90%) of (2R)-2-(3,5-di-t-butyl-4-hydroxyphenyl)thiopropanoic acid (32) as a white crystalline material: m.p. 111-112°C; ¹H NMR (CDCl₃) δ 1.33-1.46 (m, 21H), 3.58-3.65 (q, J=7.1 Hz, 1H), 5.34 (s, -OH, 1H), 7.33 (s, 2H), 9.12 (b, -CO₂H, 1H); ¹³C NMR (CDCl₃) δ 178.8, 154.8, 136.7, 131.8, 121.6, 46.0, 34.4, 30.2, 17.1; IR (KBr) 3608 (s), 3570 (s), 2960 (vs), 1692 (vs), 1424 (vs), 1287 (s), 1239 (vs), 1121 (vs), 884 (s), 774 (w) cm⁻¹.

### STEP C CHLORINATION

### (2R)-2-(3,5-Di-t-butyl-4-hydroxyphenyl)thiopropanoyl Chloride (29)

To a mixture of 10.0 g (43 mmol) of (2R)-2-(3,5-di-*t*-butyl-4-hydroxyphenyl)thiopropanoic acid (32) and 7 drops of N,N-dimethylformamide in methylene chloride (150 mL) at 0°C under a nitrogen atmophere was added 34.0 mL (68 mmol) of a 2M solution of oxalyl chloride in methylene chloride dropwise over a 1 h period. The reaction mixture was allowed to stir at room temperature overnight. The solvent and any excess oxalyl chloride was removed by rotary evaporation and dried (under vacuum) to afford 11.15 g (99.8%) of (2R)-2-(3,5-di-t-butyl-4-hydroxyphenyl)thiopropanoyl chloride (29) as a thick yellow liquid: ¹H NMR (CDCl₃) δ 1.43 (s, 18H), 1.47-1.49 (d, J=7.0 Hz, 3H), 3.83-3.90 (q, J=7.0 Hz, 1H), 5.29 (s, -OH, 1H), 7.32 (s, 2H); IR (neat) 3631 (vs), 2962 (vs), 2875 (s), 1773 (vs), 1426 (vs), 1239 (s), 1156 (s), 1123 (s), 918 (vs), 888 (w), 774 (w) cm⁻¹.

### STEP D ACYLATION AND SEPARATION

### N-Acylation of 5-Vinylpyrrolidin-2-one with (2R)-2-(3,5-Di-t-butyl-4-hydroxyphenyl)thiopropanyl chloride (29)

To a solution of 60% sodium hydride (665 mg, 19.0 mmol) and toluene (40 mL) at 0°C was added dropwise a solution of 5-vinylpyrrolidin-2-one(2.0 g, 18.0 mmol) in toluene (20 mL) over a 30 min period. The cooling bath was removed and the solution was allowed to warm to room temperature and stir there for a 30 min period to ensure complete anion generation. To the anion at 25°C was added dropwise a solution of (2R)-2-(3,5-di-t-butyl-4-hydroxyphenyl)thiopropanoyl chloride (29, 6.21 g, 19.0 mmol) in toluene (20 mL) over a 20 min period. TLC indicated that the reaction was complete upon addition of the acid chloride (silica gel plates; hexane and ethyl acetate, 2:1). Water (100 mL) was added to the reaction and the phases were separated. The aqueous phase was extracted with t-butyl methyl ether (2x100 mL). The combined organic phases were washed with a saturated aqueous sodium bicarbonate solution (1x250 mL) and dried (MgSO₄). Concentration and purification by flash chromatography on silica gel (EM 60, 0.040-0.063 partical size, 230-400 Mesh, column size: 4.5 cm diameter X 24 cm length) using hexane and ethyl acetate (10:1) afforded 2.32 g (32%) of (5S)-N-((2R)-2-(3,5-di-t-butyl-4-hydroxyphenyl)thiopropanoyl)-5-ethenylpyrrolidin-2-one (33a) as a white crystalline material: mp 105-106°C; ¹H NMR (CDCl₃) δ 1.37-1.42 (m, 21H), 1.79-1.86 (m, 1H), 1.97-2.05 (m, 1H), 2.32-2.41 (m, 1H), 2.57-2.67 (m, 1H), 4.71-4.75 (t, J=6.8 Hz, 1H), 5.03-5.17 (m, 3H), 5.33 (s, -OH, 1H), 5.78-5.87 (m, 1H), 7.23 (s, 2H); ¹³C NMR (CDCl₃) δ 174.7, 172.6, 154.7, 136.3, 135.7, 132.7, 120.9, 115.3, 58.8, 44.1, 34.3, 31.9, 30.2, 23.9, 16.4; IR (KBr) 3581 (s), 2964 (s), 1748 (vs), 1684 (vs), 1426 (s), 1345 (s), 1227 (vs), 1198 (s), 917 (w), 885 (w) cm-¹.

### STEP E HYDROLYSIS

### (5S)-5-Ethenylpyrrolidin-2-one (6) from (5S)-N-((2R)-2-(3,5-Di-t-butyl-4-hydroxyphenyl)thiopropanyl)-5-ethenylpyrrolidin-2-one (33a)

To a solution of 1.5 g (3.7 mmol) of (5S)-N-((2R)-2-(3,5-di-t-butyl-4-hydroxyphenyl)thiopropanyl)-5-ethenylpyrrolidin-2-one (33a) in methanol (25 mL) and water (5 mL) is added 257 mg (1.9 mmol) of potassium carbonate. The reaction mixture is allowed to stir at room temperature for 1.5 hours. Additional water (20 mL) is added and the solution is extracted with methylene chloride (3 x 40 mL). The combined organic extracts should be dried over magnesium sulfate. Concentration and purification by flash chromatography on silica gel (EM 60, 230-400 mesh) using hexane and ethyl acetate (9:1) affords (5S)-5-ethenylpyrrolidin-2-one (6).

### EXAMPLE 8

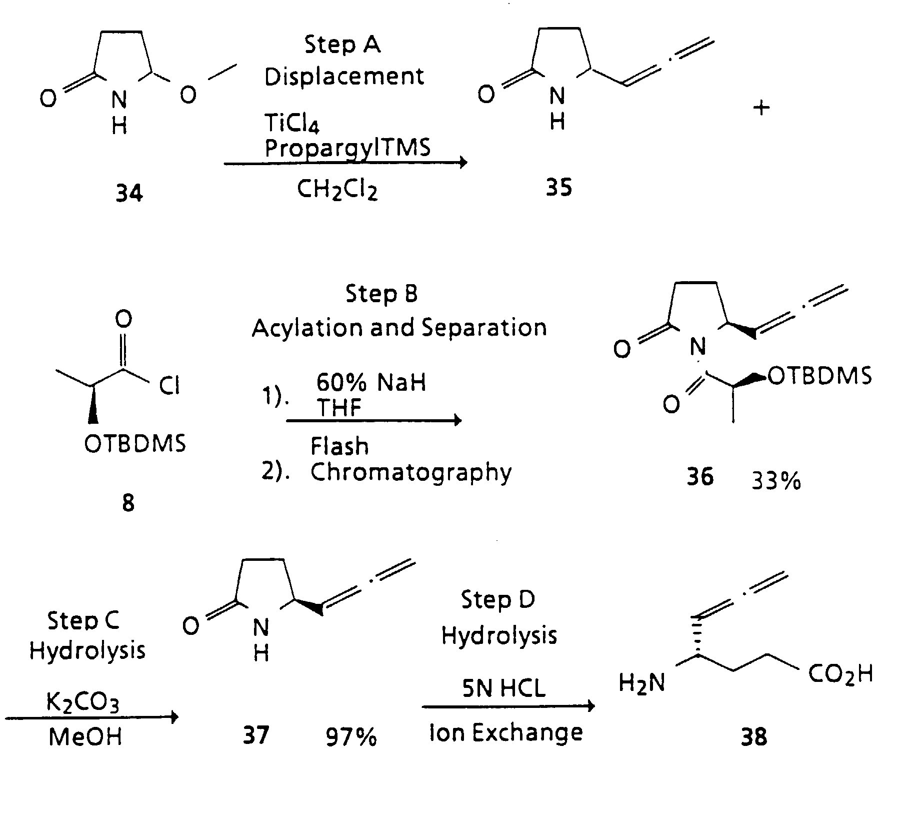

### STEP A DISPLACEMENT

### 5-(1,2-Propadienyl)pyrrolidin-2-one (35)

A solution of 5-methoxypyrrolidin-2-one (34) (2.55 g, 33.3 mmol), propargyl trimethylsilane (3.77 g, 33.7 mmol) and CH₂Cl₂ (100 mL) was cooled to 0°C. To this solution was added TiCl₄ (4.9 mL, 44.4 mmol) in CH₂Cl₂ (20 mL) over a 15 min period at ≤3°C. After stirring for 30 min, the reaction was poured through silica gel (30 g), rinsed with CH₂Cl₂ (100 mL), and this initial filtrate (120 mL) discarded. The silica gel was then washed with 5% MeOH/CH₂Cl₂ (100 mL) followed by 10% MeOH/CH₂Cl₂ (100 mL), and the combined filtrates were washed with water (50 mL). The aqueous layer was back extracted with CH₂Cl₂ (4X30 mL), and the combined organic layers dried over MgSO₄ and rotary evaporated (≤30°C) to give 5-(1,2-propadienyl)-2-pyrrolidinone (35) as a brown oil: TLC (Eluant A) R_{f}=0.45; ¹H NMR: δ 6.6 (br s, 1H, NH); 5.17 (ddd, ³J₆₄ ≃⁴J₆₈≃⁴J₆₈≃ 6.6 Hz, 1H, H6); 4.88 (d, ⁴J_{68'}=6.4, 1H, H8'); 4.21 (m, 1H, H5); 2.35 (m, 3H, H3, H4, H4'); 1.93 (m, 1H, H3'); Cl Mass spec: m/z 124 (MH⁺), 84 (MH⁺-C₃H₄).

### STEP B ACYLATION AND SEPARATION

### (5S)-N-((2S)-2-(1,1-Dimethylethyl)dimethylsilyloxy)propanoyl)-5-(1,2-propadienyl)pyrrolidin-2-one (36)

To a solution of NaH (80%, 1.60 g, 55.5 mmol) in THF (60 mL) at 0°C was added 5-(1,2-propandienyl)pyrrolidin-2-one (35) (2.73 g, 22.2 mmol) in THF (20 mL) over a 10 minute period. The mixture was allowed to warm to room temperature, and a solution of (2S)-2-((1,1-dimethylethyl)dimethylsilyloxy)propanoyl chloride (18) (7.4 g, 33.3 mmol) in THF (20 mL) was added over a 10 minute period. After stirring for 2 h at room temperature, the solvent was removed by rotary evaporation (≤30°C) to afford a brown oil. This oil was dissolved in CH₂Cl₂ (100 mL), washed with H₂O (30 mL) and saturated NaHCO₃ (30 mL) and dried over MgSO₄. Rotary evaporation (30°C) and purification by flash chromatography (15% EtOAc/Hexane, 200 mL silica gel) gave (5S)-N-((2S)-2-(1,1-dimethylethyl)dimethylsilyloxy)propanoyl)-5-(1,2-propadienyl)pyrrolidin-2-one (2.23 g, 33%) as a clear oil: TLC (eluant B) R_{f}=0.35; ¹H NMR: δ 5.36 (q, ³J_{H-Me}=6.5 Hz 1H, -C(O)CHMeOTBDMS); 5.30 (ddd, ³J₆₅=4.3,⁴J₆₈=6.4, ⁴J_{68'}=6.5, 1H, H6); 4.91 (m, 1H, H5); 4.86 (dd, ⁴J₈₆=6.4, ⁵J₈₅=0.8, 1H, H8); 4.85, ⁴J_{8'6}=6.5, ⁵J_{8'}5=1.2, 1H H8'); 2.67 (ddd, ²J_{33'}=17.6, ³J₃₄=11.4, ³J_{34'}=9.0, 1H, H3); 2.45 (ddd, ³J_{3'4}=8.7, ³J_{3'4}=2.4, 1H, H3'); 2.21 (dddd, ²J_{44'}≃12.5, ³J₄₃=11.4, ³J_{43'}=8.7, ³J₄₅≃8.5, 1H, H4); 2.03 (dddd, ³J_{4'3}=9.0, ³J_{43'}=2.4, ³J_{4'5}≃2.2, 1H, H4'), 1.34 (d, ³J_{H-Me}=6.6, 3H, -C(O)CHMeOTBDMS); 0.87 (s, 9H, -OSiMe₂t-butyl); 0.06 and 0.02 (s, 3H ea, -OSiMe₂t-butyl).

### STEP C HYDROLYSIS

### (5S)-5-(1,2-Propadienyl)pyrrolidin-2-one (37)

A solution of (5S)-N-((2S)-2-(1,1-dimethylethyl)dimethyl-silyloxy)propanoyl)-5-(1,2-propadienyl)pyrrolidin-2-one (2.70 g, 8.7 mmol), K₂CO₃ (0.6 g, 4.4 mmol), and MeOH (40 mL) was stirred at room temperature for 1.5 hours. The solvent was removed by rotary evaporation (≤30°C) to give a yellow gum. This gum was dissolved in CH₂Cl₂ (20 mL), filtered to remove salts and applied to a silica gel plug (50 g). This plug was rinsed with 10% MeOH/CH₂Cl₂, discarding the initial eluent which contains the chiral auxiliary, to give (5S)-5-(1,2-Propadienyl)pyrrolidin-2-one (1.04 g, 97%) as a clear oil: TLC (Eluent A) R_{f}=0.45; ¹H NMR: δ 6.6 (br s, 1H, NH); 5.17 (ddd, ³J₆₄≃⁴J₆₈≃⁴J₆₈≃6.6 Hz, 1H, H6); 4.88 (d, ⁴J₆₈≃6.7 Hz, 1H, H8); 4.88 (d, ⁴J_{68'}=6.4, 1H, H8'); 4.21 (m, 1H, H5); 2.35 (m, 3H, H3, H4, H4'); 1.93 (m, 1H, H3'); Cl Mass spec: m/z 124 (MH⁺), 84 (MH⁺-C₃H₄).

### STEP D HYDROLYSIS

### (4S)-4-Amino-5,6-heptadienoic acid (38)

A solution of (5S)-5-(1,2-propadienyl)pyrrolidin-2-one (37) (1.1g, 9.0 mmol), 5N HCl (10 mL) and H₂O (4 mL) was heated to 80°C for 18 hours. the solution was cooled, concentrated and neutralized to pH 6 with 1N NaOH. This solution was put on a Dowex 1X2-100 ion exchange column (OH) form. The column was rinsed with distilled H₂O (800 mL) and the compound was removed with 0.5 N acetic acid. Lyophilization gave (4S)-4-amino-5,6-heptadienoic acid as an off-white foamy solid (1.01 g, 80%).

## Claims (Claims for the following Contracting State(s): AT, BE, CH, DE, DK, FR, GB, GR, IT, LI, LU, NL, SE)

1. An optical isomer of the formula 3 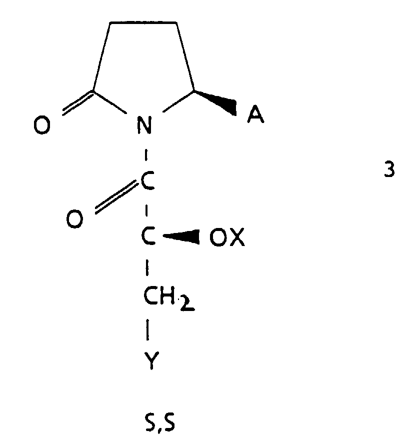 in which A is represented by -CH=CH₂ or-CH=C=CH₂ , and X and Y are each simultaneously represented by

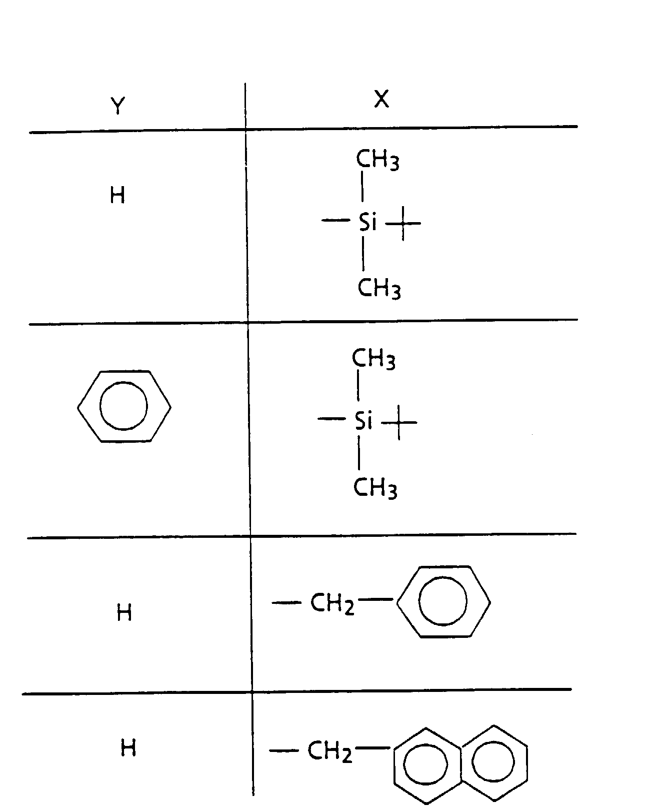

2. An optical isomer of the formula 3'' 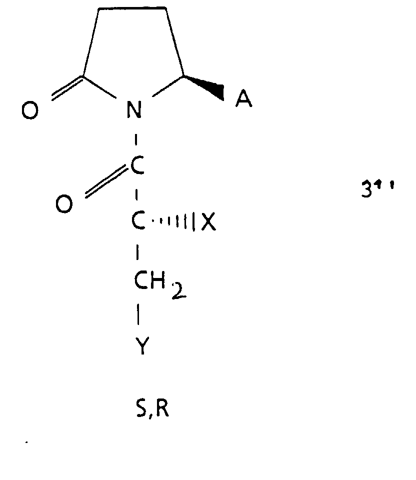 in which A is represented by -CH=CH₂ or -CH=C=CH₂ , and X and Y are each simultaneously represented by

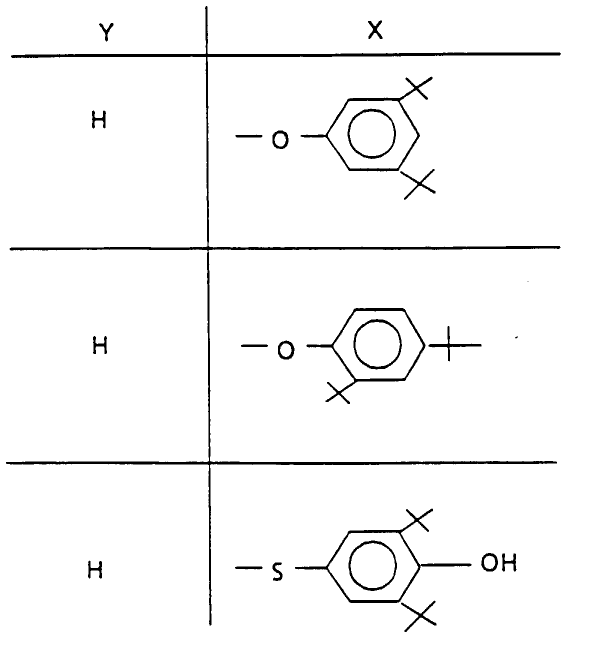

3. A process for producing an optical isomer according to claim 1 comprising separating diastereomers of the formula: 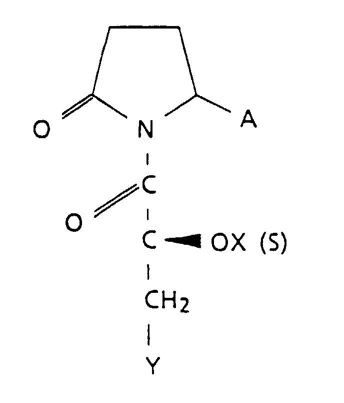 in which A, X, and Y are as defined above.

4. A process according to claim 3 in which said diastereomer is produced by carrying out an acylation reaction between a lactic acid derivative of the formula 2 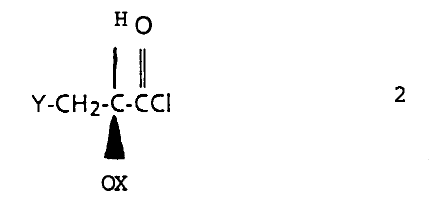 and a pyrrolidinone derivative of the formula 1 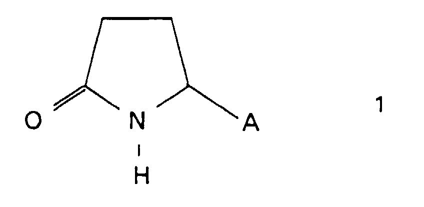 in which X, Y, and A are as above.

5. A process for producing a compound according to claim 2 comprising separating diastereomers of the formula: 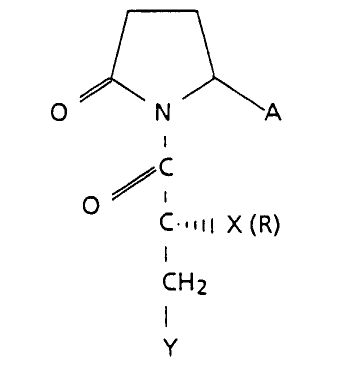 in which A, X, and Y are as above.

6. A process according to claim 5 in which said diastereomer is produced by carrying out an acylation reaction betwen a lactic acid derivative of the formula 6 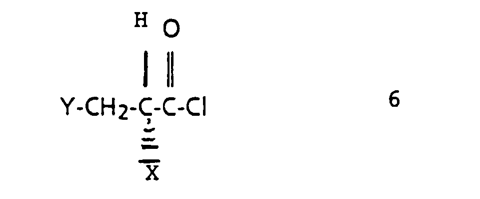 and a pyrrolidinone derivtive as described by the formula 1 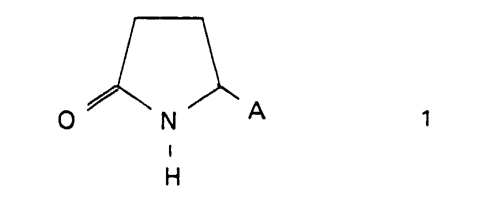 in which X, Y, and A are as above.

7. A process for producing an S-pyrrolidinone derivative of the formula 4 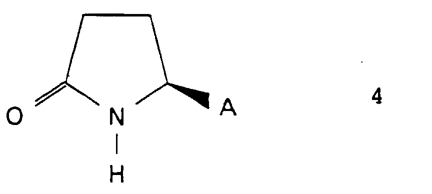 in which A is represented by -CH=CH₂ or -CH=C=CH₂ comprising:
a) separating diastereomers of the formula: 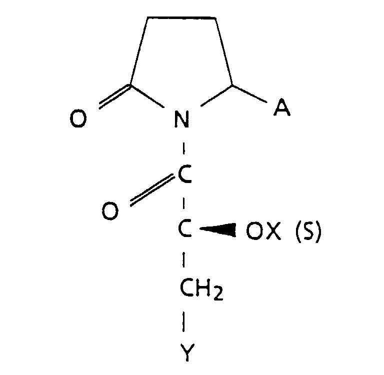 in which A is as above and X and Y are simultaneously represented by the following substituents; 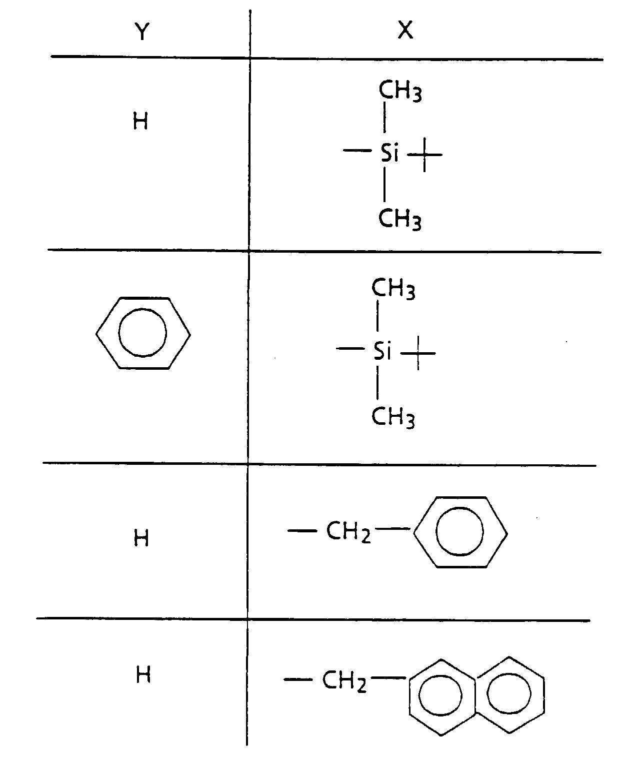 and
b) subjecting the resulting S,S isomer to a hydrolysis reaction.

8. A process for producing a compound of the formula 5 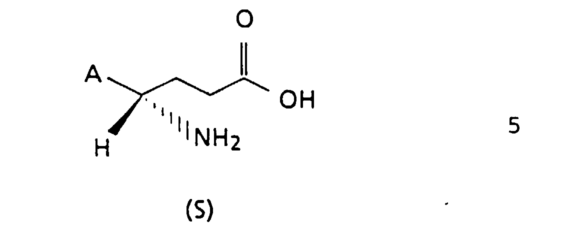 in which A is represented by CH₂=CH- or CH₂=C=CH- comprising:
a) separating diastereomers of the formula: 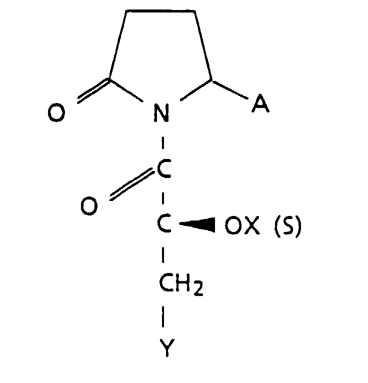 in which A is as above and X and Y are simultaneously represented by the following substituents; 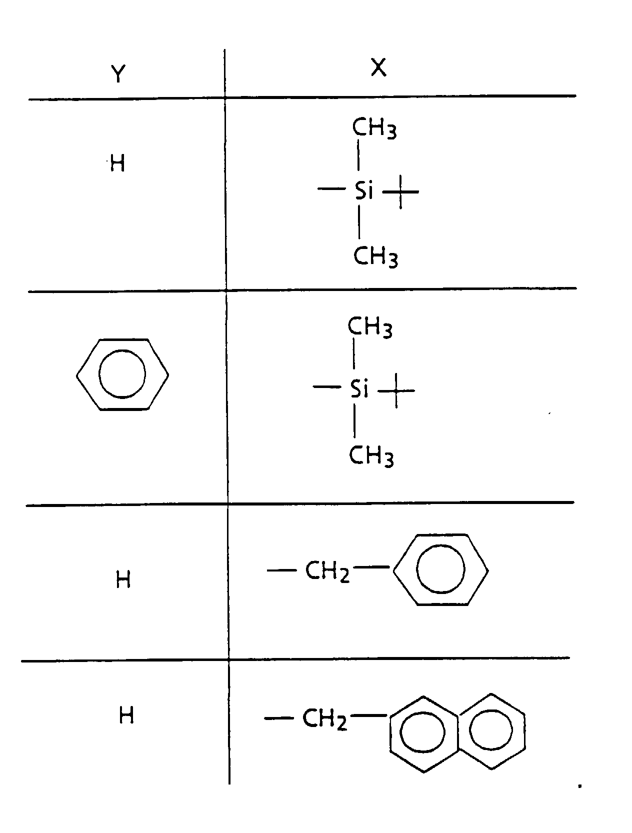 and
b) subjecting the resulting S,S isomer to a hydrolysis reaction.

9. A process for producing an S-pyrrolidinone derivative of the formula 4 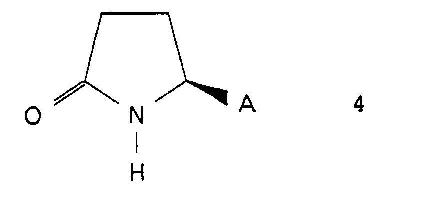 in which A is represented by -CH=CH₂ or -CH=C=CH₂ comprising:
a) separating diastereomers of the formula: 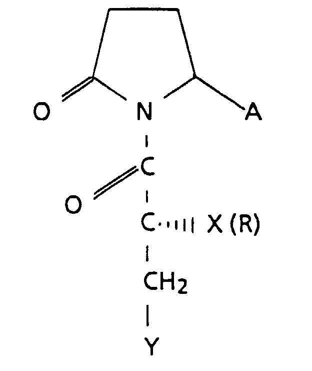 in which A is as above and X and Y are simultaneously represented by the following substituents, 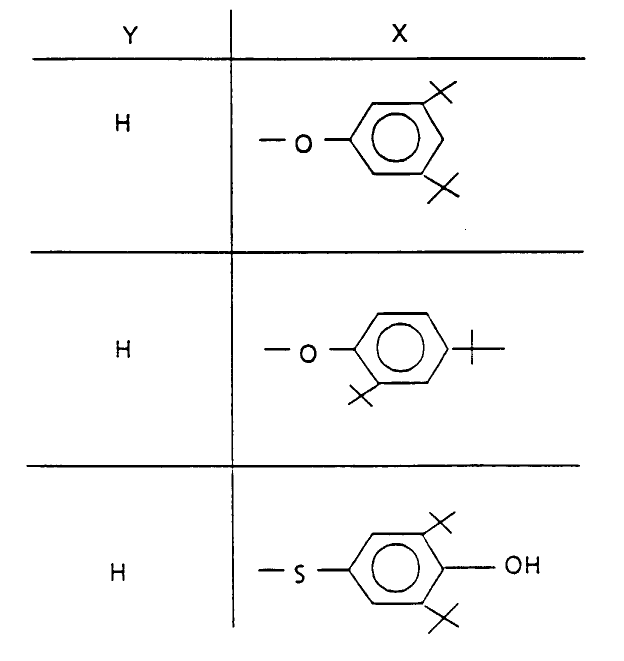 and
b) subjecting the resulting S,R isomer to a hydrolysis reaction.

10. A process for producing a compound of the formula 5 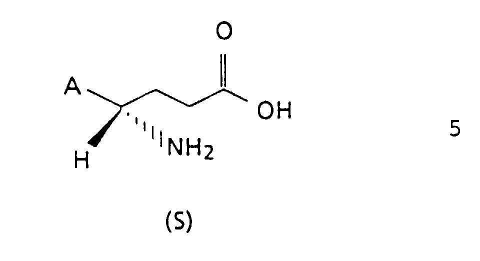 in which A is represented by CH₂=CH- or CH₂=C=CH- comprising:
a) separating diastereomers of the formula: 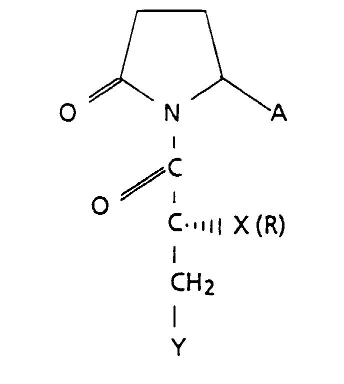 in which A is as above and X and Y are simultaneously represented by the following substituents; 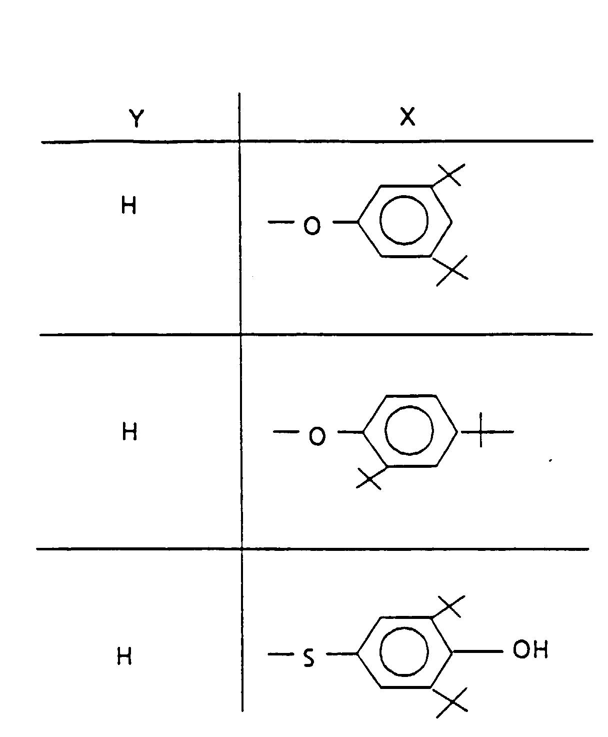 and
b) subjecting the resulting S,R isomer to a hydrolysis reaction.

11. A compound of the formula 6 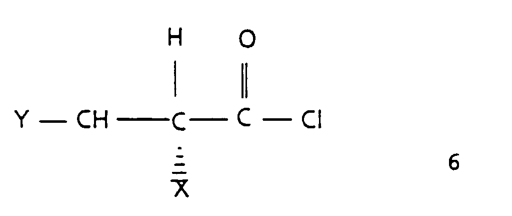 in which A is represented by -CH=CH₂ or -CH=C=CH₂, and X and Y are each simultaneously represented by

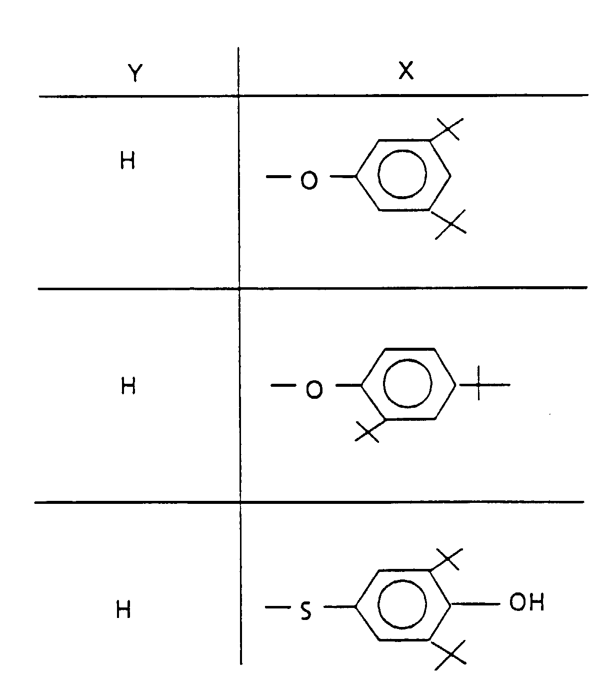

12. A compound of the formula 2 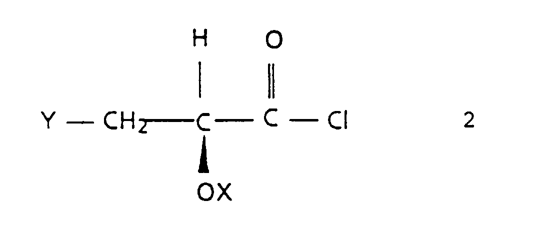 in which A is represented by -CH=CH₂ or -CH=C=CH₂, and X and Y are each simultaneously represented by

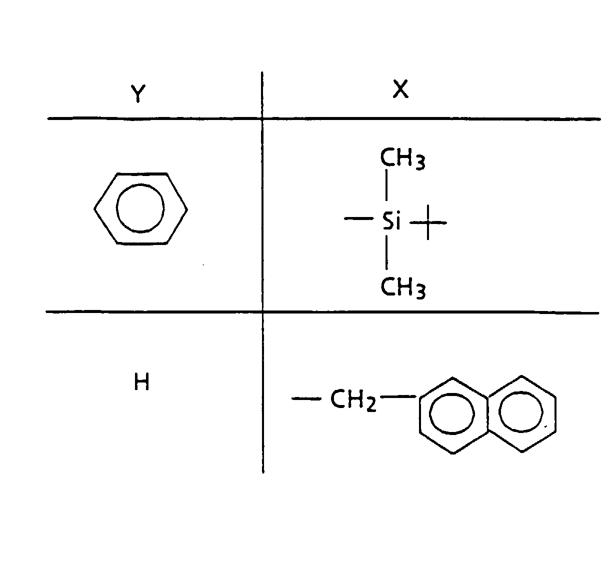

## Claims (Claims for the following Contracting State(s): ES)

1. A method for the preparation of an optical isomer of the formula 3 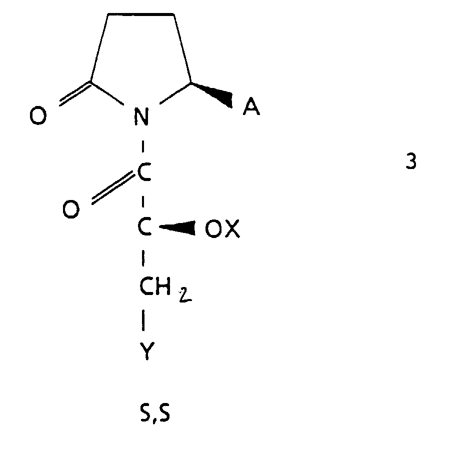 in which A is represented by -CH=CH₂ or -CH=C=CH₂,and X and Y are each simultaneously represented by 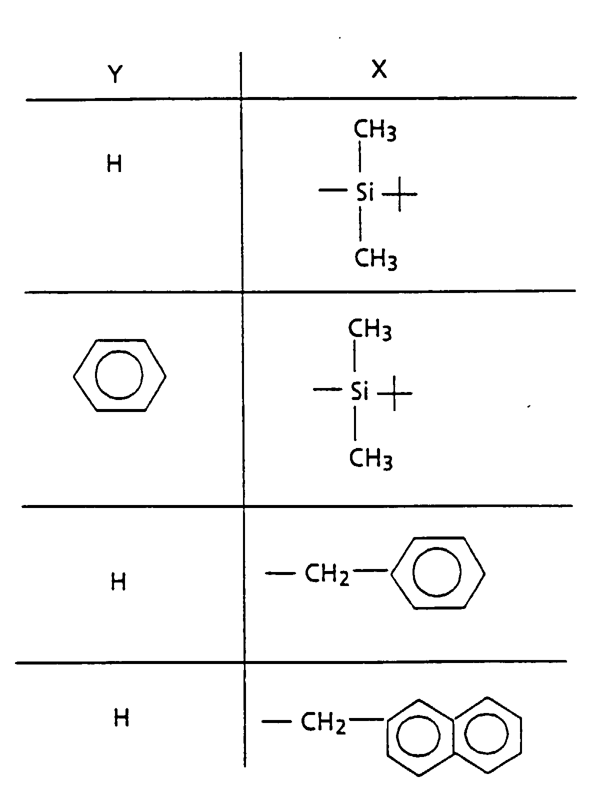 comprising separating diastereomers of the formula: 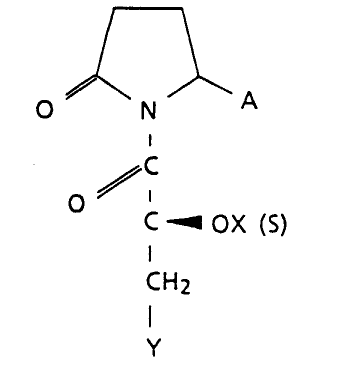 in which A, X and Y are as defined above.

2. A method for the preparation of an optical isomer of the formula 3'' 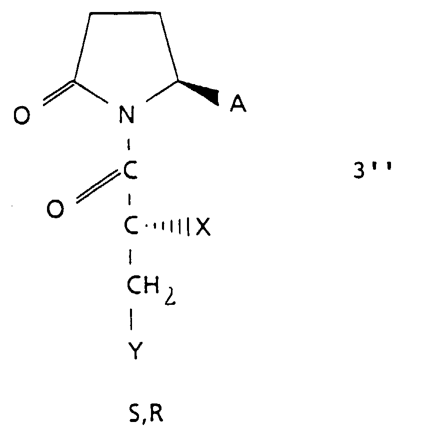 in which A is represented by -CH=CH₂ or -CH=C=CH₂,and X and Y are each simultaneously represented by 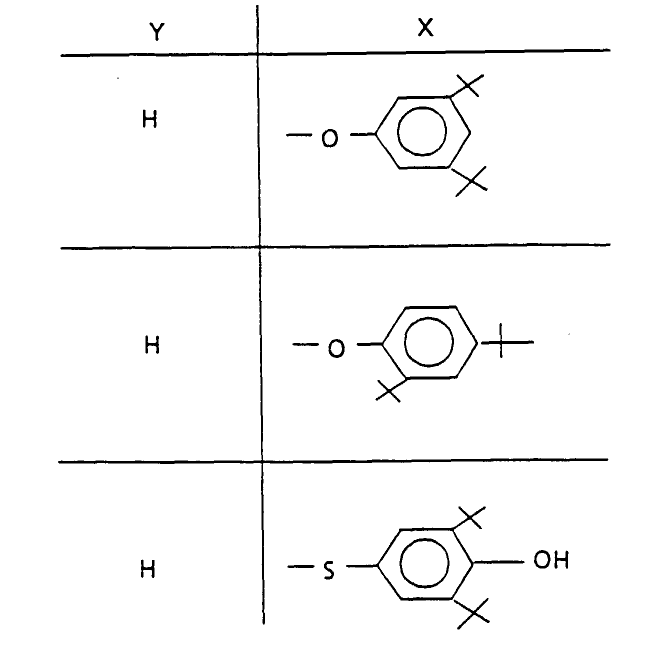 comprising separating diastereomers of the formula: 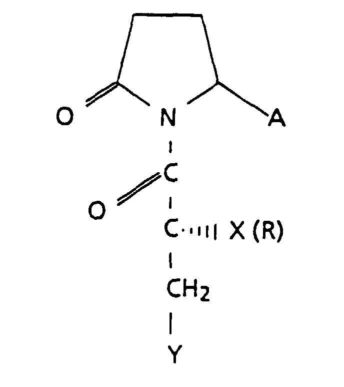 in which A, X, and Y are as above.

3. A process according to claim 1 in which said diastereomer is produced by carrying out an acylation reaction between a lactic acid derivative of the formula 2 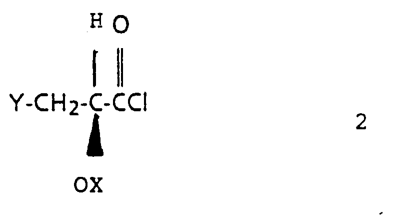 and a pyrrolidinone derivative of the formula 1 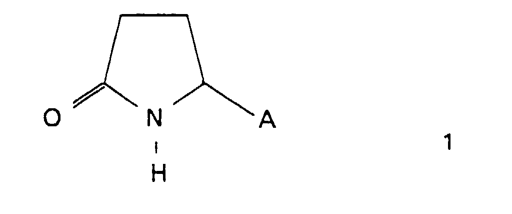 in which X, Y, and A are as above.

4. A process according to claim 2 in which said diastereomer is produced by carrying out an acylation reaction betwen a lactic acid derivative of the formula 6 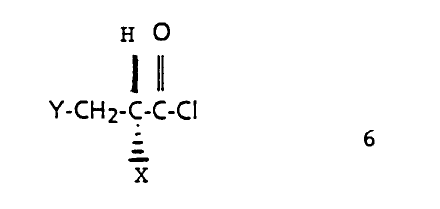 and a pyrrolidinone derivtive as described by the formula 1 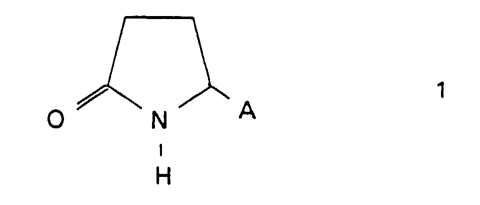 in which X, Y, and A are as above.

5. A process for producing an S-pyrrolidinone derivative of the formula 4 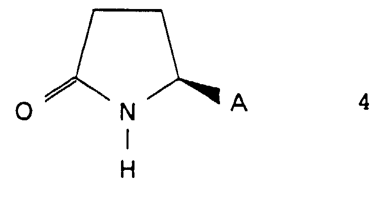 in which A is represented by -CH=CH₂ or -CH=C=CH₂ comprising:
a) separating diastereomers of the formula: 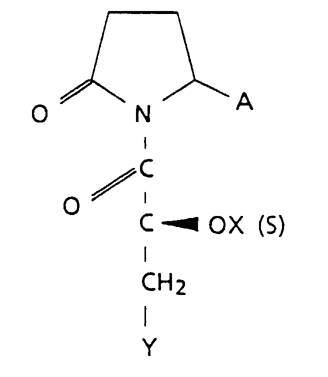 in which A is as above and X and Y are simultaneously represented by the following substituents; 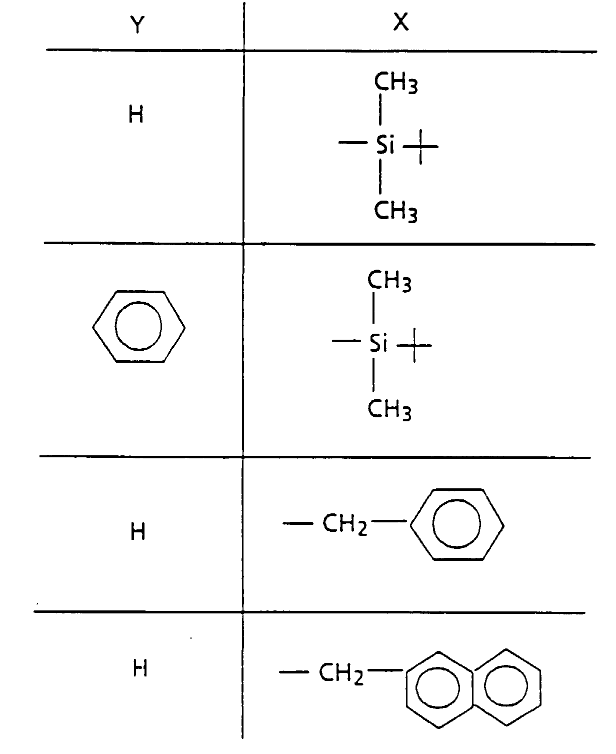 and
b) subjecting the resulting S,S isomer to a hydrolysis reaction.

6. A process for producing a compound of the formula 5 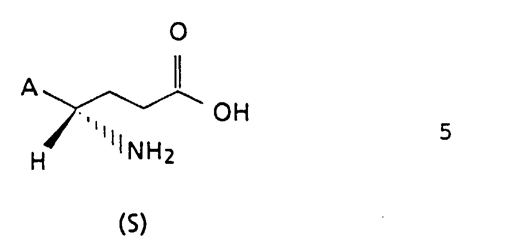 in which A is represented by CH₂=CH- or CH₂=C=CH- comprising:
a) separating diastereomers of the formula: 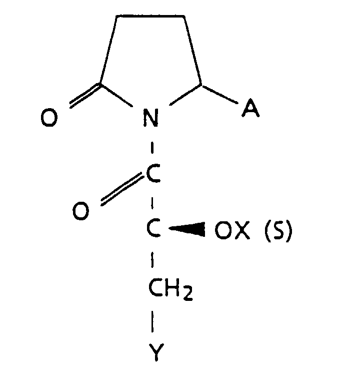 in which A is as above and X and Y are simultaneously represented by the following substituents; 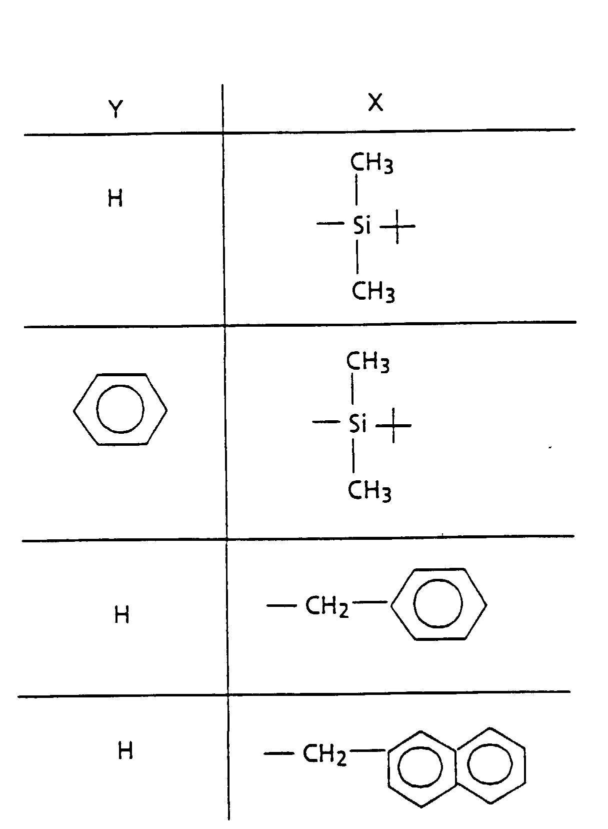 and
b) subjecting the resulting S,S isomer to a hydrolysis reaction.

7. A process for producing an S-pyrrolidinone derivative of the formula 4 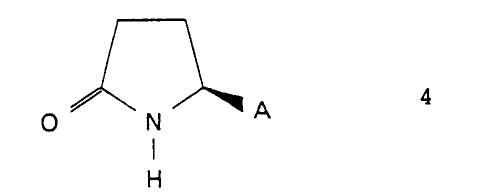 in which A is represented by -CH=CH₂ or -CH=C=CH₂ comprising:
a) separating diastereomers of the formula: 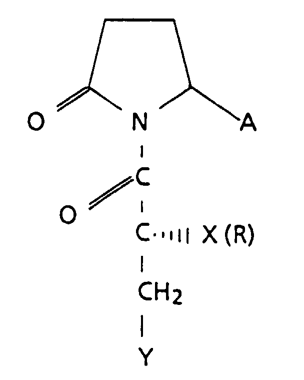 in which A is as above and X and Y are simultaneously represented by the following substituents, 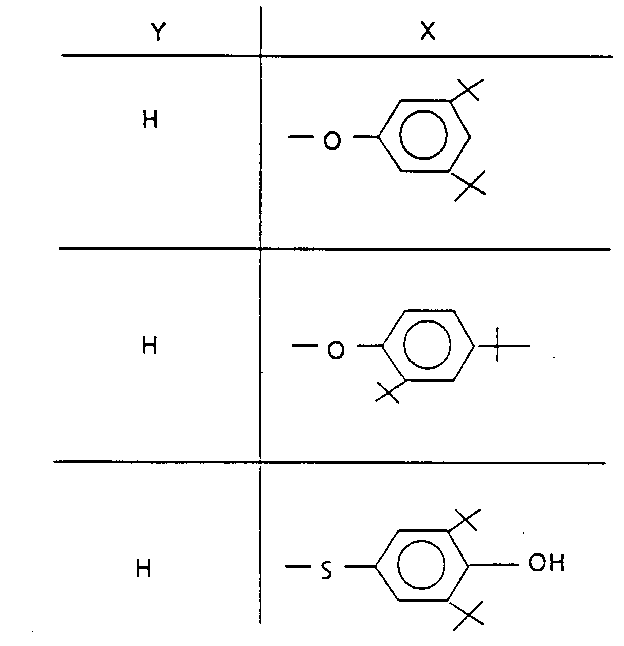 and
b) subjecting the resulting S,R isomer to a hydrolysis reaction.

8. A process for producing a compound of the formula 5 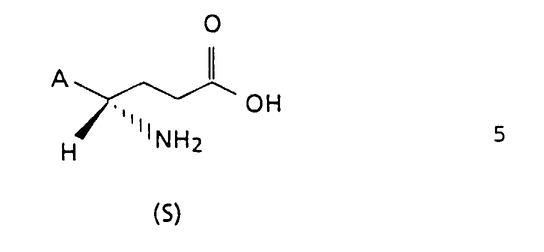 in which A is represented by CH₂=CH- or CH₂=C=CH-comprising:
a) separating diastereomers of the formula: 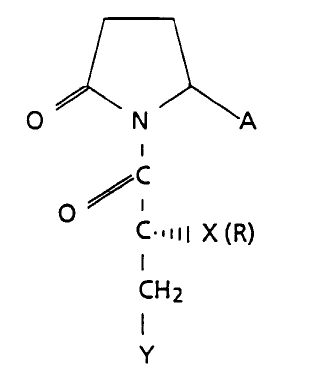 in which A is as above and X and Y are simultaneously represented by the following substituents; 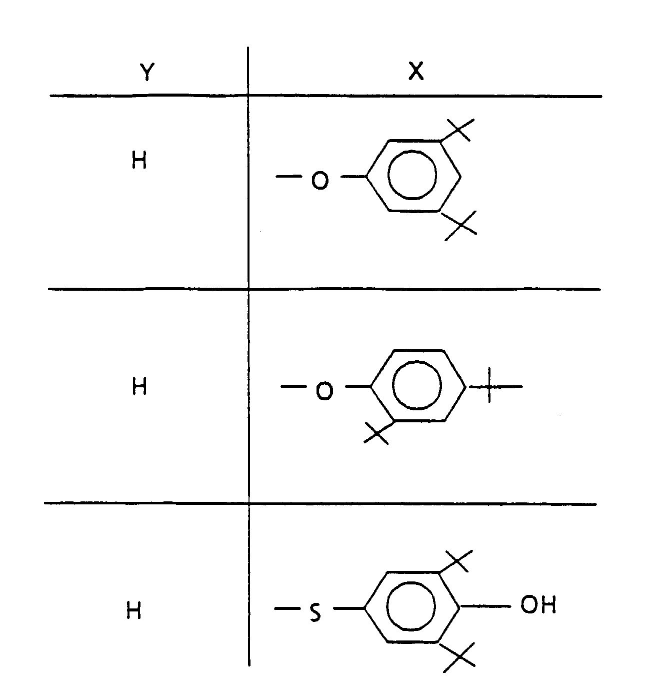 and
b) subjecting the resulting S,R isomer to a hydrolysis reaction.

## Patentansprüche (Patentansprüche für folgende(n) Vertragsstaat(en): AT, BE, CH, DE, DK, FR, GB, GR, IT, LI, LU, NL, SE)

1. Optisches Isomer der Formel 3 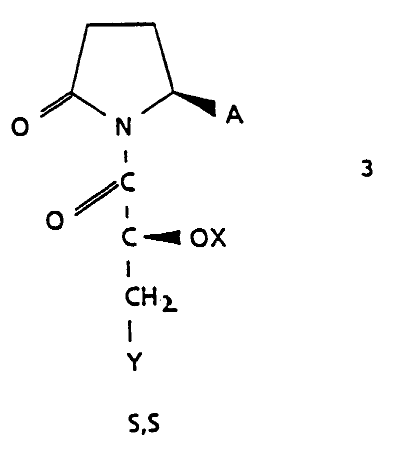 in der A durch -CH=CH₂ oder -CH=C=CH₂ wiedergegeben wird, und X und Y jeweils gleichzeitig durch 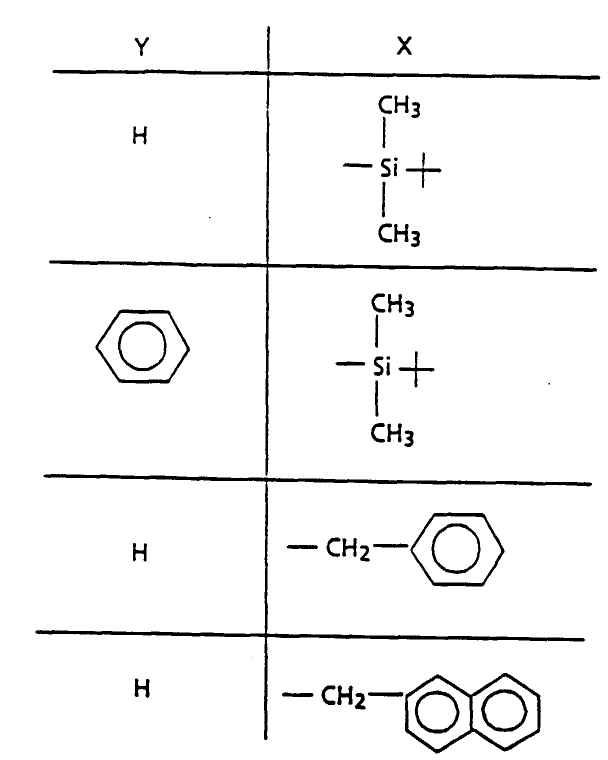 wiedergegeben werden.

2. Optisches Isomer der Formel 3" 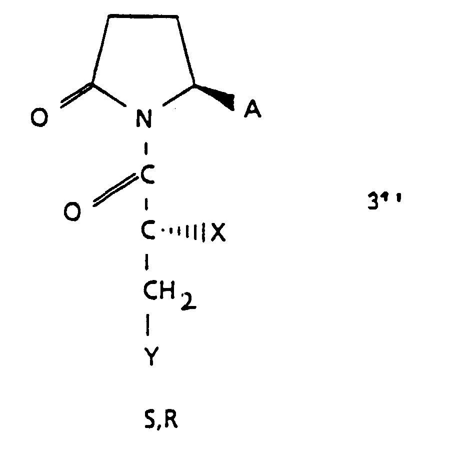 in der A durch -CH=CH₂ oder -CH=C=CH₂ wiedergegeben wird, und X und Y jeweils gleichzeitig durch 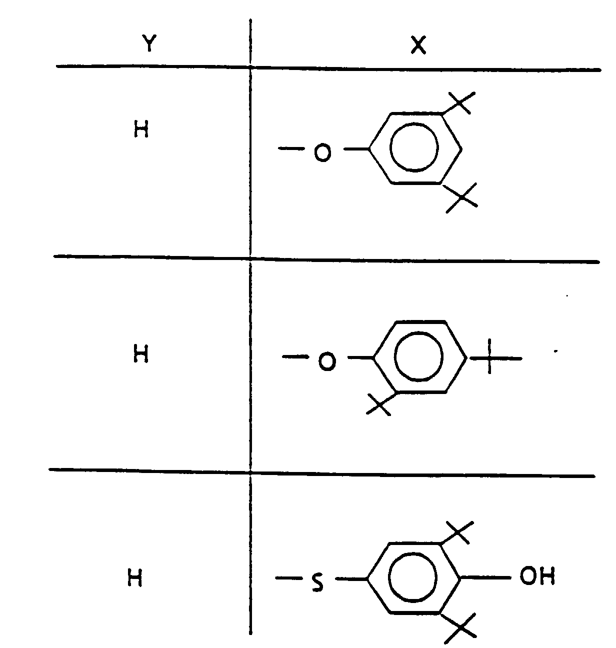 wiedergegeben werden.

3. Verfahren zur Herstellung eines optischen Isomeren nach Anspruch 1, umfassend die Trennung der Diastereomeren der Formel : 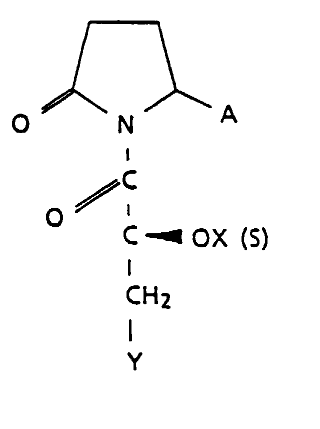 in der A, X und Y wie vorstehend definiert sind.

4. Verfahren nach Anspruch 3, wobei das Diastereomere hergestellt wird, indem eine Acylierungsreaktion zwischen einem Milchsäurederivat der Formel 2 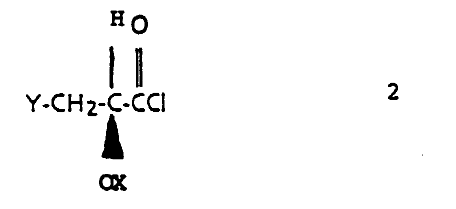 und einem Pyrrolidinonderivat der Formel 1 durchgeführt wird 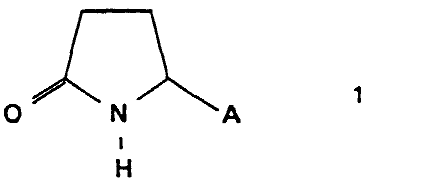 wobei X, Y und A wie vorstehend definiert sind.

5. Verfahren zur Herstellung einer Verbindung nach Anspruch 2, umfassend die Trennung der Diastereomeren der Formel : 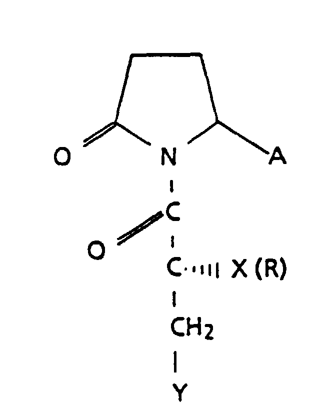 in der A, X und Y wie vorstehend definiert sind.

6. Verfahren nach Anspruch 5, wobei das Diastereomere hergestellt wird, indem eine Acylierungsreaktion zwischen einem Milchsäurederivat der Formel 6 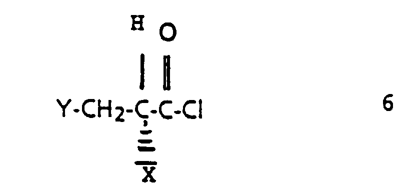 und einem Pyrrolidinonderivat der Formel 1 durchgeführt wird 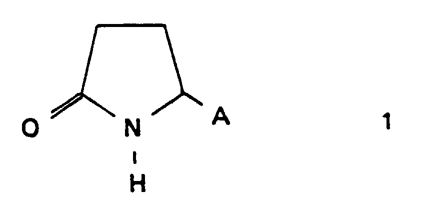 wobei X, Y und A wie vorstehend definiert sind.

7. Verfahren zur Herstellung eines S-Pyrrolidinonderivats der Formel 4 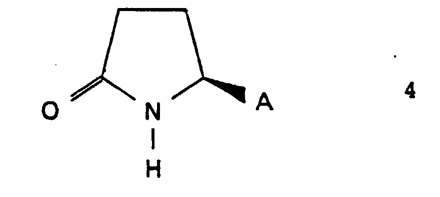 in der A durch -CH=CH₂ oder -CH=C=CH₂ wiedergegeben wird, umfassend
a) die Trennung der Diastereomeren der Formel: 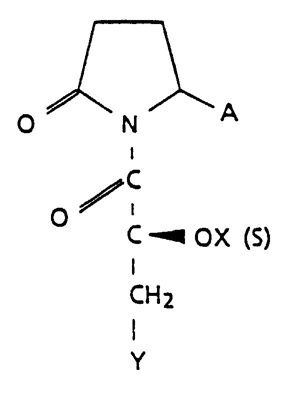 wobei A wie vorstehend definiert ist, und X und Y gleichzeitig durch die folgenden Substituenten wiedergegeben werden: 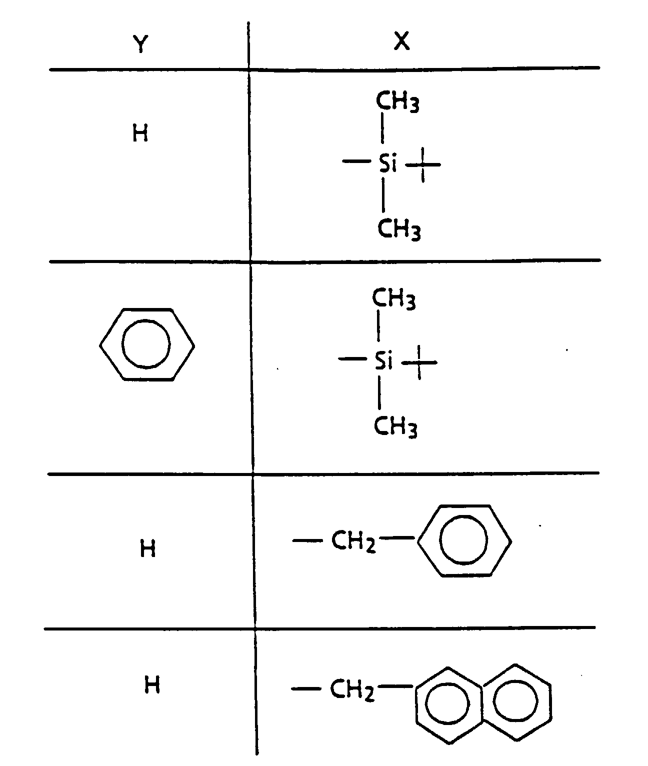 und
b) die Durchführung einer Hydrolysereaktion mit dem erhaltenen S,S-Isomeren.

8. Verfahren zur Herstellung einer Verbindung der Formel 5 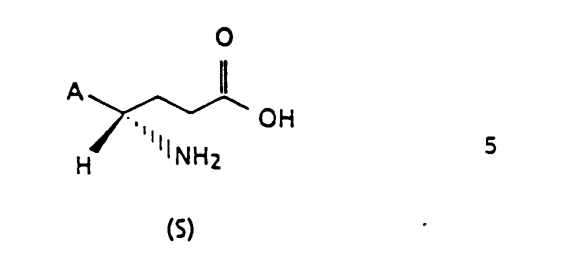 in der A durch -CH=CH₂ oder -CH=C=CH₂ wiedergegeben wird, umfassend:
a) die Trennung der Diastereomeren der Formel: 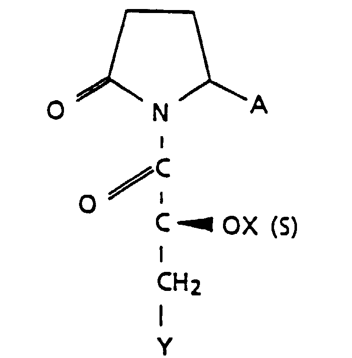 wobei A wie vorstehend definiert ist, und X und Y gleichzeitig durch die folgenden Substituenten wiedergegeben werden: 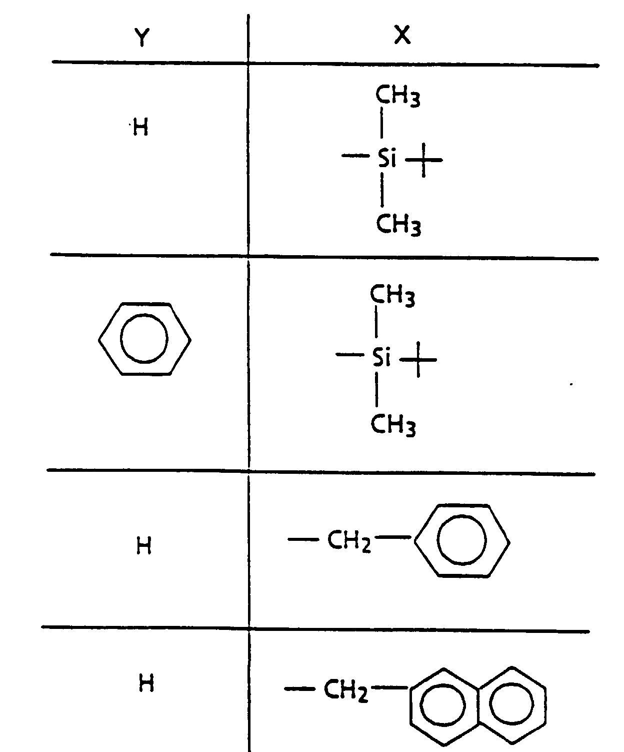 und
b) die Durchführung einer Hydrolysereaktion mit dem erhaltenen S,S-Isomeren.

9. Verfahren zur Herstellung eines S-Pyrrolidinonderivats der Formel 4 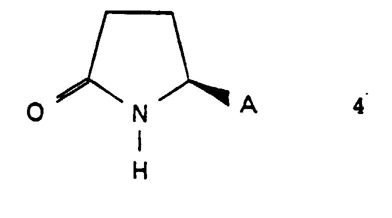 in der A durch -CH=CH₂ oder -CH=C=CH₂ wiedergegeben wird, umfassend:
a) die Trennung der Diastereomeren der Formel: 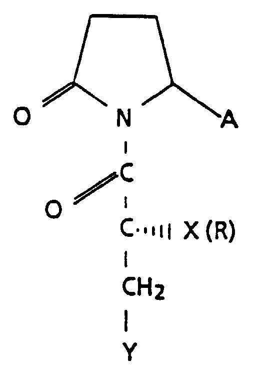 wobei A wie vorstehend definiert ist, und X und Y gleichzeitig durch die folgenden Substituenten wiedergegeben werden: 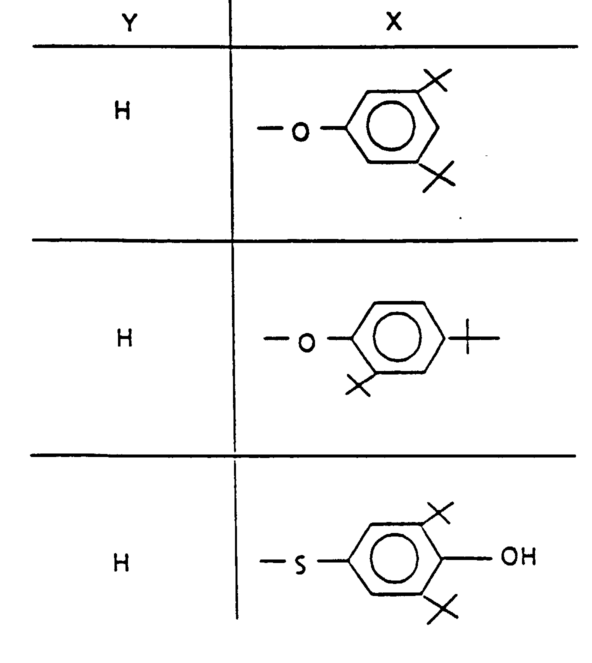 und
b) die Durchführung einer Hydrolysereaktion mit dem erhaltenen S,R-Isomeren.

10. Verfahren zur Herstellung einer Verbindung der Formel 5 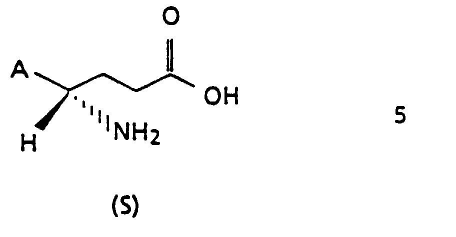 in der A durch -CH=CH₂ oder -CH=C=CH₂ wiedergegeben wird, umfassend:
a) die Trennung der Diastereomeren der Formel: 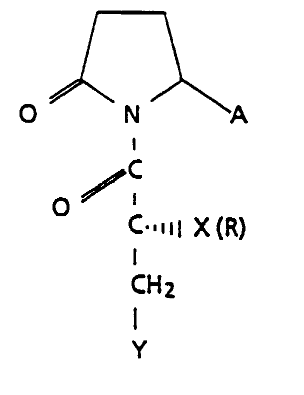 wobei A wie vorstehend definiert ist, und X und Y gleichzeitig durch die folgenden Substituenten wiedergegeben werden: 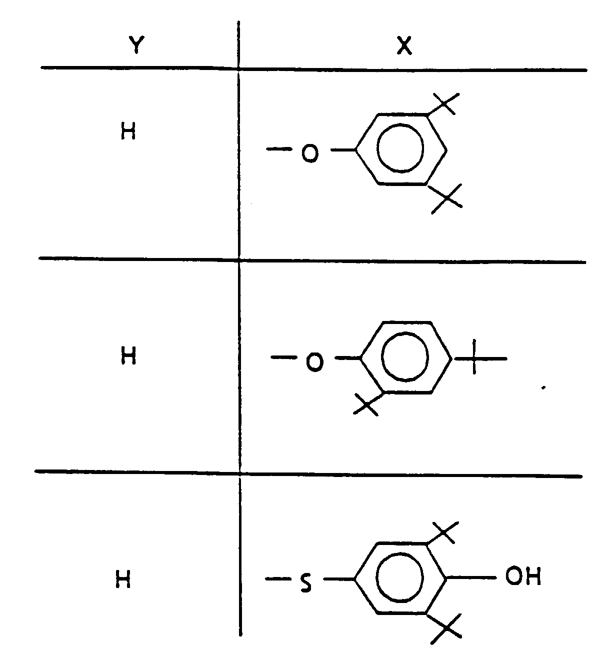 und
b) die Durchführung einer Hydrolysereaktion mit dem erhaltenen S,R-Isomeren.

11. Verbindung der Formel 6 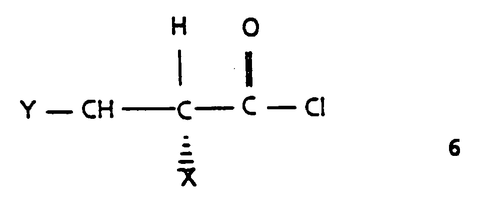 in der A durch -CH=CH₂ oder -CH=C=CH₂ wiedergegeben wird, und X und Y jeweils gleichzeitig durch 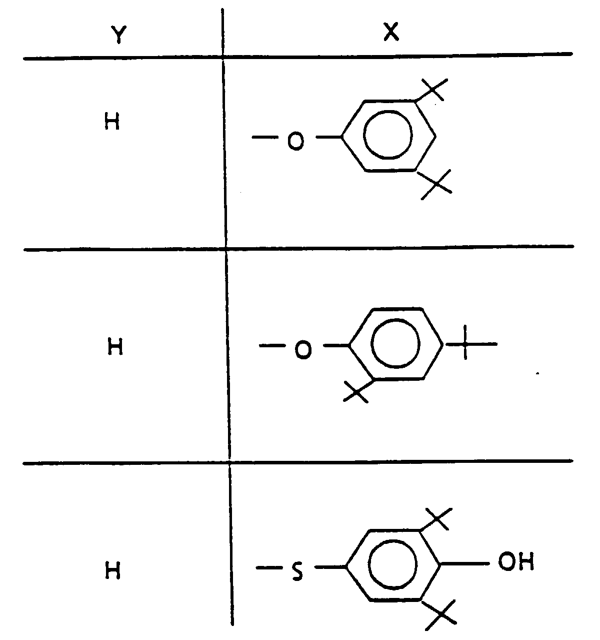 wiedergegeben werden.

12. Verbindung der Formel 2 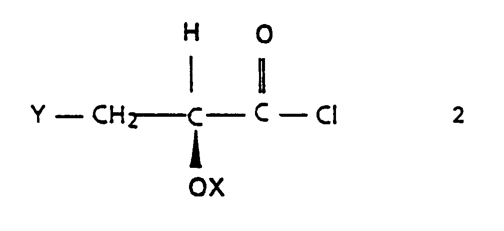 in der A durch -CH=CH₂ oder -CH=C=CH₂ wiedergegeben wird, und X und Y jeweils gleichzeitig durch 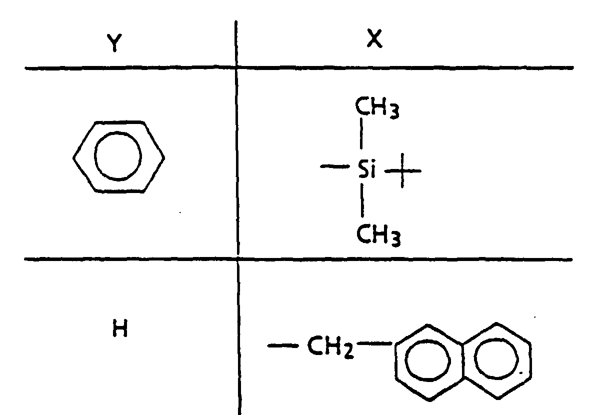 wiedergegeben werden.

## Patentansprüche (Patentansprüche für folgende(n) Vertragsstaat(en): ES)

1. Verfahren zur Herstellung eines optischen Isomeren der Formel 3 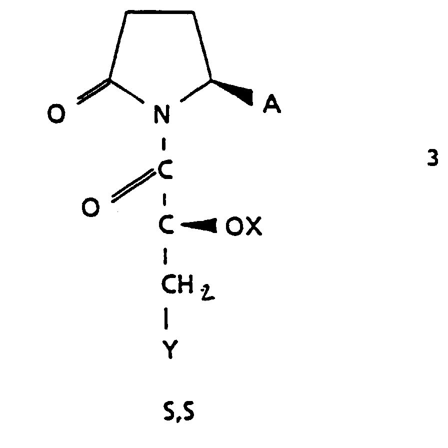 in der A durch -CH=CH₂ oder -CH=C=CH₂ wiedergegeben wird, und X und Y jeweils gleichzeitig durch 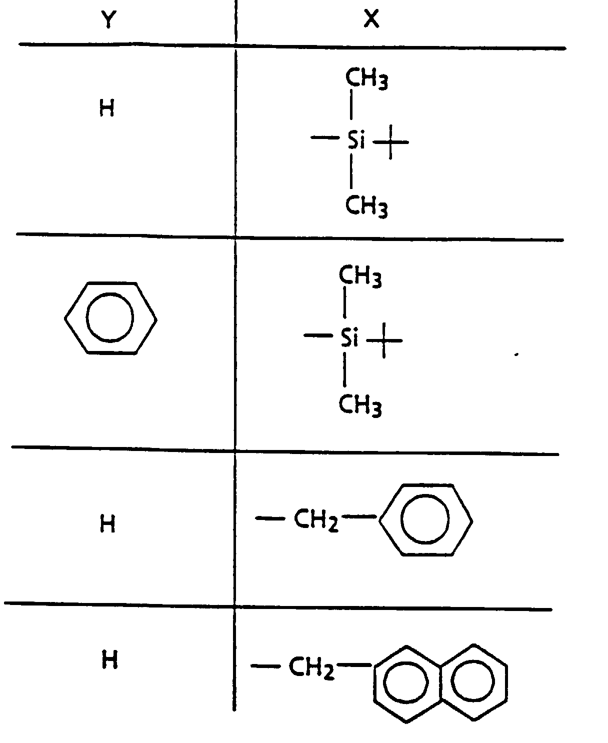 wiedergegeben werden, umfassend die Trennung der Diastereomeren der Formel : 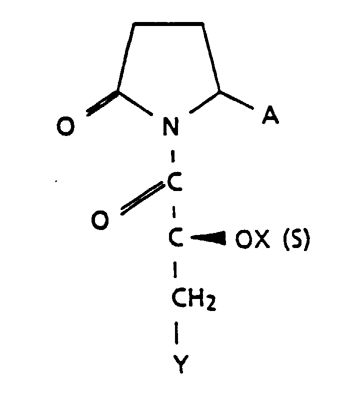 in der A, X und Y wie vorstehend definiert sind.

2. Verfahren zur Herstellung eines optischen Isomeren der Formel 3" 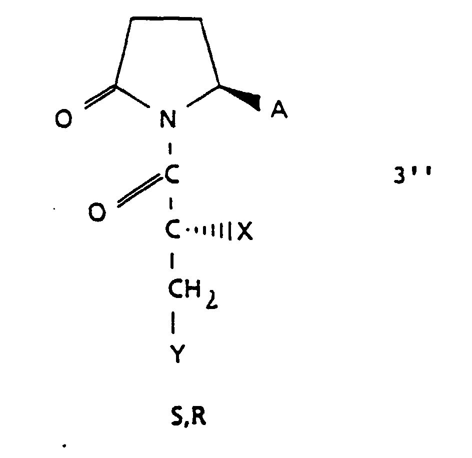 in der A durch -CH=CH₂ oder -CH=C=CH₂ wiedergegeben wird, und X und Y jeweils gleichzeitig durch 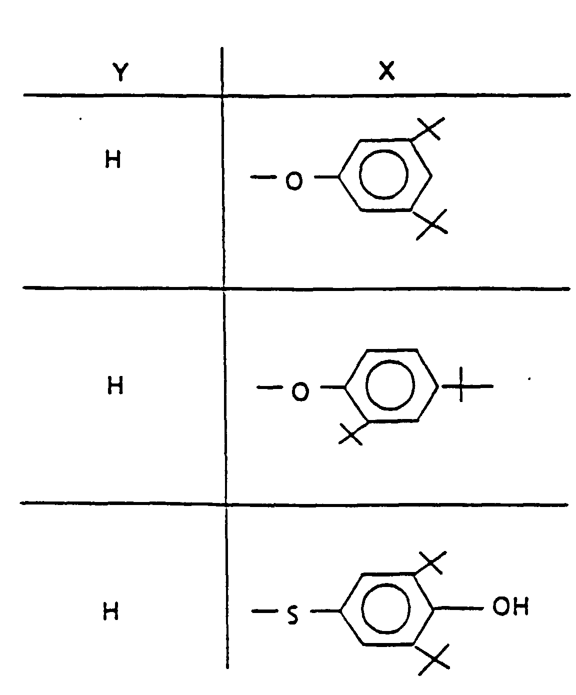 wiedergegeben werden, umfassend die Trennung der Diastereomeren der Formel : 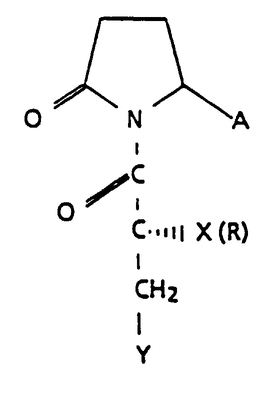 in der A, X und Y wie vorstehend definiert sind.

3. Verfahren nach Anspruch 1, wobei das Diastereomere hergestellt wird, indem eine Acylierungsreaktion zwischen einem Milchsäurederivat der Formel 2 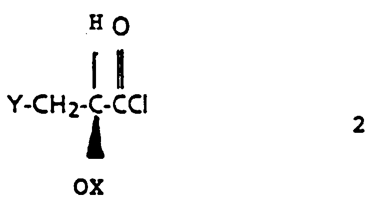 und einem Pyrrolidinonderivat der Formel 1 durchgeführt wird 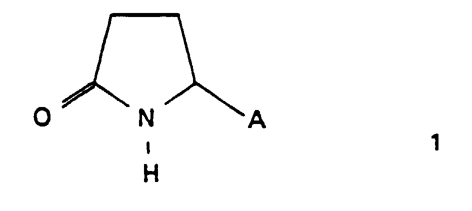 wobei X, Y und A wie vorstehend definiert sind.

4. Verfahren nach Anspruch 2, wobei das Diastereomere hergestellt wird, indem eine Acylierungsreaktion zwischen einem Milchsäurederivat der Formel 6 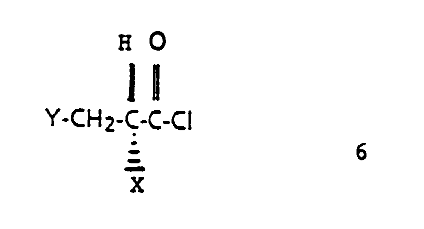 und einem Pyrrolidinonderivat der Formel 1 durchgeführt wird 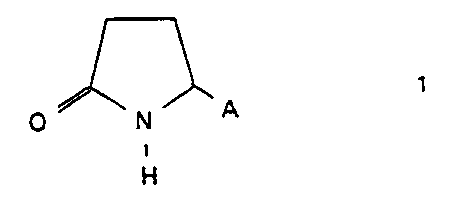 wobei X, Y und A wie vorstehend definiert sind.

5. Verfahren zur Herstellung eines S-Pyrrolidinonderivats der Formel 4 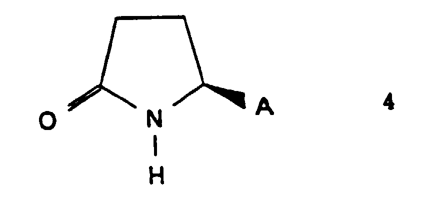 in der A durch -CH=CH₂ oder -CH=C=CH₂ wiedergegeben wird, umfassend
a) die Trennung der Diastereomeren der Formel: 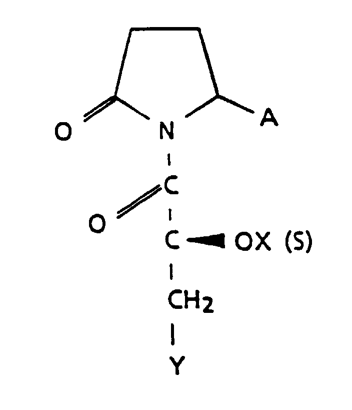 wobei A wie vorstehend definiert ist, und X und Y gleichzeitig durch die folgenden Substituenten wiedergegeben werden: 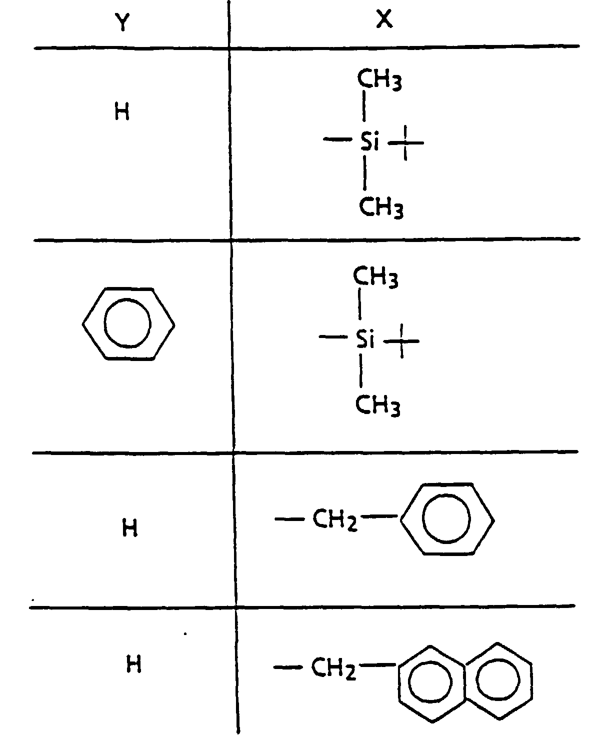 und
b) die Durchführung einer Hydrolysereaktion mit dem erhaltenen S,S-Isomeren.

6. Verfahren zur Herstellung einer Verbindung der Formel 5 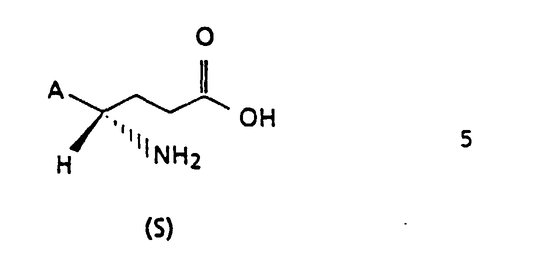 in der A durch -CH=CH₂ oder -CH=C=CH₂ wiedergegeben wird, umfassend:
a) die Trennung der Diastereomeren der Formel: 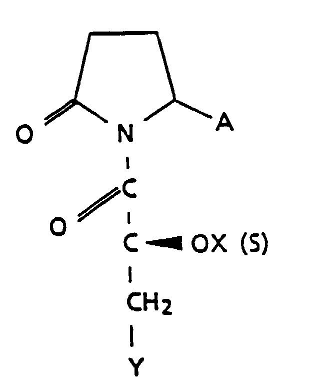 wobei A wie vorstehend definiert ist, und X und Y gleichzeitig durch die folgenden Substituenten wiedergegeben werden: 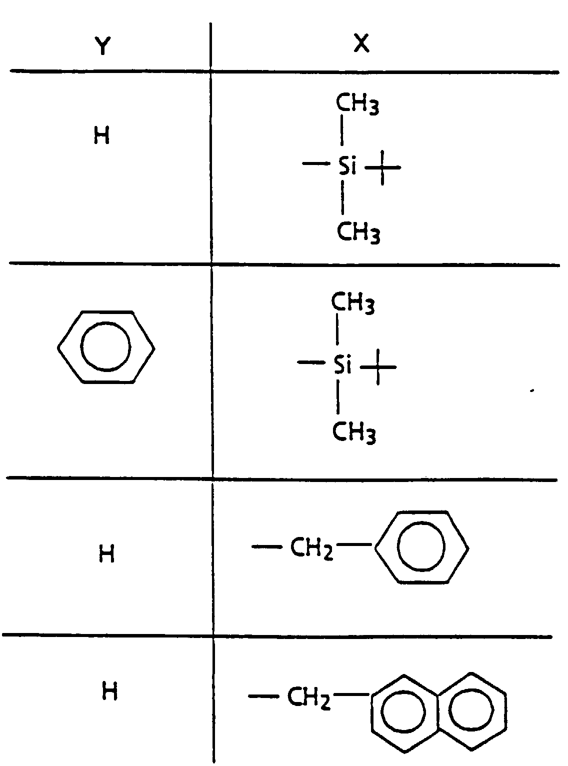 und
b) die Durchführung einer Hydrolysereaktion mit dem erhaltenen S,S-Isomeren.

7. Verfahren zur Herstellung eines S-Pyrrolidinonderivats der Formel 4 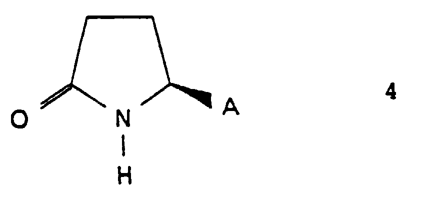 in der A durch -CH=CH₂ oder -CH=C=CH₂ wiedergegeben wird, umfassend:
a) die Trennung der Diastereomeren der Formel: 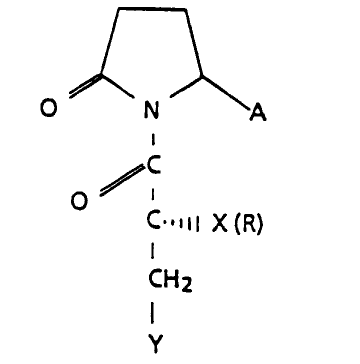 wobei A wie vorstehend definiert ist, und X und Y gleichzeitig durch die folgenden Substituenten wiedergegeben werden: 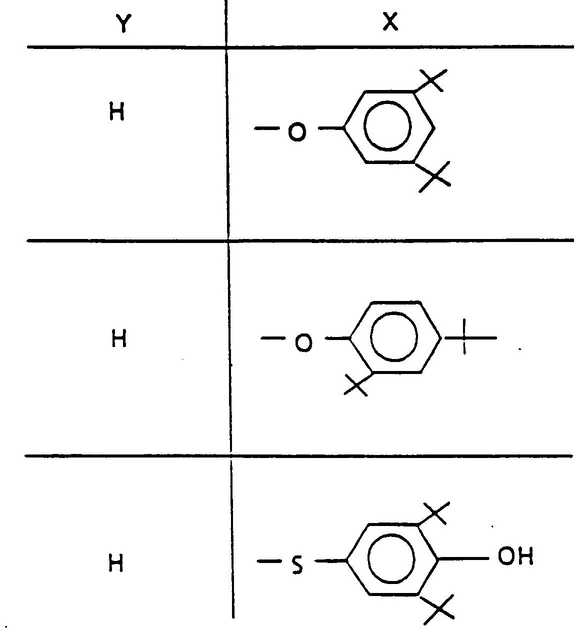 und
b) die Durchführung einer Hydrolysereaktion mit dem erhaltenen S,R-Isomeren.

8. Verfahren zur Herstellung einer Verbindung der Formel 5 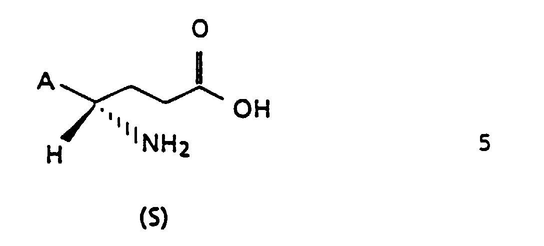 in der A durch -CH=CH₂ oder -CH=C=CH₂ wiedergegeben wird, umfassend:
a) die Trennung der Diastereomeren der Formel: 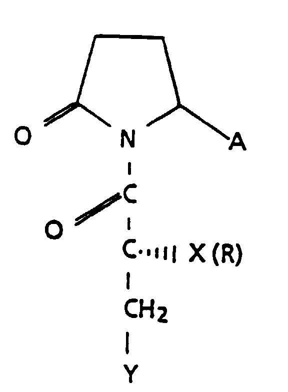 wobei A wie vorstehend definiert ist, und X und Y gleichzeitig durch die folgenden Substituenten wiedergegeben werden: 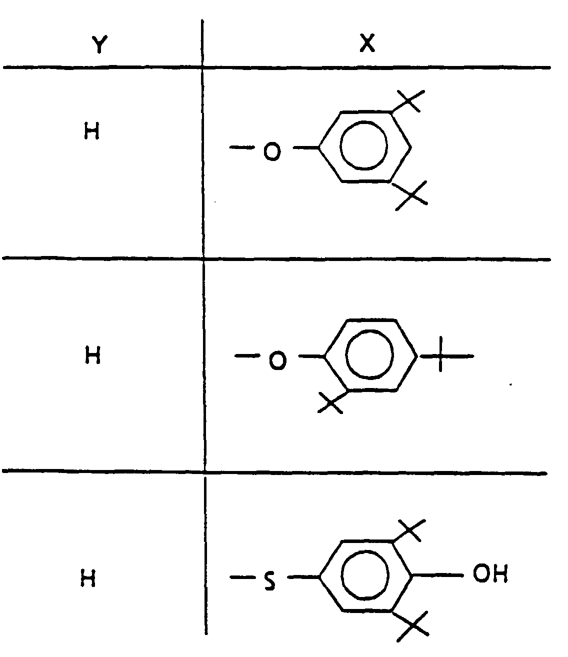 und
b) die Durchführung einer Hydrolysereaktion mit dem erhaltenen S,R-Isomeren.

## Revendications (Revendications pour l'(les) Etat(s) contractant(s) suivant(s): AT, BE, CH, DE, DK, FR, GB, GR, IT, LI, LU, NL, SE)

1. Isomère optique de formule 3 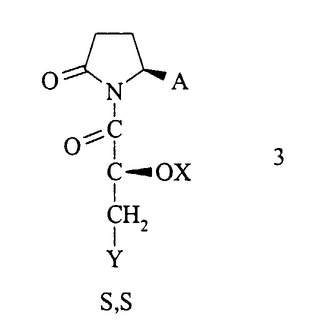 dans laquelle A est représenté par -CH=CH₂ ou -CH=C=CH₂ et X et Y sont chacun représentés simultanément par

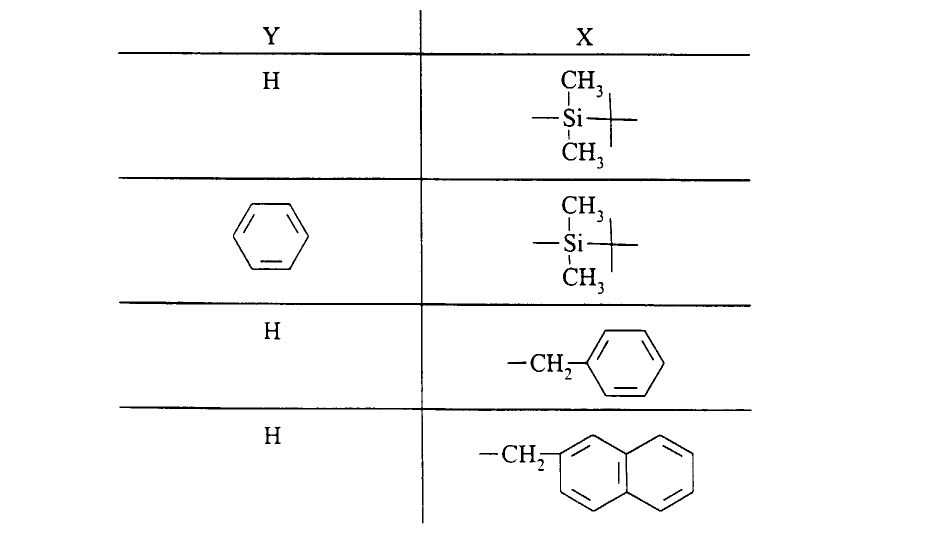

2. Isomère optique de formule 3" 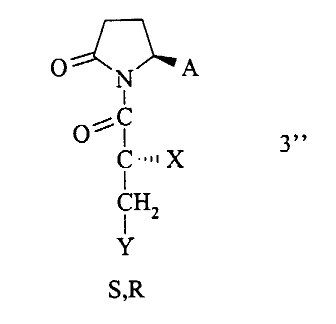 dans laquelle A est représenté par -CH=CH₂ ou -CH=C=CH₂ et X et Y sont chacun représentés simultanément par

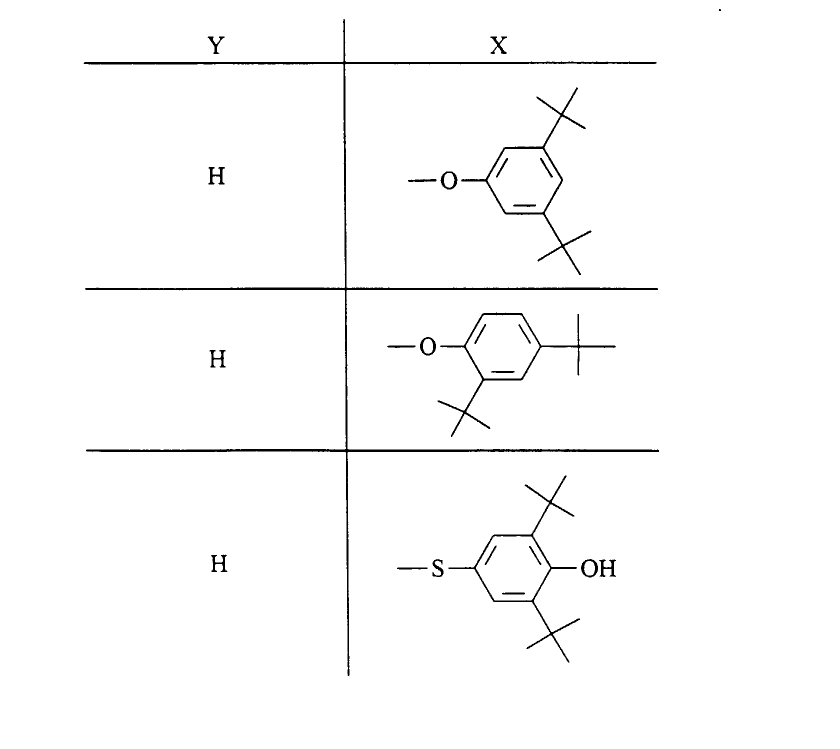

3. Procédé d'obtention d'un isomère optique selon la revendication 1 comprenant la séparation des diastéréoisomères de formule : 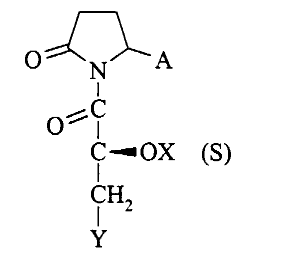 dans laquelle A, X et Y sont tels que définis ci-dessus.

4. Procédé selon la revendication 3, dans lequel le dit diastéréoisomère est obtenu en effectuant une réaction d'acylation entre un dérivé d'acide lactique de formule 2 : 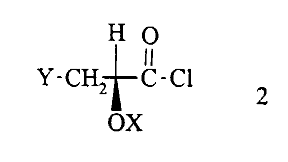 et un dérivé de pyrrolidinone de formule 1 : 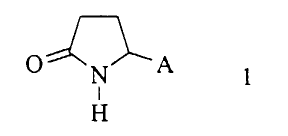 dans lesquelles X, Y et A sont tels que définis ci-dessus.

5. Procédé d'obtention d'un composé selon la revendication 2 comprenant la séparation des diastéréoisomères de formule : 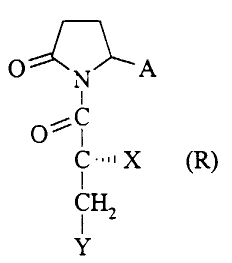 dans laquelle A, X et Y sont tels que définis ci-dessus.

6. Procédé selon la revendication 5, dans lequel le dit diastéréoisomère est obtenu en effectuant une réaction d'acylation entre un dérivé d'acide lactique de formule 6 : 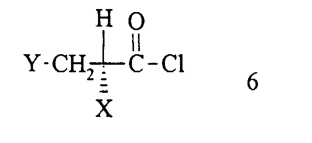 et un dérivé de pyrrolidinone tel que décrit par la formule 1 : 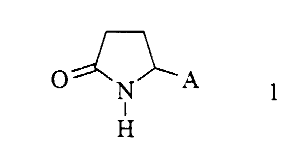 dans lesquelles X, Y et A sont tels que définis ci-dessus.

7. Procédé d'obtention d'un dérivé de S-pyrrolidinone de formule 4 : 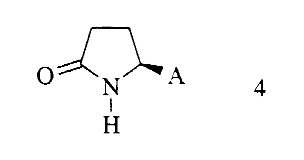 dans laquelle A est représenté par -CH=CH₂ ou -CH=C=CH₂, comprenant :
a) la séparation des diastéréoisomères de formule : 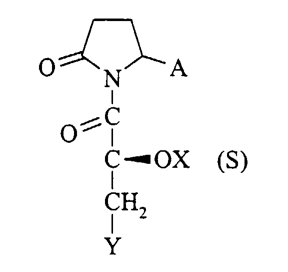 dans laquelle A est tel que défini ci-dessus et X et Y sont représentés simultanément par les substituants suivants : 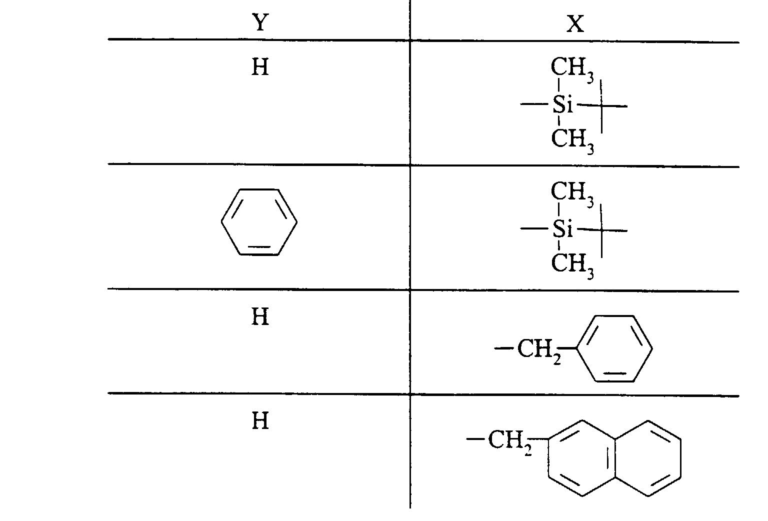
et b) la soumission de l'isomère S,S résultant à une réaction d'hydrolyse.

8. Procédé d'obtention d'un composé de formule 5 : 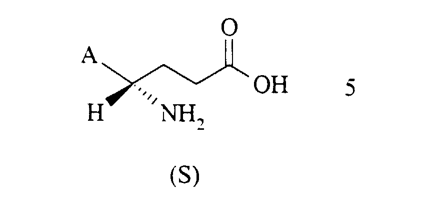 dans laquelle est représenté par -CH=CH₂ ou -CH=C=CH₂, comprenant :
a) la séparation des diastéréoisomères de formule : 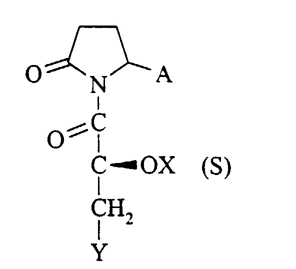 dans laquelle A est tel que défini ci-dessus et X et Y sont représentés simultanément par les substituants suivants : 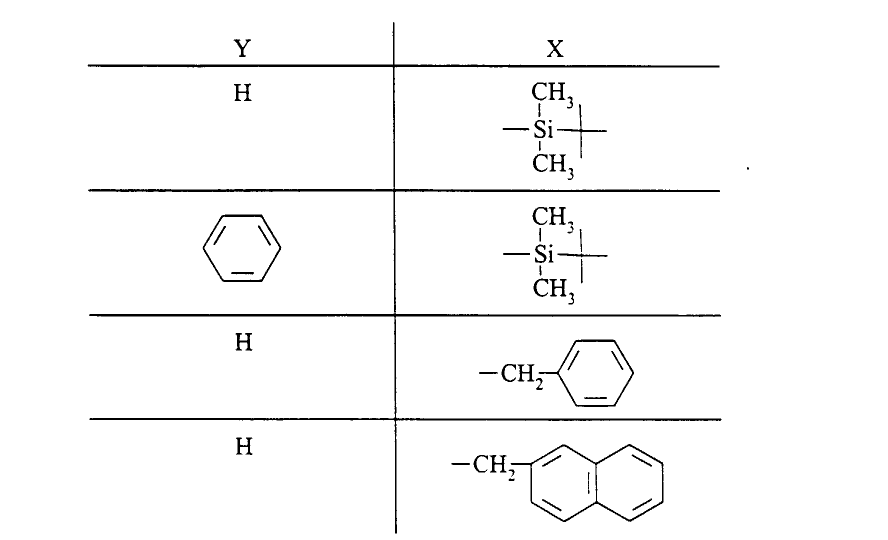
et b) la soumission de l'isomère S,S résultant à une réaction d'hydrolyse.

9. Procédé d'obtention d'un dérivé de S-pyrrolidinone de formule 4 : 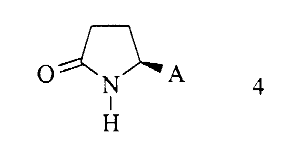 dans laquelle A est représenté par -CH=CH₂ ou -CH=C=CH₂, comprenant :
a) la séparation des diastéréoisomères de formule : 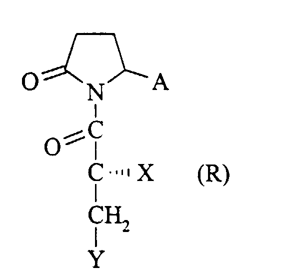 dans laquelle A est tel que défini ci-dessus et X et Y sont représentés simultanément par les substituants suivants : 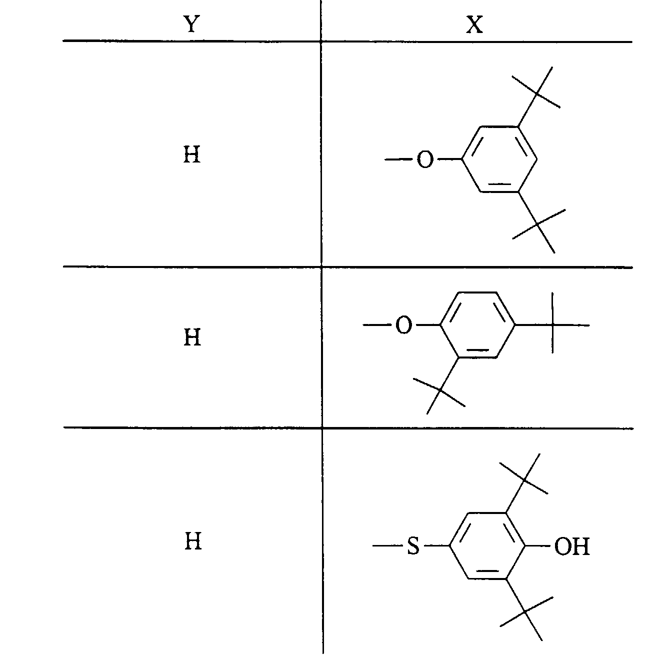
et b) la soumission de l'isomère S,R résultant à une réaction d'hydrolyse.

10. Procédé d'obtention d'un composé de formule 5 : 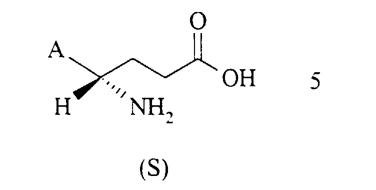 dans laquelle A est représenté par -CH=CH₂ ou -CH=C=CH₂, comprenant :
a) la séparation des diastéréoisomères de formule : 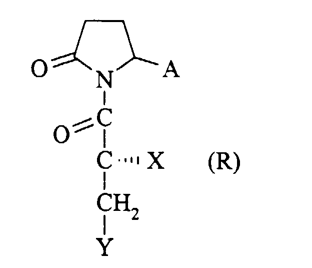 dans laquelle A est tel que défini ci-dessus et X et Y sont représentés simultanément par les substituants suivants : 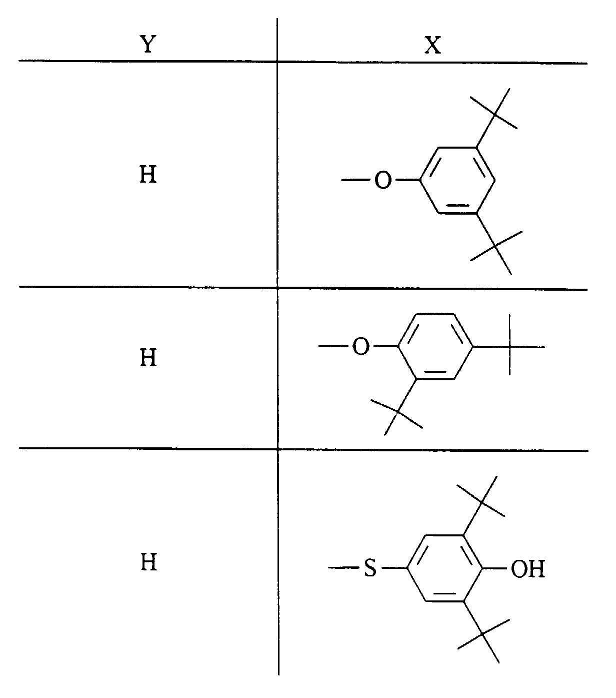
et b) la soumission de l'isomère S,R résultant à une réaction d'hydrolyse.

11. Composé de formule 6 : 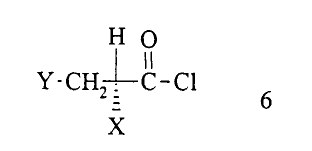 dans laquelle A est représenté par -CH=CH₂ ou -CH=C=CH₂ et X et Y sont chacun représentés simultanément par

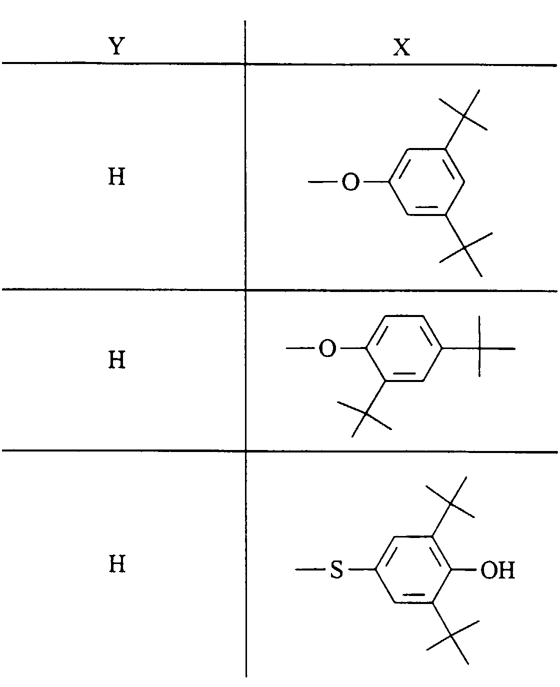

12. Composé de formule 2 : 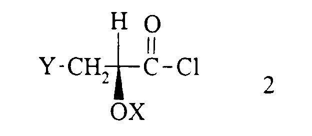 dans laquelle A est représenté par -CH=CH₂ ou -CH=C=CH₂ et X et Y sont chacun représentés simultanément par

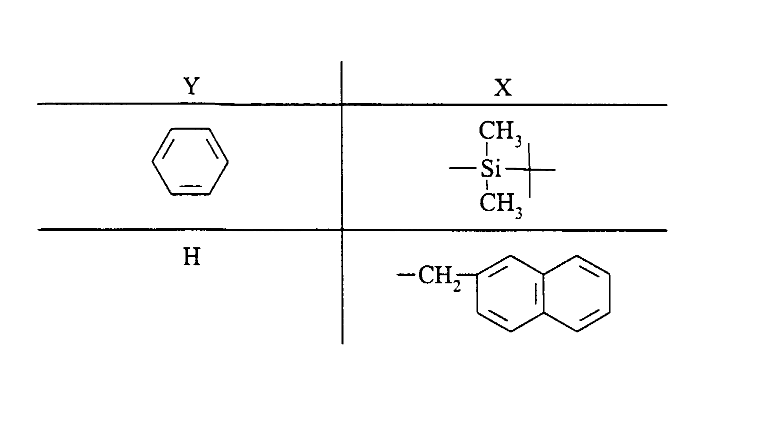

## Revendications (Revendications pour l'(les) Etat(s) contractant(s) suivant(s): ES)

1. Procédé pour la préparation d'un isomère optique de formule 3 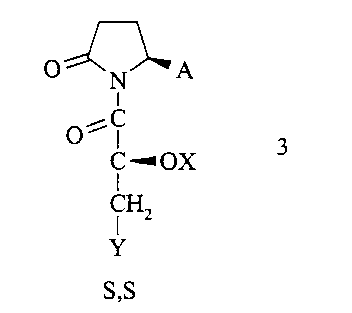 dans laquelle A est représenté par -CH=CH₂ ou -CH=C=CH₂ et X et Y sont chacun représentés simultanément par 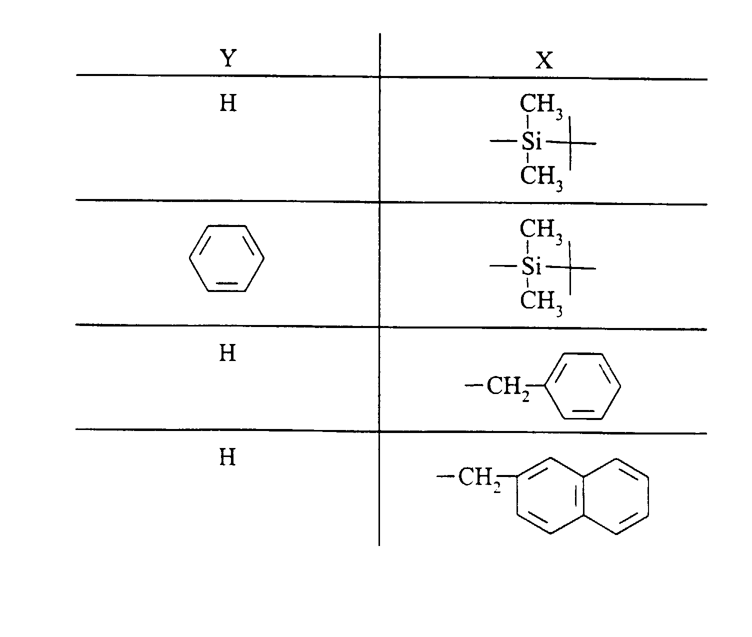 comprenant la séparation des diastéréoisomères de formule : 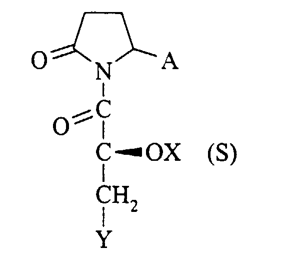 dans laquelle A, X et Y sont tels que définis ci-dessus.

2. Procédé pour la préparation d'un isomère optique de formule 3" 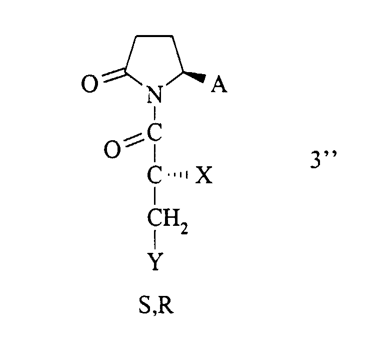 dans laquelle A est représenté par -CH=CH₂ ou -CH=C=CH₂ et X et Y sont chacun représentés simultanément par 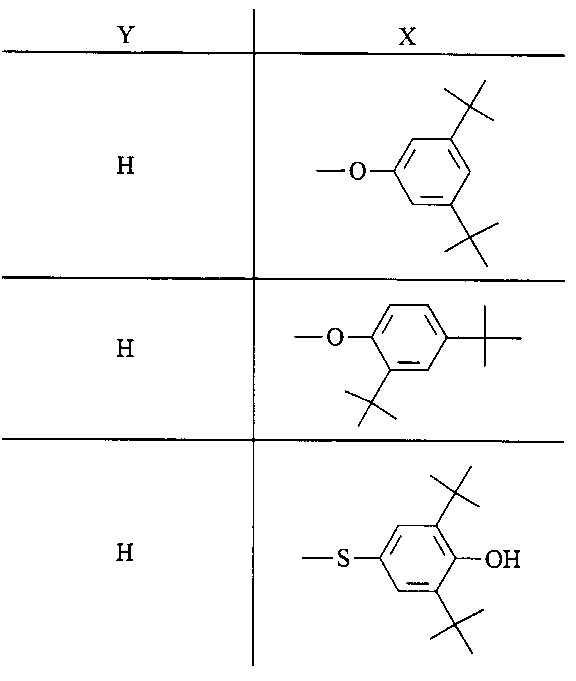 comprenant la séparation des diastéréoisomères de formule : 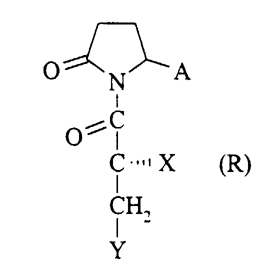 dans laquelle A, X et Y sont tels que définis ci-dessus.

3. Procédé selon la revendication 1, dans lequel le dit diastéréoisomère est obtenu en effectuant une réaction d'acylation entre un dérivé d'acide lactique de formule 2 : 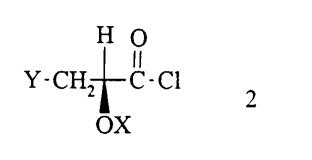 et un dérivé de pyrrolidinone de formule 1 : 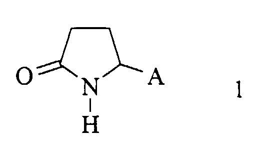 dans lesquelles X, Y et A sont tels que définis ci-dessus.

4. Procédé selon la revendication 2, dans lequel le dit diastéréoisomère est obtenu en effectuant une réaction d'acylation entre un dérivé d'acide lactique de formule 6 : 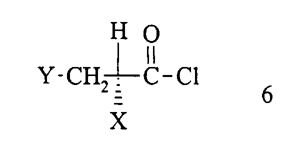 et un dérivé de pyrrolidinone tel que décrit par la formule 1 : 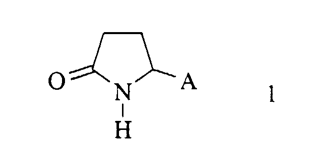 dans lesquelles X, Y et A sont tels que définis ci-dessus.

5. Procédé d'obtention d'un dérivé de S-pyrrolidinone de formule 4 : 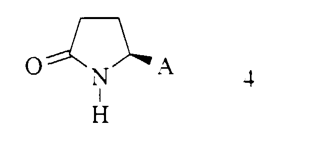 dans laquelle A est représenté par -CH=CH₂ ou -CH=C=CH₂, comprenant :
a) la séparation des diastéréoisomères de formule : 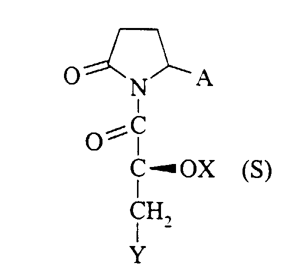 dans laquelle A est tel que défini ci-dessus et X et Y sont représentés simultanément par les substituants suivants : 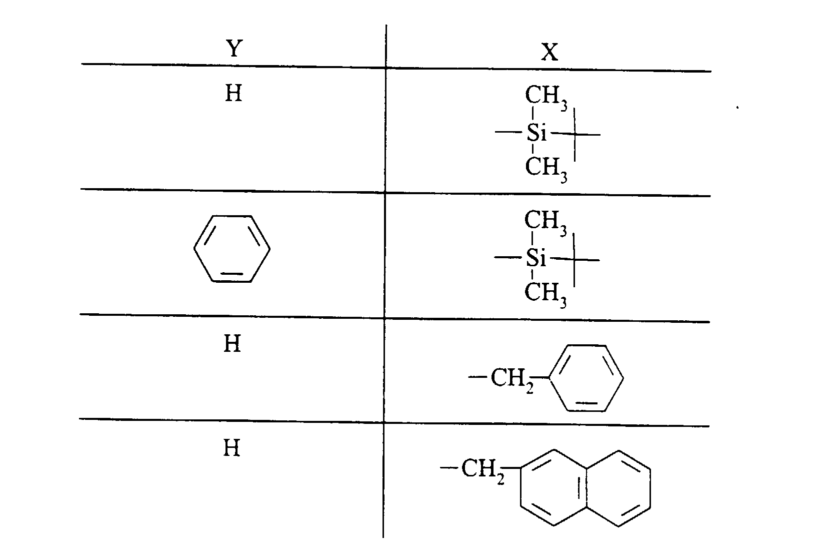
et b) la soumission de l'isomère S,S résultant à une réaction d'hydrolyse.

6. Procédé d'obtention d'un composé de formule 5 : 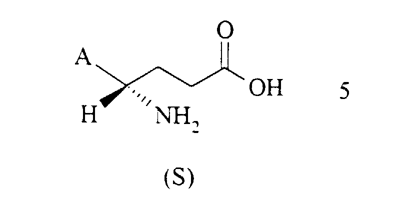 dans laquelle A est représenté par -CH=CH₂ ou -CH=C=CH₂, comprenant :
a) la séparation des diastéréoisomères de formule : 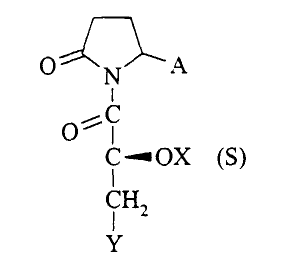 dans laquelle A est tel que défini ci-dessus et X et Y sont représentés simultanément par les substituants suivants : 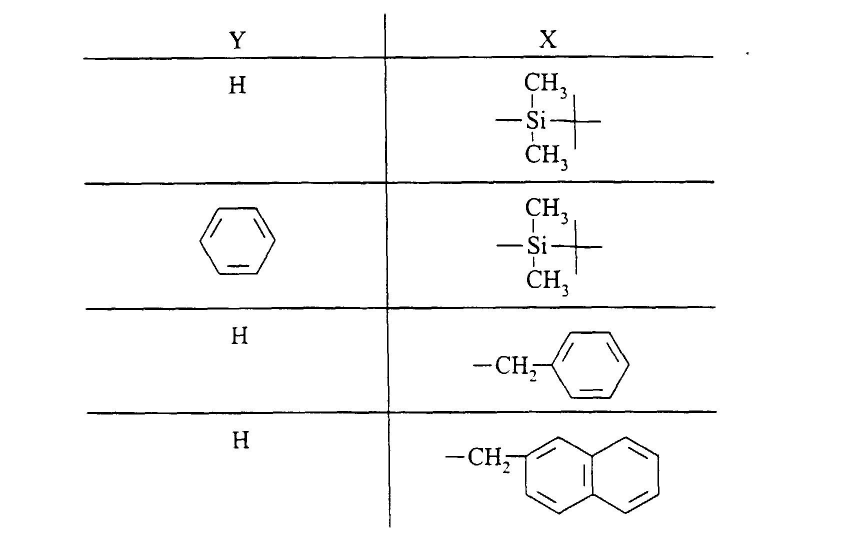
et b) la soumission de l'isomère S,S résultant à une réaction d'hydrolyse.

7. Procédé d'obtention d'un dérivé de S-pyrrolidinone de formule 4 : 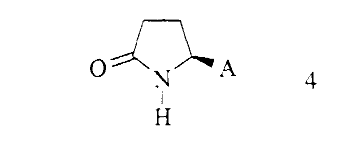 dans laquelle A est représenté par -CH=CH₂ ou -CH=C=CH₂, comprenant
a) la séparation des diastéréoisomères de formule : 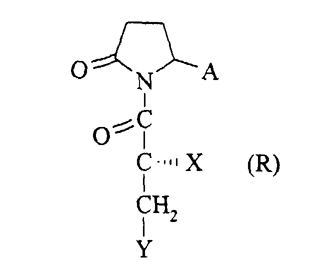 dans laquelle A est tel que défini ci-dessus et X et Y sont représentés simultanément par les substituants suivants : 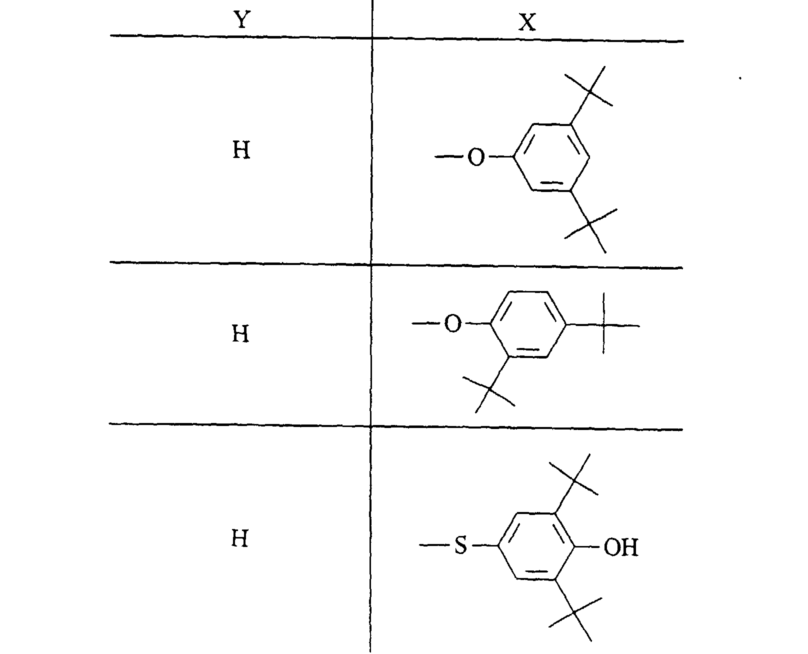
et b) la soumission de l'isomère S,R résultant à une réaction d'hydrolyse.

8. Procédé d'obtention d'un composé de formule 5 : 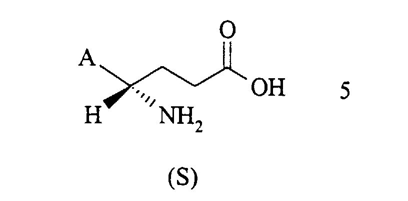 dans laquelle A est représenté par -CH=CH₂ ou -CH=C=CH₂, comprenant :
a) la séparation des diastéréoisomères de formule : 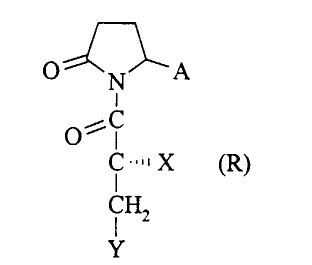 dans laquelle A est tel que défini ci-dessus et X et Y sont représentés simultanément par les substituants suivants : 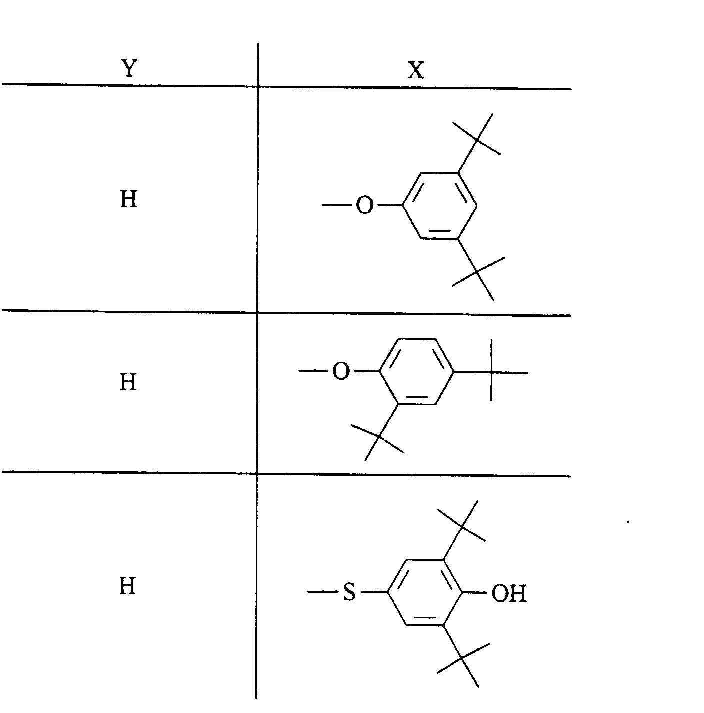
et b) la soumission de l'isomère S,R résultant à une réaction d'hydrolyse.
